# EUROPEAN PATENT SPECIFICATION

(11) **EP 4 608 820 B1**
(45) Date of publication and mention of the grant of the patent: **09.12.2026**
(21) Application number: 23798139.4
(22) Date of filing: 24.10.2023
(51) Int. Cl.: C07D 401/14, C07D 413/14, C07D 471/08, C07D 487/08, A61P 35/00, A61K 31/4439, A61K 31/444, A61K 31/4995

(54) **HETEROCYCLIC COMPOUNDS CAPABLE OF ACTIVATING STING**
HETEROCYCLISCHE VERBINDUNGEN ZUR STING-AKTIVIERUNG
COMPOSÉS HÉTÉROCYCLIQUES CAPABLES D'ACTIVER UN STING

(30) Priority: 26.10.2022 EP 22203737
(43) Date of publication of application: 03.09.2025
(73) Proprietor: Boehringer Ingelheim International GmbH, 55216 Ingelheim am Rhein (DE)
(72) Inventor: GRAHAM, Keith Andrew Newton, 55216 INGELHEIM AM RHEIN (DE); CAROTTA, Sebastian, 55216 INGELHEIM AM RHEIN (DE); DAHMANN, Georg, 55216 INGELHEIM AM RHEIN (DE); GODBOUT, Cédrickx, 55216 INGELHEIM AM RHEIN (DE); HANDSCHUH, Sandra Ruth, 55216 INGELHEIM AM RHEIN (DE); MISSEL, Timo, 55216 INGELHEIM AM RHEIN (DE); NAR, Herbert, 55216 INGELHEIM AM RHEIN (DE); OOST, Thorsten, 55216 INGELHEIM AM RHEIN (DE); REISER, Ulrich, 55216 INGELHEIM AM RHEIN (DE); SCHMIDT, Esther, 55216 INGELHEIM AM RHEIN (DE); TREU, Matthias, 55216 INGELHEIM AM RHEIN (DE)
(74) Representative: Boehringer Global Patents
(86) International application number: PCT/EP2023/079580
(87) International publication number: WO 2024/089006

(56) References cited:
- EP-A1- 3 868 764
- WO-A1-2020/010092
- DING CHUNYONG ET AL: "Small molecules targeting the innate immune cGAS-STING-TBK1 signaling pathway", vol. 10, no. 12, 1 December 2020 (2020-12-01), pages 2272 - 2298, XP055944009, ISSN: 2211-3835, Retrieved from the Internet <URL:https://www.ncbi.nlm.nih.gov/pmc/articles/PMC7745059/pdf/main.pdf> DOI: 10.1016/j.apsb.2020.03.001

## Description

### FIELD OF THE INVENTION

The present invention relates to small molecules capable of activating STING (Stimulator of Interferon Genes), and their salts. Specifically, the present invention relates to heterocyclic compounds capable of activating STING. Furthermore, the invention relates to pharmaceutical compositions and combinations comprising these compounds, as well as their use as a medicament. These compounds and pharmaceutical compositions comprising at least one of these compounds may be suitable as a medicament, e.g., for the therapy of cancer such as canine and/or feline cancer, and as vaccine adjuvant, e.g., for use in swine. Therefore, the invention also relates to compounds and pharmaceutical compositions comprising at least one of these compounds for use in treating feline or canine cancer. Any reference to methods of treatment herein are to be understood as compounds for use in such methods.

### BACKGROUND OF THE INVENTION

STING is one of the pattern-recognition receptors (PRRP) which plays a central role in the innate immune system, distinguishing pathogens and host cells by detecting extracellular and intracellular danger signals including damage-associated molecular patterns (DAMP) and pathogen-associated molecular patterns (PAMP). These recognition processes constitute the first line of defense against viral and bacterial infections and malignant cells. However, pathogens, as well as cancer cells, have evolved ways to evade recognition by the immune system. The aim of immunotherapies is thus to initiate an antigen specific immune response or to re-activate a pre-existing response in certain cell types of the immune system against the pathogenic invaders or cancerous cells.

Among the PRRPs, STING (also known as TMEM173, MPYS, MITA, ERIS) belongs to the family of nucleic acid sensors and is the adaptor for cytosolic DNA signaling. In mammalian cells, in a healthy state, DNA is compartmentalized in the nucleus. In pathogenic situations, such as invasions of DNA-containing pathogens, or in malignant cells, DNA is present in the cytoplasm. Here, STING is critical for detecting the above-described cytosolic DNA and to induce an immune reaction against the pathogenic event.

Being an innate immune defense mechanism member, STING is expressed in almost all cell types especially endothelial, epithelial and immune cells such as macrophages and dendritic cells. A predominant expression of STING in dogs and cats is observed in spleen, lung, blood, lymph nodes and brain (Zhang et al, Microbial Pathogenesis, 113, 202 - 208, 2017; Zhang et al, Veterinary Immunology and Immunopathology 169, 54 - 62, 2016).

In its basal state, STING exists as a dimer with its N-terminal domain anchored in the ER and the C-terminal domain residing in the cytosol. Cyclic dinucleotides (CDNs), generated by the protein cyclic GMP-AMP Synthase (cGAS) are the natural ligands of STING (Ablasser et al, Nature 498, 380 - 384, 2013). Binding of CDNs to STING induces conformational changes which allows the binding and activation of the TANK binding kinase (TBK1) and interferon regulatory factor 3 (IRF3), followed by the relocalization from the ER to perinuclear endosomes (Liu et al, Science 347, Issue 6227, 2630-1 - 2630-14, 2015). Phosphorylation of the transcription factor IRF3 and NF-kB by TBK1 results in expression of multiple cytokines, including type I interferon (IFN).

Type I IFN production by antigen presenting cells, and other cell types, is considered a key event in the activation of T cells and thereby the differentiation of antigen specific effector CD4 and CD8 T cells. It was shown that the lack of type I IFN resulted in a reduced T cell dependent immune response against viral infections or tumor cells (Zitvogel et al, Nature Reviews Immunology 15, 405 - 414, 2015). On the other hand, the presence of a type I IFN signature during cancer therapy is associated with increased numbers of tumor infiltrating T cells and potentially favorable clinical outcome (Sistigu et al, Nature Medicine 20, 1301-1309, 2014).

The anti-tumor impact of type I interferons has been investigated *in vitro* and *in vivo* for feline and canine cancers. Based on field studies where human recombinant interferons were administrated to tumor bearing dogs (lymphoma, fibrosarcoma, osteosarcoma, mycosarcoma, liposarcoma), delayed or prevented local relapses and metastases were documented (Klotz et al, Veterinary Immunology and Immunopathology 191, 80 - 93, 2017).

Efficient secretion of type I IFN in the tumor microenvironment and the induction of a T cell dependent immune response against cancer cells depends on the presence of STING, as shown in recent studies in mice (Woo et al, Immunity 41, 5, 830 - 842, 2014; Corrales et al, Cell Reports 11, 1018 - 1030, 2015; Deng et al, Immunity 41, 5, 843 - 852, 2014). The deletion of STING resulted in reduced type I IFN levels in the tumor microenvironment and in a reduced anti-tumor effect in several mouse tumor models, thereby highlighting the importance of the presence of type I IFN. On the other hand, the specific activation of STING resulted in an improved, antigen specific T cell immune response against cancer cells.

Type I interferons can significantly enhance anti-tumor immune responses by inducing activation of both the adaptive and the innate immune cells.

Given the importance of type I IFN in several malignancies including viral infections and cancer therapy, strategies that allow the specific activation of STING are of therapeutic interest. STING activation may be synergistic with various approved chemotherapeutic agents or other anti-cancer therapies such as radiotherapy (Wu et al., Med Res Rev 2020 May;40(3):1117-1141) or with infectious disease therapies.

In the prior art, small molecule modulators of STING are for example described in WO 2020/075790, EP3868764, WO2020010092 and Acta Pharm. Sin. B, vol. 10, no. 12, 1 December 2020 (2020-12-01), pages 2272-2298.

### SUMMARY OF THE INVENTION

Compounds according to the present invention are novel activators of STING as demonstrated in an *ex vivo* system using canine whole blood.

In one aspect, the present invention relates to a compound of formula (I) 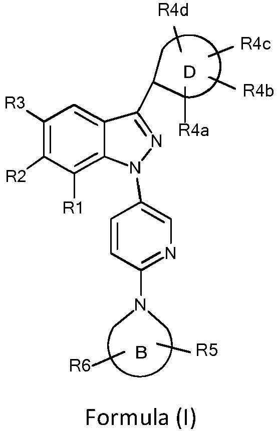 wherein
**B** is a group selected from among the group consisting of
a 5-7-membered monocyclic heterocyclyl containing 1 or 2 N-atoms,
a 6-membered bicyclic heterocyclyl containing 1 N-atom,
a 7-11 membered bicyclic heterocyclyl containing 1 or 2 N-atoms,
a 7-membered bicyclic heterocyclyl containing 1 N-atom and 1 O-atom,
a 6-membered monocyclic heterocyclyl containing 1 N-atom and 1 heteroatom selected from among the group consisting of O and S,
a 9-membered bicyclic heterocyclyl containing 3 heteroatoms, 2 of which are N and the other is O,
a 9-membered bicyclic heterocyclyl containing 1 N-atom and 1 S-atom,
a 10-membered bicyclic heterocyclyl containing 3 N-atoms, 2 of which are substituted with C₁-₆-alkyl,
phenyl,
a 9-membered bicyclic heteroaryl containing 3 N-atoms,
-C₁₋₄-alkylene-pyrimidine, and
-C₁-₄-alkylene-O-C₁-₃-alkyl;
**D** is a group selected from among the group consisting of
a 9-membered bicyclic heteroaryl containing 2 N-atoms,
a 10-membered bicyclic heteroaryl containing 1 N-atom, and
benzodioxole;
**R¹** is selected from among the group consisting of -H **or** -C₁-₆-alkyl;
**R²** is selected from among the group consisting of -H, halogen, preferably fluorine or chlorine, more preferably fluorine, **and** -C₁-₆-alkyl;
**R³** is selected from among the group consisting of -H, halogen, preferably fluorine or chlorine, more preferably fluorine, **and** -C₁-₆-alkyl;
**R^{4a}, R^{4b}** and **R^{4c}** are each independently selected from -H, halogen, preferably fluorine or chlorine, more preferably fluorine, and C₁₋₆-alkyl, with the proviso that at least one of **R^{4a}, R^{4b}** and **R^{4c}** is halogen;
**R^{4d}** is selected from -C₁-₆-alkyl and C₃-₆ cycloalkyl;
**R⁵** is absent **or** is selected from among the group consisting of -H, -C₁-₆-alkyl, -S(O₂)-C₁-₆-alkyl, -NH-S(O₂)-C₁-₆-alkyl, =O, -C(O)-C₁-₆-alkyl, -C(O)H, -C(O)OH, -C(O)NH₂, -C(O)O-C₁-₆-alkyl, -NR^{5.1}R^{5.2}, -C₁-₆-alkylene-C(O)OH, -S(O₂)-NH₂, -pyrolidin-2-one-1-yl, -tetrazolyl, **and** a 5-membered heteroaryl with 1 or 2 heteroatoms selected from among the group consisting of N and O which is substituted with R^{5.3};
**R^{5.1}** is selected from among the group consisting of -H, -C₁-₆-alkyl, -C(O)-C₁-₆-alkyl **and** -C₁-₆-alkylene-O-C₁-₆-alkyl;
**R^{5.2}** is selected from among the group consisting of -H, -C₁-₆-alkyl, -C(O)-C₁-₆-alkyl **and** -C₁-₆-alkylene-O-C₁-₆-alkyl;
**R^{5.3}** is selected from among the group consisting of -H, -C₁-₆-alkyl **and** a 6-membered heteroaryl with 1 or 2 heteroatoms selected from a group consisting of N and O;
**R⁶** is absent **or** is selected from among the group consisting of -H, -C₁-₆-alkyl, =O **and** -C(O)OH;
or a pharmaceutically acceptable salt thereof.

In another aspect, the invention relates to a pharmaceutical composition comprising at least one compound according to formula (I) or a pharmaceutically acceptable salt thereof and a pharmaceutically acceptable carrier.

In another aspect, the invention relates to a compound according to formula (I) or a pharmaceutically acceptable salt thereof or a pharmaceutical composition including the same for use as a medicament.

In another aspect, the invention relates to a compound according to formula (I) or a pharmaceutically acceptable salt thereof or a pharmaceutical composition including the same for use in the treatment of feline or canine cancer.

### DETAILED DESCRIPTION OF THE INVENTION

The compounds of the present invention exhibit several advantageous properties, such as favorable binding affinity to STING from various mammalian species, e.g., cat, mouse, swine and dog, especially well to dog STING, and favorable cellular activity as measured by cellular EC₅₀, i.e., in canine whole blood.

Thus, in a further aspect the invention provides new compounds of formula (I), including salts thereof, which activate STING and therefore induce cytokine production in STING-dependent fashion *in vitro* and/or *in vivo,* e.g., in dogs, and possess suitable pharmacological and pharmacokinetic properties for use in therapy, i.e., for use as medicaments.

Binding of compounds to proteins can be determined by known methods such as surface plasmon resonance, scintillation proximity assay, isothermal titration calorimetry or differential scanning fluorimetry. In the latter test, the temperature at which a protein unfolds, also called the melting temperature Tₘ, is measured by changes in fluorescence of a dye that binds to the hydrophobic parts of the protein. Tₘ shifts upon binding of a small molecule are correlated with the binding affinity of this small molecule. A high binding affinity of a STING agonist is reflected by a shift in Tₘ of >10 °C, preferably >13 °C, more preferably > 15 °C. When measured with a binding assay, the compounds in accordance with the invention preferably show an interaction with dog STING (dSTING) reflected by a shift in Tₘ of > 15 °C, more preferably > 20 °C, and even more preferably > 25 °C, as determined by DSF.

Since STING has been demonstrated to stimulate production of type I interferons, such as interferon beta (IFNb), in myeloid and dendritic cells, the potency of STING agonists can be evaluated in a canine whole blood (cWB) assay with IFNb secretion as a readout. In this assay, freshly collected canine blood is incubated with a STING agonist, and interferon beta levels in the supernatant are quantified by ELISA. The compounds according to the present invention typically show a cellular EC50 of below 10 µM, preferably below 5 µM, more preferably below 1 µM, most preferably below 0.5 µM. In accordance with the invention, a combination of a high dDSF and low dWB is especially favorable.

The compounds of the invention according to general formula (I) 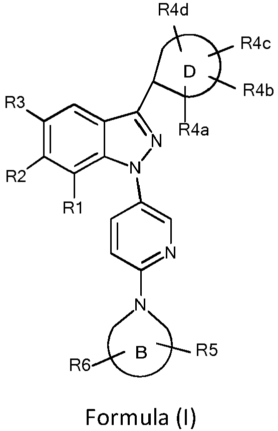 wherein
**B** is a group selected from among the group consisting of
a 5-7-membered monocyclic heterocyclyl containing 1 or 2 N-atoms,
a 6-membered bicyclic heterocyclyl containing 1 N-atom,
a 7-11 membered bicyclic heterocyclyl containing 1 or 2 N-atoms,
a 7-membered bicyclic heterocyclyl containing 1 N-atom and 1 O-atom,
a 6-membered monocyclic heterocyclyl containing 1 N-atom and 1 heteroatom selected from among the group consisting of O and S,
a 9-membered bicyclic heterocyclyl containing 3 heteroatoms, 2 of which are N and the other is O,
a 9-membered bicyclic heterocyclyl containing 1 N-atom and 1 S-atom,
a 10-membered bicyclic heterocyclyl containing 3 N-atoms, 2 of which are substituted with C₁-₆-alkyl,
phenyl,
a 9-membered bicyclic heteroaryl containing 3 N-atoms,
-C₁₋₄-alkylene-pyrimidine, and
-C₁-₄-alkylene-O-C₁-₃-alkyl;
**D** is a group selected from among the group consisting of
a 9-membered bicyclic heteroaryl containing 2 N-atoms,
a 10-membered bicyclic heteroaryl containing 1 N-atom, and benzodioxole;
**R¹ is** selected from among the group consisting of -H **or** -C₁-₆-alkyl;
**R²** is selected from among the group consisting of -H, halogen, preferably fluorine or chlorine, more preferably fluorine, **and** -C₁-₆-alkyl;
**R³** is selected from among the group consisting of -H, halogen, preferably fluorine or chlorine, more preferably fluorine, **and** -C₁-₆-alkyl;
**R^{4a}, R^{4b}** and **R^{4c}** are each independently selected from -H, halogen, preferably fluorine or chlorine, more preferably fluorine, and C₁₋₆-alkyl, with the proviso that at least one of **R^{4a}, R^{4b}** and **R^{4c}** is halogen;
**R^{4d}** is selected from -C₁-₆-alkyl and C₃-₆ cycloalkyl;
**R⁵** is absent **or** is selected from among the group consisting of -H, -C₁-₆-alkyl, -S(O₂)-C₁-₆-alkyl, -NH-S(O₂)-C₁-₆-alkyl, =O, -C(O)-C₁-₆-alkyl, -C(O)H, -C(O)OH, -C(O)NH₂, -C(O)O-C₁-₆-alkyl, -NR^{5.1}R^{5.2}, -C₁-₆-alkylene-C(O)OH, -S(O₂)-NH₂, -pyrolidin-2-one-1-yl, -tetrazolyl, **and** a 5-membered heteroaryl with 1 or 2 heteroatoms selected from among the group consisting of N and O, substituted with R^{5.3};
**R^{5.1}** is selected from among the group consisting of -H, -C₁-₆-alkyl, -C(O)-C₁-₆-alkyl **and** -C₁-₆-alkylene-O-C₁-₆-alkyl;
**R^{5.2}** is selected from among the group consisting of -H, -C₁-₆-alkyl, -C(O)-C₁-₆-alkyl **and** -C₁-₆-alkylene-O-C₁-₆-alkyl;
**R^{5.3} is** selected from among the group consisting of -H, -C₁-₆-alkyl **and** a 6-membered heteroaryl with 1 or 2 heteroatoms selected from a group consisting of N and O;
**R⁶** is absent **or** is selected from among the group consisting of -H, -C₁-₆-alkyl, =O **and** -C(O)OH;
or a pharmaceutically acceptable salt thereof are particularly suitable for the treatment of pathophysiological processes associated with or modulated by STING, particularly for the treatment of cancer, for example feline or canine cancer or for the use as a vaccine adjuvants in, e.g., swines.

Accordingly, in another aspect, the present invention further relates to compounds of formula (I) as defined herein or pharmaceutically acceptable salts thereof or a pharmaceutical composition comprising at least one compound of formula (I) for use as a medicament.

Another aspect of the invention relates to compounds of formula (I) as defined herein or pharmaceutically acceptable salts thereof or a pharmaceutical composition comprising at least one compound of formula (I) for use in the treatment of feline or canine cancer. Other aspects of the present invention will become apparent to the person skilled in the art directly from the foregoing and following description and examples.

### USED TERMS AND DEFINITIONS

Terms not specifically defined herein should be given the meanings that would be given to them by one of skill in the art in light of the disclosure and the context. As used in the specification, however, unless specified to the contrary, the following terms have the meaning indicated and the following conventions are adhered to.

In the groups, radicals, or moieties defined below, the number of carbon atoms is often specified preceding the group, for example, C₁₋₆-alkyl means an alkyl group or radical having 1 to 6 carbon atoms. In general, in groups like HO, H₂N, (O)S, (O)₂S, NC (cyano), HOOC, F₃C or the like, the skilled artisan can see the radical attachment point(s) to the molecule from the free valences of the group itself. For combined groups comprising two or more subgroups, the last named subgroup is the radical attachment point, for example, the substituent "aryl-C₁₋₃-alkylene" means an aryl group which is bound to a C₁₋₃-alkyl-group, the latter of which is bound to the core or to the group to which the substituent is attached.

In case a compound of the present invention is depicted in the form of a chemical name and as a formula, in case of any discrepancy the formula shall prevail. An asterisk or a wavy line may be used in sub-formulas to indicate the bond which is connected to the core molecule as defined.

For example, the term "3-carboxypropyl-group" represents the following substituent: 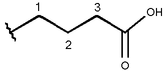 wherein the carboxy group is attached to the third carbon atom of the propyl group. The terms "1-methylpropyl-", "2,2-dimethylpropyl-" or "cyclopropylmethyl-" group represent the following groups:

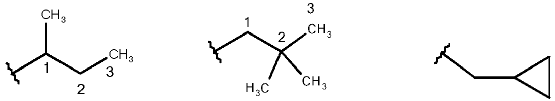

The wavy line may be used in sub-formulas to indicate the bond which is connected to the core molecule as defined. Alternatively, the asterisk may be used in sub-formulas to indicate the bond which is connected to the core molecule as defined.

### 1.1.1.1 Term Substituted

The term "substituted" as used herein, means that one or more hydrogens on the designated atom are replaced by a group selected from a defined group of substituents, provided that the designated atom's normal valence is not exceeded, and that the substitution results in a stable compound. Likewise, the term "substituted" may be used in connection with a chemical moiety instead of a single atom, e.g. "substituted alkyl", "substituted aryl" or the like.

### 1.1.1.2 Stereochemistry-Solvates-Hydrates

Unless specifically indicated, throughout the specification and the appended claims, a given chemical formula or name shall encompass tautomers and all stereo, optical and geometrical isomers (e.g. enantiomers, diastereomers, E/Z isomers etc...) and racemates thereof as well as mixtures in different proportions of the separate enantiomers, mixtures of diastereomers, or mixtures of any of the foregoing forms where such isomers and enantiomers exist, as well as solvates thereof such as for instance hydrates.

Unless specifically indicated, also "pharmaceutically acceptable salts" as defined in more detail below shall encompass solvates thereof such as for instance hydrates.

### 1.1.1.3 Stereoisomers

In general, substantially pure stereoisomers can be obtained according to synthetic principles known to a person skilled in the field, e.g. by separation of corresponding mixtures, by using stereochemically pure starting materials and/or by stereoselective synthesis. It is known in the art how to prepare optically active forms, such as by resolution of racemic forms or by synthesis, e.g. starting from optically active starting materials and/or by using chiral reagents.

Enantiomerically pure compounds of this invention or intermediates may be prepared via asymmetric synthesis, for example by preparation and subsequent separation of appropriate diastereomeric compounds or intermediates which can be separated by known methods (e.g. by chromatographic separation or crystallization) and/or by using chiral reagents, such as chiral starting materials, chiral catalysts or chiral auxiliaries.

Further, it is known to the person skilled in the art how to prepare enantiomerically pure compounds from the corresponding racemic mixtures, such as by chromatographic separation of the corresponding racemic mixtures on chiral stationary phases; or by resolution of a racemic mixture using an appropriate resolving agent, e.g. by means of diastereomeric salt formation of the racemic compound with optically active acids or bases, subsequent resolution of the salts and release of the desired compound from the salt; or by derivatization of the corresponding racemic compounds with optically active chiral auxiliary reagents, subsequent diastereomer separation and removal of the chiral auxiliary group; or by kinetic resolution of a racemate (e.g. by enzymatic resolution); by enantioselective crystallization from a conglomerate of enantiomorphous crystals under suitable conditions; or by (fractional) crystallization from a suitable solvent in the presence of an optically active chiral auxiliary.

### 1.1.1.4 Salts

The phrase "pharmaceutically acceptable" is employed herein to refer to those compounds, materials, compositions, and/or dosage forms which are, within the scope of sound medical judgment, suitable for use in contact with the tissues of mammals without excessive toxicity, irritation, allergic response, or other problem or complication, and commensurate with a reasonable benefit/risk ratio.

As used herein, "pharmaceutically acceptable salt" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like.

For example, such salts include salts from benzenesulfonic acid, benzoic acid, citric acid, ethanesulfonic acid, fumaric acid, gentisic acid, hydrobromic acid, hydrochloric acid, maleic acid, malic acid, malonic acid, mandelic acid, methanesulfonic acid, 4-methylbenzenesulfonic acid, phosphoric acid, salicylic acid, succinic acid, sulfuric acid and tartaric acid.

Further pharmaceutically acceptable salts can be formed with cations from ammonia, L-arginine, calcium, 2,2'-iminobisethanol, L-lysine, magnesium, *N*-methyl-D-glucamine , potassium, sodium and tris(hydroxymethyl)-aminomethane.

The pharmaceutically acceptable salts of the present invention can be synthesized from the parent compound which contains a basic or acidic moiety by conventional chemical methods. Generally, such salts can be prepared by reacting the free acid or base forms of these compounds with a sufficient amount of the appropriate base or acid in water or in an organic diluent such as ether, ethyl acetate, ethanol, isopropanol, or acetonitrile, or a mixture thereof.

Salts of other acids than those mentioned above which for example are useful for purifying or isolating the compounds of the present invention (e.g. trifluoro acetate salts,) also comprise a part of the invention.

### 1.1.1.5 Halogen

The term halogen denotes fluorine, chlorine, bromine and iodine.

### 1.1.1.6 Heteroatoms

Heteroatoms can be present in all the possible oxidation stages. For example, sulphur can be present as sulphoxide (R-S(O)-R') and sulphone (-R-S(O)₂-R').

### 1.1.1.7 Alkyl

The term "C₁₋ₙ-alkyl", wherein n is an integer selected from 2, 3, 4, 5 or 6, preferably 4, 5 or 6, either alone or in combination with another radical denotes an acyclic, saturated, branched or linear hydrocarbon radical with 1 to n C atoms. For example the term C₁₋₅-alkyl embraces the radicals H₃C-, H₃C-CH₂-, H₃C-CH₂-CH₂-, H₃C-CH(CH₃)-, H₃C-CH₂-CH₂-CH₂-, H₃C-CH₂-CH(CH₃)-, H₃C-CH(CH₃)-CH₂-, H₃C-C(CH₃)₂-, H₃C-CH₂-CH₂-CH₂-CH₂-, H₃C-CH₂-CH₂-CH(CH₃)-, H₃C-CH₂-CH(CH₃)-CH₂-, H₃C-CH(CH₃)-CH₂-CH₂-, H₃C-CH₂-C(CH₃)₂-, H₃C-C(CH₃)₂-CH₂-, H₃C-CH(CH₃)-CH(CH₃)- and H₃C-CH₂-CH(CH₂CH₃)-.

### 1.1.1.8 Alkylene

The term "C₁₋ₙ-alkylene" wherein n is an integer selected from 2, 3, 4, 5 or 6, preferably 4, 5 or 6, either alone or in combination with another radical, denotes an acyclic, saturated, branched or linear chain divalent alkyl radical containing from 1 to n carbon atoms. For example the term C₁₋₄-alkylene includes -CH₂-, -CH₂-CH₂-, -CH(CH₃)-, -CH₂-CH₂-CH₂-, -C(CH₃)₂-, -CH(CH₂CH₃)-, -CH(CH₃)-CH₂-, -CH₂-CH(CH₃)-, -CH₂-CH₂-CH₂-CH₂-, -CH₂-CH₂-CH(CH₃)-, -CH(CH₃)-CH₂-CH₂-, -CH₂-CH(CH₃)-CH₂-, -CH₂-C(CH₃)₂-, -C(CH₃)₂-CH₂-, -CH(CH₃)-CH(CH₃)-, -CH₂-CH(CH₂CH₃)-, -CH(CH₂CH₃)-CH₂-, -CH(CH₂CH₂CH₃)- , -CH(CH(CH₃))₂- and -C(CH₃)(CH₂CH₃)-.

### 1.1.1.9 Alkenyl

The term "C₂₋ₘ-alkenyl" is used for a group "C₂₋ₘ-alkyl" wherein m is an integer selected from 3, 4, 5 or 6, preferably 4, 5 or 6, if at least two carbon atoms of said group are bonded to each other by a double bond.

### 1.1.1.10 Alkenylene

The term "C₂₋ₘ-alkenylene" is used for a group "C₂₋ₘ-alkylene" wherein m is an integer selected from 3, 4, 5 or 6, preferably 4, 5 or 6, if at least two carbon atoms of said group are bonded to each other by a double bond.

### 1.1.1.11 Alkynyl

The term "C₂₋ₘ-alkynyl" is used for a group "C₂₋ₘ-alkyl" wherein m is an integer selected from 3, 4, 5 or 6, preferably 4, 5 or 6, if at least two carbon atoms of said group are bonded to each other by a triple bond.

### 1.1.1.12 Alkynylene

The term "C₂₋ₘ-alkynylene" is used for a group "C₂₋ₘ-alkylene" wherein m is an integer selected from 3, 4, 5 or 6, preferably 4, 5 or 6, if at least two of those carbon atoms of said group are bonded to each other by a triple bond.

### 1.1.1.13 Cycloalkyl

The term "C₃₋ₖ-cycloalkyl", wherein k is an integer selected from 3, 4, 5, 6, 7 or 8, preferably 4, 5 or 6, either alone or in combination with another radical denotes a cyclic, saturated, unbranched hydrocarbon radical with 3 to k C atoms. For example the term C₃₋₇-cycloalkyl includes cyclopropyl, cyclobutyl, cyclopentyl, cyclohexyl and cycloheptyl.

### 1.1.1.14 Cycloalkenyl

The term "C₃₋ₖ-cycloalkenyl", wherein k is an integer integer selected from 3, 4, 5, 6, 7 or 8, preferably 4, 5 or 6, either alone or in combination with another radical, denotes a cyclic, unsaturated, but non-aromatic, unbranched hydrocarbon radical with 3 to k C atoms, at least two of which are bonded to each other by a double bond. For example the term C₃₋₇-cycloalkenyl includes cyclopropenyl, cyclobutenyl, cyclopentenyl, cyclopentadienyl, cyclohexenyl, cyclohexadienyl, cycloheptenyl cycloheptadienyl and cycloheptatrienyl.

### 1.1.1.15 Halo-(alkyl, alkylene or cycloalkyl)

The term "halo" added to an "alkyl", "alkylene" or "cycloalkyl" group (saturated or unsaturated) defines an alkyl, alkylene or cycloalkyl group wherein one or more hydrogen atoms are replaced by a halogen atom selected from among fluorine, chlorine or bromine, preferably fluorine and chlorine, particularly preferred is fluorine. Examples include: H₂FC-, HF₂C-, F₃C-.

### 1.1.1.16 Carbocyclyl

The term "carbocyclyl", either alone or in combination with another radical, means a mono-, bi- or tricyclic ring structure consisting of 3 to 14 carbon atoms. The term "carbocyclyl" refers to fully saturated, partially saturated and aromatic ring systems. The term "carbocyclyl" encompasses fused, bridged and spirocyclic systems.

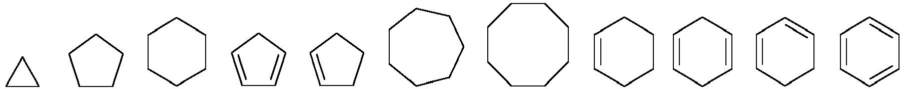

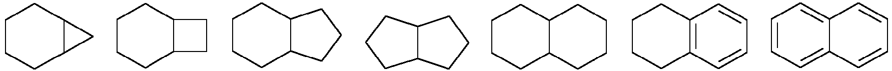

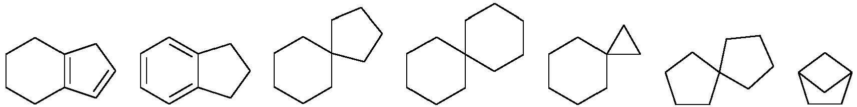

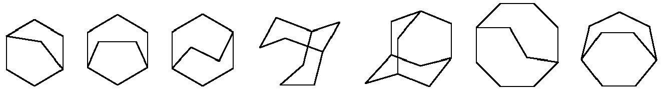

### 1.1.1.17 Aryl

The term "aryl" as used herein, either alone or in combination with another radical, denotes a carbocyclic aromatic monocyclic group containing 6 carbon atoms which is optionally further fused to a second five- or six-membered, carbocyclic group which is aromatic, saturated or unsaturated. Aryl includes, but is not limited to, phenyl, indanyl, indenyl, naphthyl, anthracenyl, phenanthrenyl, tetrahydronaphthyl and dihydronaphthyl.

### 1.1.1.18 Heterocyclyl

The term "heterocyclyl" means a saturated or unsaturated mono- or polycyclic ring system optionally comprising aromatic rings, containing one or more heteroatoms selected from N, O, S, SO, SO₂ , consisting of 3 to 14 ring atoms wherein none of the heteroatoms is part of the aromatic ring. The term "heterocyclyl" is intended to include all the possible isomeric forms.

Thus, the term "heterocyclyl" includes the following exemplary structures (not depicted as radicals as each form is optionally attached through a covalent bond to any atom so long as appropriate valences are maintained):

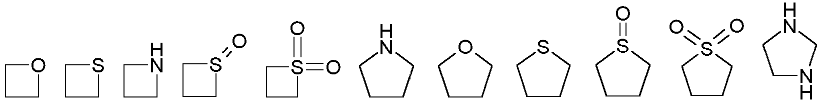

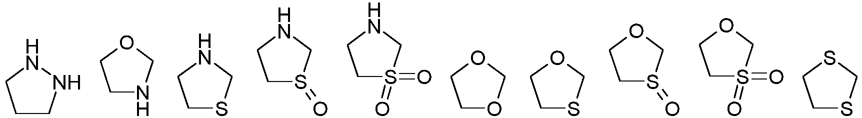

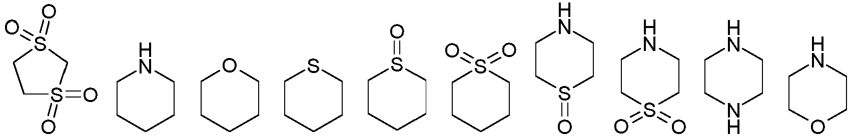

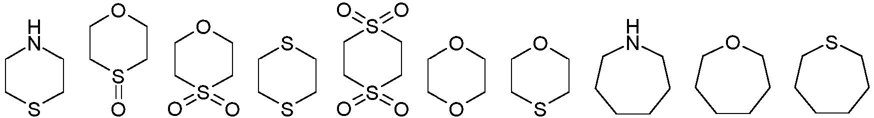

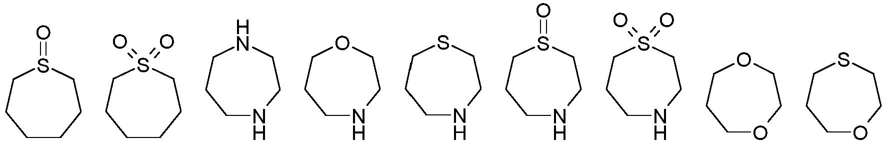

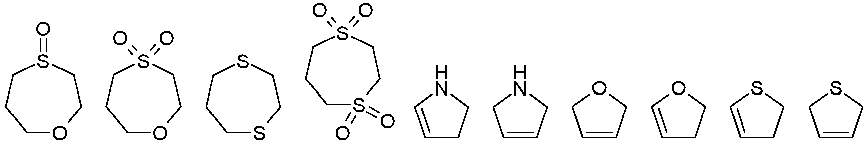

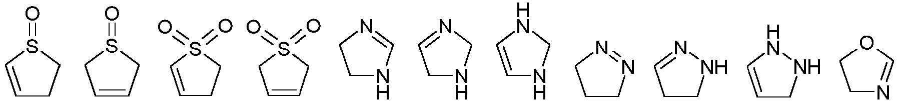

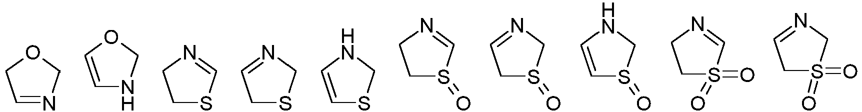

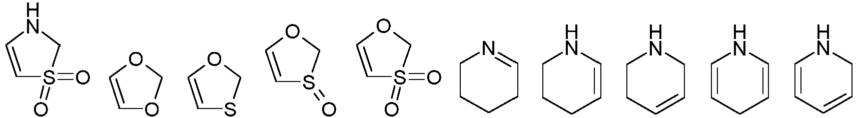

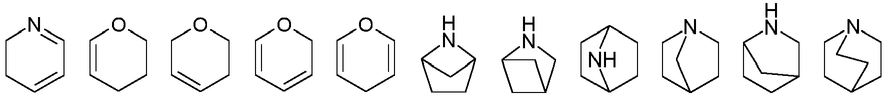

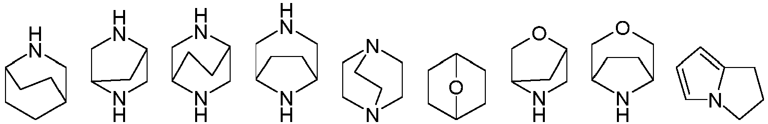

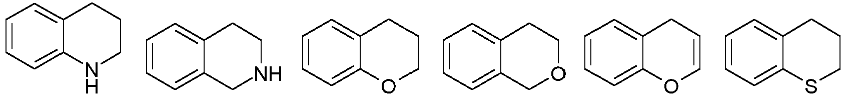

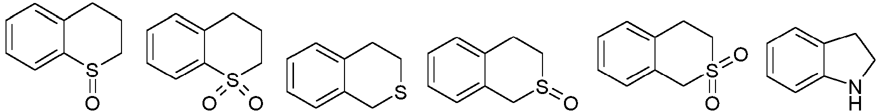

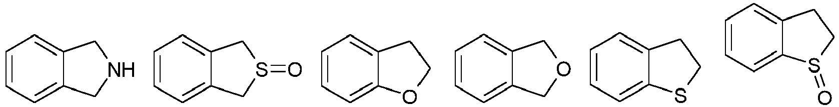

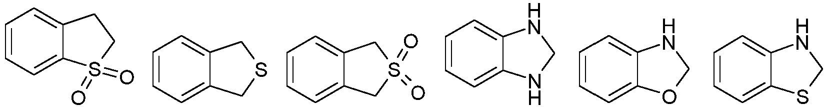

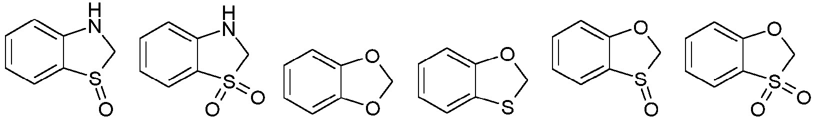

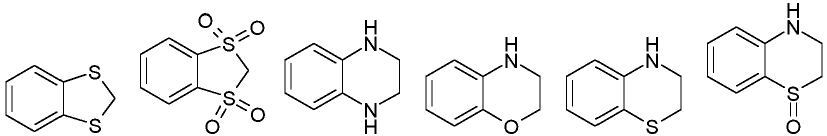

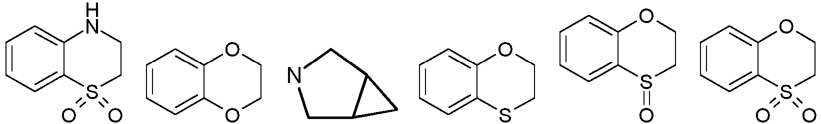

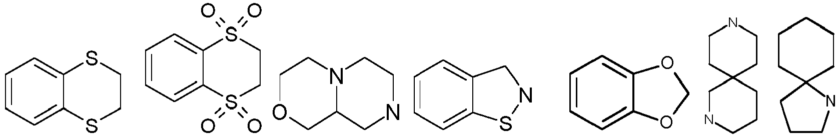

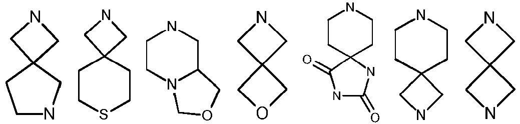

### 1.1.1.19 Heteroaryl

The term "heteroaryl" means a mono- or polycyclic-ring system, comprising at least one aromatic ring, containing one or more heteroatoms selected from N, O, S, SO or SO₂, consisting of 5 to 14 ring atoms wherein at least one of the heteroatoms is part of an aromatic ring, wherein the resulting ring system must be chemically stable. The term "heteroaryl" is intended to include all the possible isomeric forms.

Thus, the term "heteroaryl" includes the following exemplary structures (not depicted as radicals as each form is optionally attached through a covalent bond to any atom so long as appropriate valences are maintained):

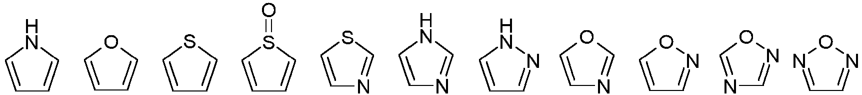

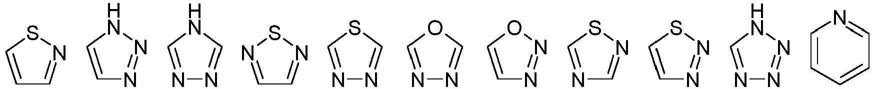

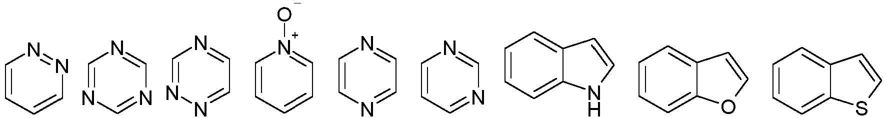

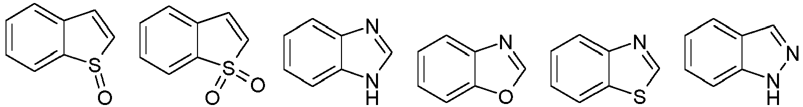

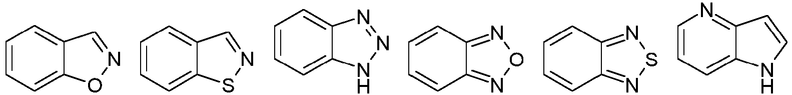

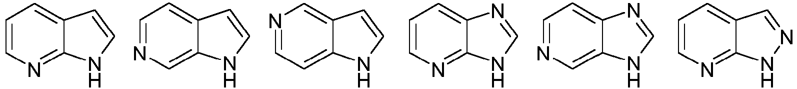

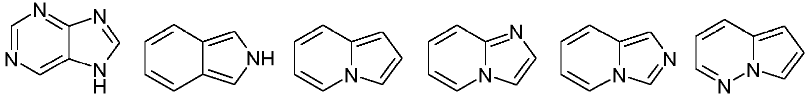

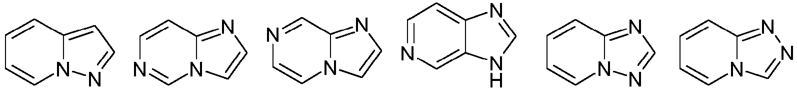

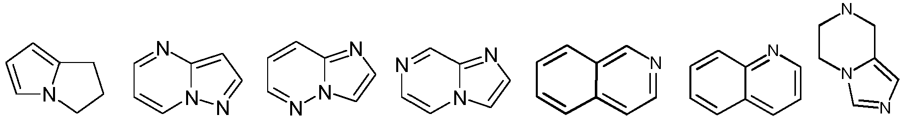

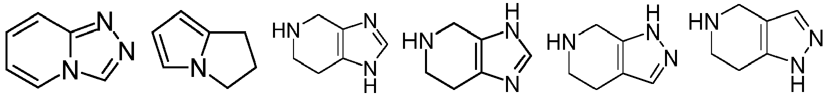

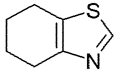

Many of the terms given above may be used repeatedly in the definition of a formula or group and in each case have one of the meanings given above, independently of one another.

The term "bicyclic ring systems" means groups consisting of 2 joined cyclic substructures including spirocyclic, fused, and bridged ring systems.

### PREFERRED EMBODIMENTS

One particularly preferred embodiment of the invention relates to a compound of formula (I) or a pharmaceutically acceptable salt thereof, wherein R¹ is -C₁-₆-alkyl; and at least one of R^{4a}, R^{4b} and R^{4c} is fluorine or chlorine, and the other ones are -H or -C₁-₃-alkyl; and R^{4d} is -C₁-₆-alkyl.

In another particularly preferred embodiment, the invention relates to a compound of formula (I) or a pharmaceutically acceptable salt thereof, wherein D is selected from among the group consisting of 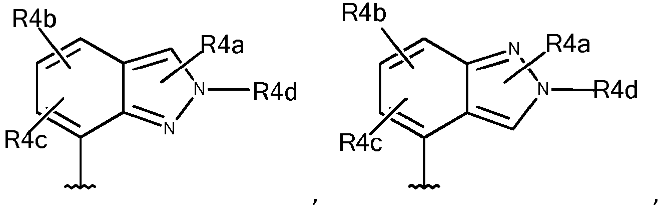 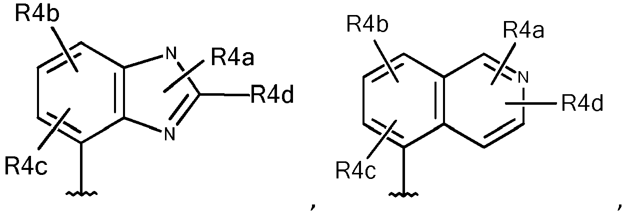 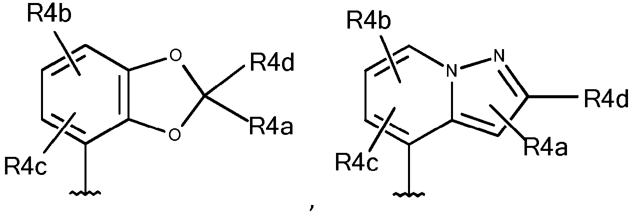 and 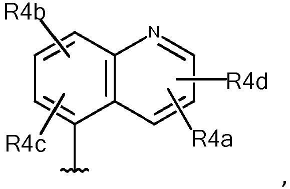 wherein R^{4d} is C₁₋₆-alkyl.

In accordance with the present invention, as far as not indicated in the chemical formula, R^{4a}, R^{4b}, R^{4c} and/or R^{4d} may be attached at any position of the bicyclic structure.

In another particularly preferred embodiment, the invention relates to a compound of formula (I) or a pharmaceutically acceptable salt thereof, wherein D is

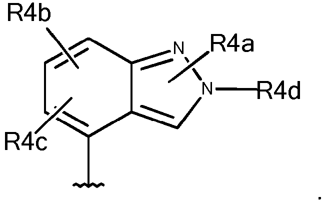

In another particularly preferred embodiment, the invention relates to a compound of formula (I) or a pharmaceutically acceptable salt thereof, wherein D is selected from among the group consisting of the following structures: 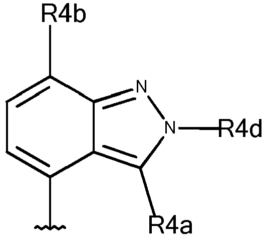
wherein R^{4a} is -H; R^{4b} is halogen, preferably chlorine or fluorine, more preferably fluorine; and R^{4d} is methyl; or
R^{4a} is halogen, preferably chlorine or fluorine, more preferably fluorine; R^{4b} is -H; and R^{4d} is methyl; or
R^{4a} is halogen, preferably chlorine or fluorine, more preferably fluorine; R^{4b} is halogen, preferably chlorine or fluorine, more preferably fluorine; and R^{4d} is methyl;
   or 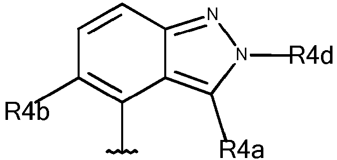
wherein R^{4a} is -H; R^{4b} is halogen, preferably chlorine or fluorine, more preferably fluorine; and R^{4d} is methyl; or
R^{4a} is halogen, preferably chlorine or fluorine, more preferably fluorine; R^{4b} is -H; and R^{4d} is methyl; or
R^{4a} is halogen, preferably chlorine or fluorine, more preferably fluorine; R^{4b} is halogen, preferably chlorine or fluorine, more preferably fluorine; and R^{4d} is methyl;
   or 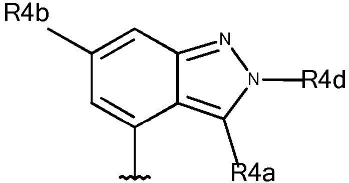
wherein R^{4a} is -H; R^{4b} is halogen, preferably chlorine or fluorine, more preferably fluorine; and R^{4d} is methyl; or
R^{4a} is halogen, preferably chlorine or fluorine, more preferably fluorine; R^{4b} is -H; and R^{4d} is methyl; or
R^{4a} is halogen, preferably chlorine or fluorine, more preferably fluorine; R^{4b} is halogen, preferably chlorine or fluorine, more preferably fluorine; and R^{4d} is -methyl;
   or 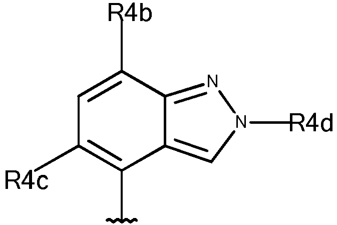
wherein R^{4b} is -H or methyl; R^{4c} is halogen, preferably chlorine or fluorine, more preferably fluorine; and R^{4d} is methyl; or
R^{4b} is halogen, preferably chlorine or fluorine, more preferably fluorine; R^{4c} is -H or methyl; R^{4d} is methyl; or
R^{4b} is halogen, preferably chlorine or fluorine, more preferably fluorine; R^{4c} is halogen, preferably chlorine or fluorine, more preferably fluorine; and R^{4d} is -methyl.

In another particularly preferred embodiment relates to a compound of formula (I) or a pharmaceutically acceptable salt thereof, wherein D is selected from among the group consisting of the following structures: 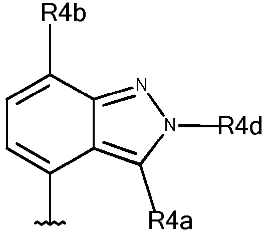
wherein R^{4a} is -H; R^{4b} is halogen, preferably chlorine or fluorine, more preferably fluorine; and R^{4d} is methyl; or
R^{4a} is halogen, preferably chlorine or fluorine, more preferably fluorine; R^{4b} is -H; and
R^{4d} is methyl; or
R^{4a} is halogen, preferably chlorine or fluorine, more preferably fluorine; R^{4b} is halogen, preferably chlorine or fluorine, more preferably fluorine; and R^{4d} is methyl;
   or 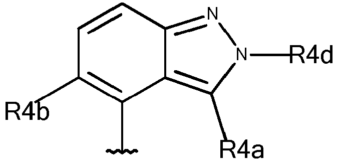
wherein R^{4a} is halogen, preferably chlorine or fluorine, more preferably fluorine; R^{4b} is halogen, preferably chlorine or fluorine, more preferably fluorine; and R^{4d} is methyl;
   or 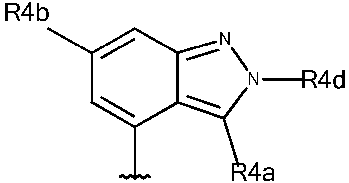
wherein R^{4a} is halogen, preferably chlorine or fluorine, more preferably fluorine; R^{4b} is halogen, preferably chlorine or fluorine, more preferably fluorine; and R^{4d} is -methyl;
   or 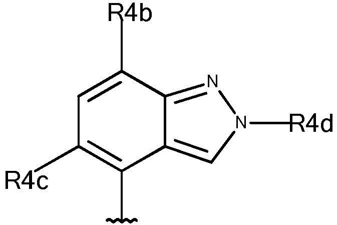
wherein R^{4b} is halogen, preferably chlorine or fluorine, more preferably fluorine; R^{4c} is halogen, preferably chlorine or fluorine, more preferably fluorine; and R^{4d} is -methyl.

In another particularly preferred embodiment, the invention relates to a compound of formula (I) or a pharmaceutically acceptable salt thereof, wherein D is 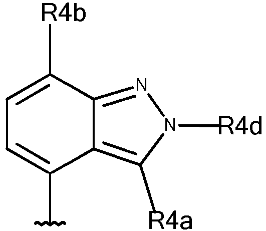 wherein R^{4a} is fluorine; R^{4b} is -H; and R^{4d} is methyl.

In another particularly preferred embodiment, the invention relates to a compound of formula (I) or a pharmaceutically acceptable salt thereof, wherein R¹ is methyl; R² is -H or halogen; and R³-H or halogen.

In another particularly preferred embodiment, the invention relates to a compound of formula (I) or a pharmaceutically acceptable salt thereof, wherein
R¹ is methyl, R² is -H; and R³ is -H; or
R¹ is methyl, R² is -H; and R³ is fluorine; or
R¹ is methyl, R² is fluorine; and R³ is -H.

In another particularly preferred embodiment, the invention relates to a compound of formula (I) or a pharmaceutically acceptable salt thereof, wherein B is selected from among the group consisting of 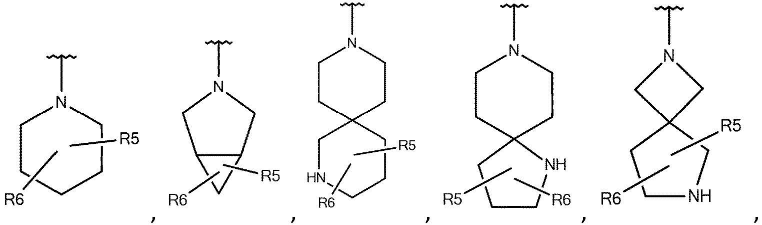 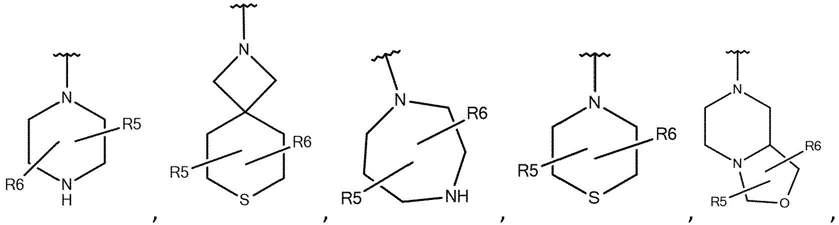 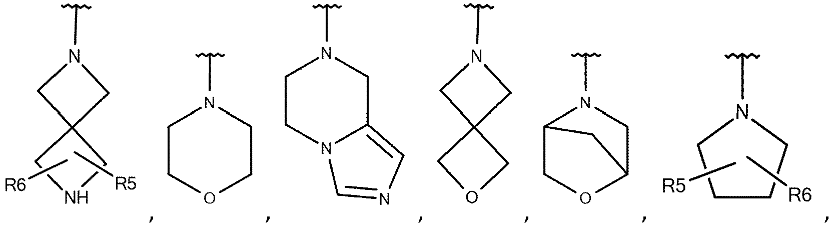 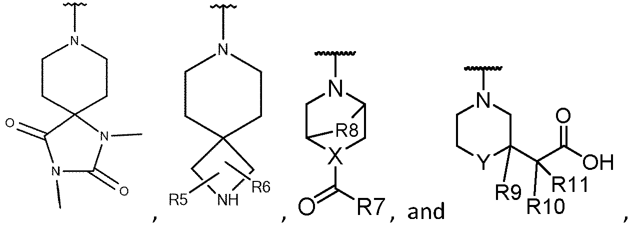 wherein R⁷ is selected from H, -C₁-₆-alkyl, -C₃-₆-cycloalkyl and -OH; R⁸ is (CH₂)ₙ, wherein n is an integer of 1-3, preferably 1 or 2; R⁹ is selected from among the group consisting of H, -C₁-₆-alkyl, and -C₃-₆-cycloalkyl; R¹⁰ is selected from among the group consisting of H, -C₁-₆-alkyl, and -C₃-₆-cycloalkyl; R¹¹ is selected from among the group consisting of H, -C₁-₆-alkyl, and -C₃-₆-cycloalkyl; X is CH or N; and Y is -O-, -S-, -S(O)-, -S(O)₂-. In this embodiment, it is further preferred that R⁷ is not -OH if X is N.

In another particularly preferred embodiment, the invention relates to a compound of formula (I) or a pharmaceutically acceptable salt thereof, wherein B is selected from among the group consisting of 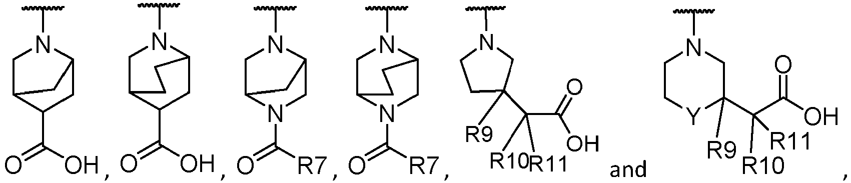 wherein R⁷ is selected from among the group consisting of -H and -C₁-₆-alkyl; R⁹ is selected from among the group consisting of -H and methyl, and is preferably -H; R¹⁰ is selected from among the group consisting of -H and methyl, and is preferably -H; R¹¹ is selected from among the group constating of -H and methyl, and is preferably -H; and Y is O.

Further particularly preferred compounds of the invention are: 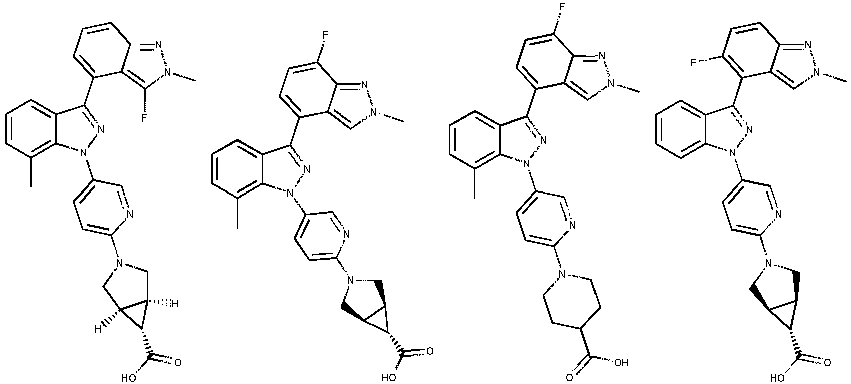 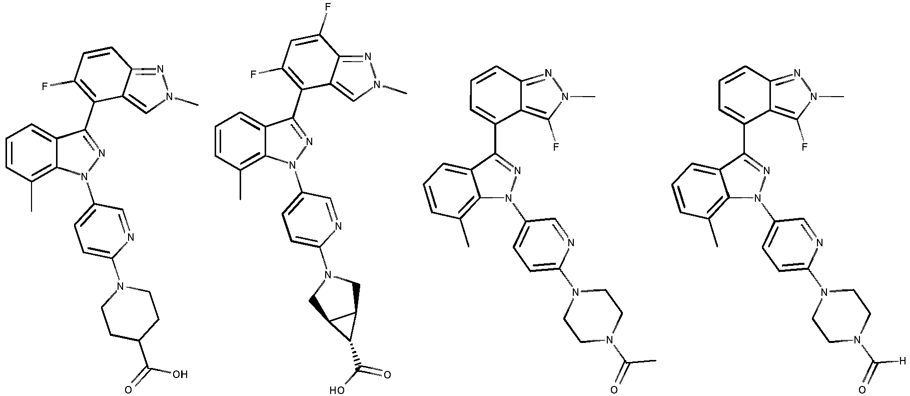 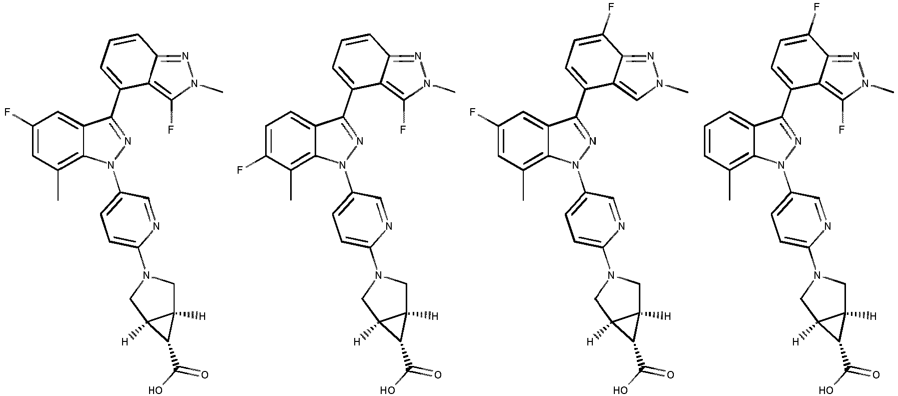 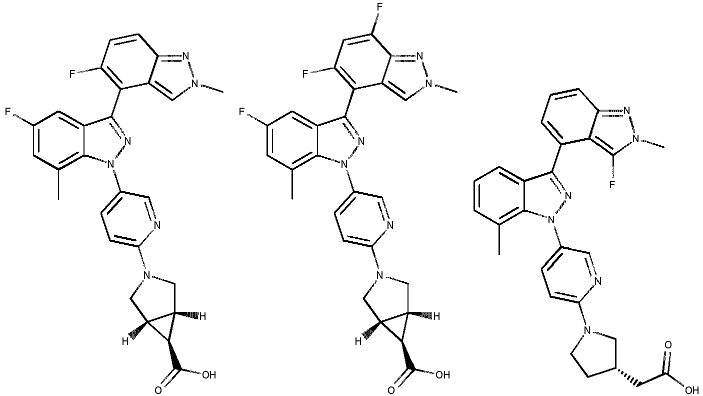 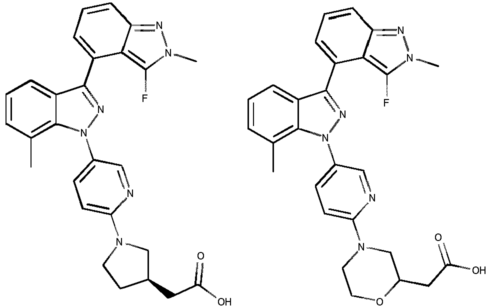 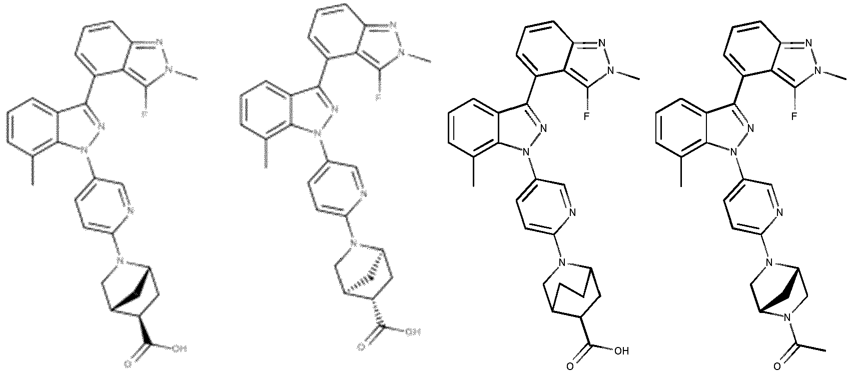 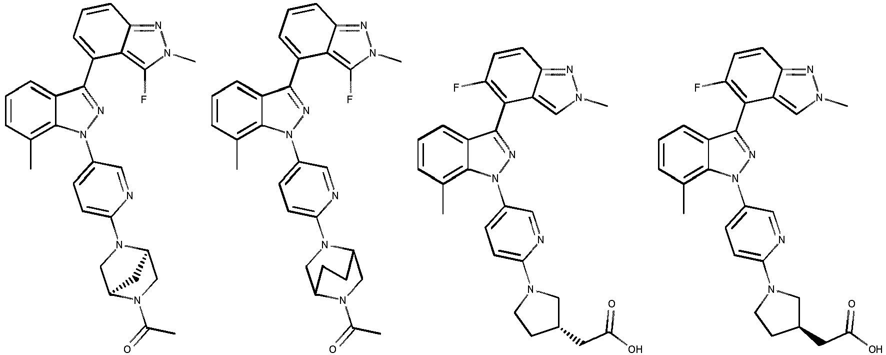 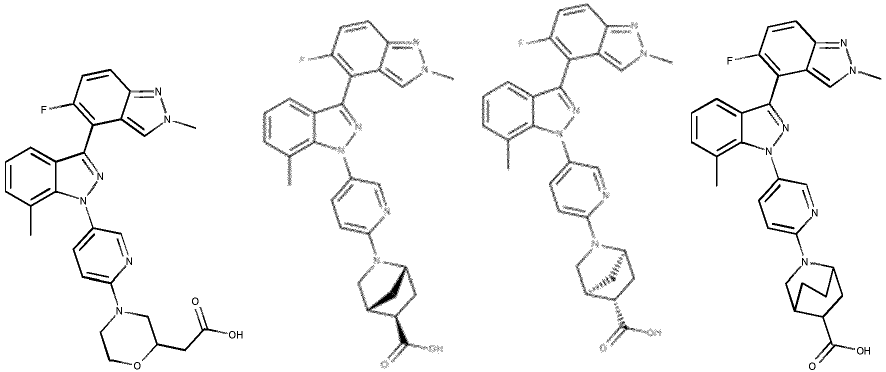 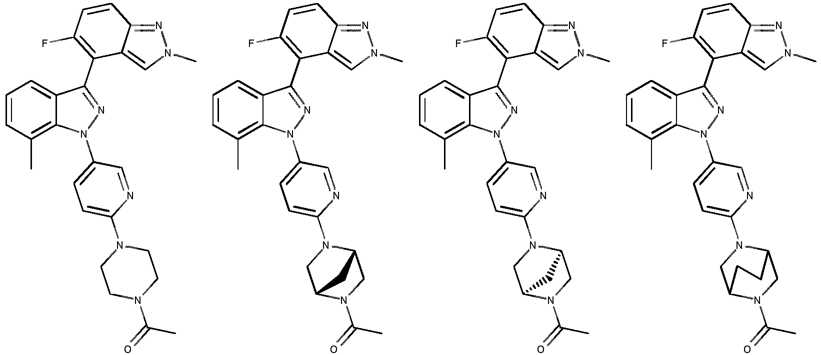 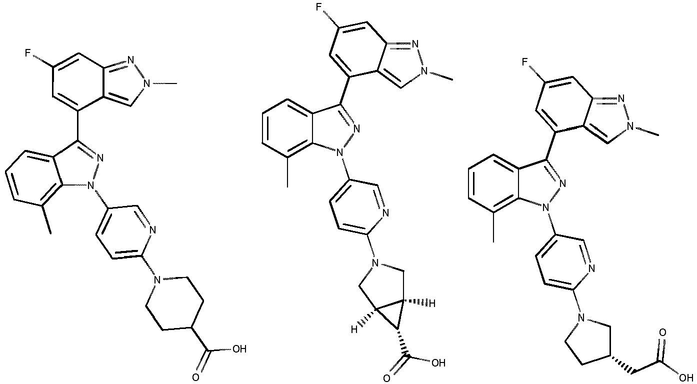 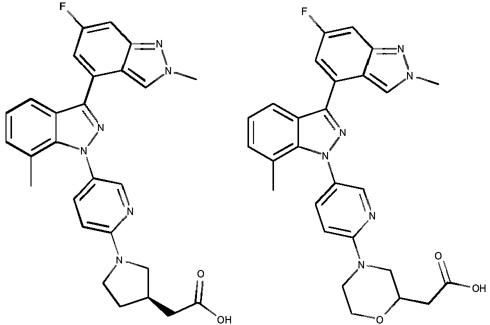 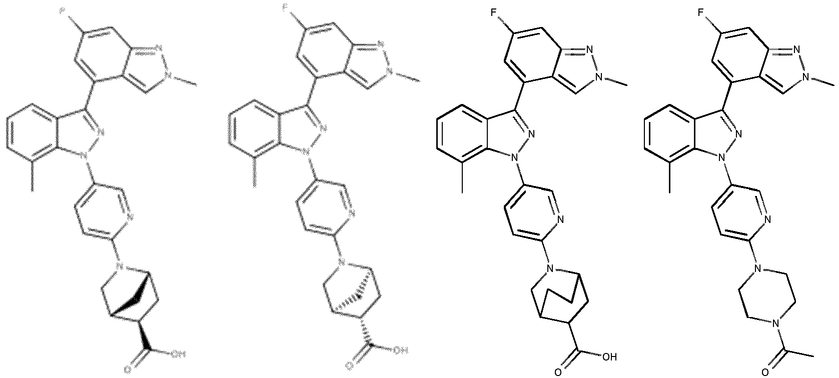 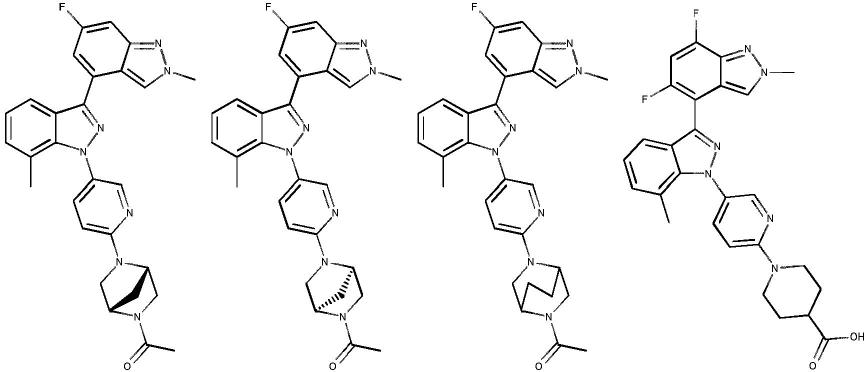 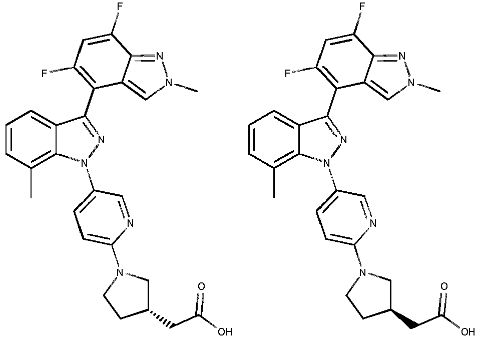 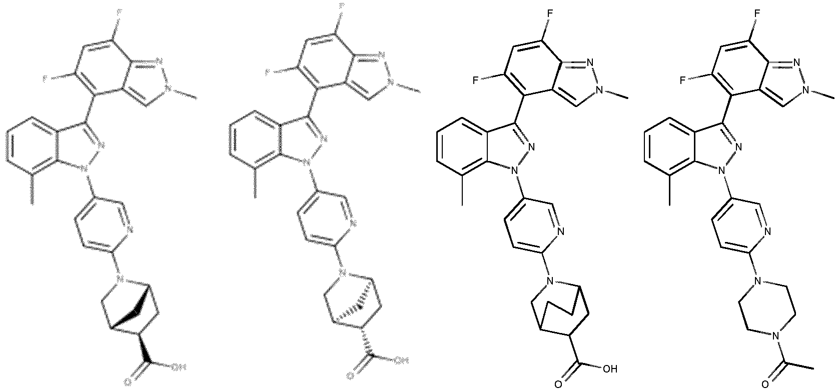 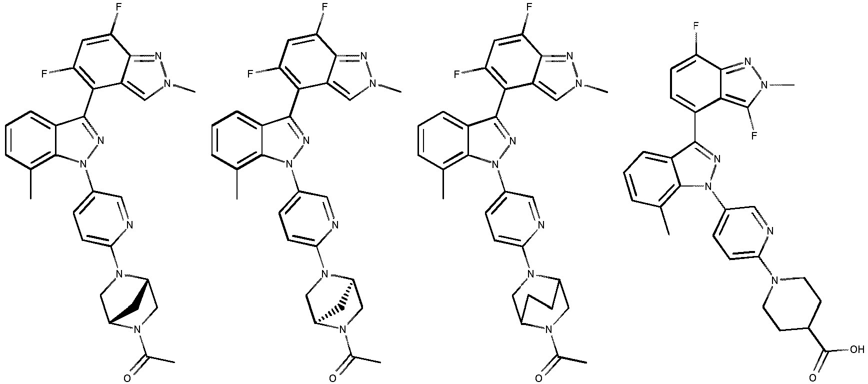 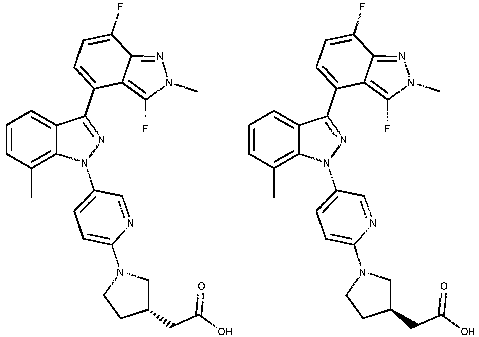 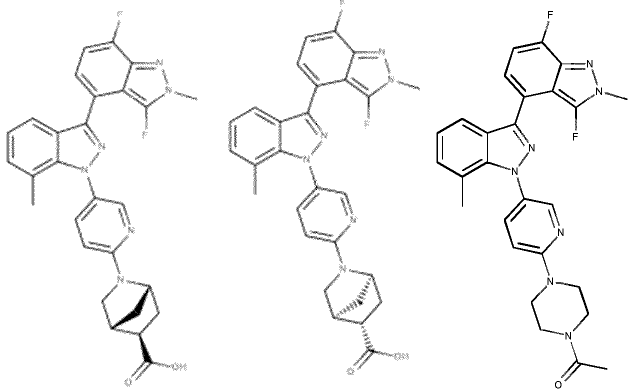 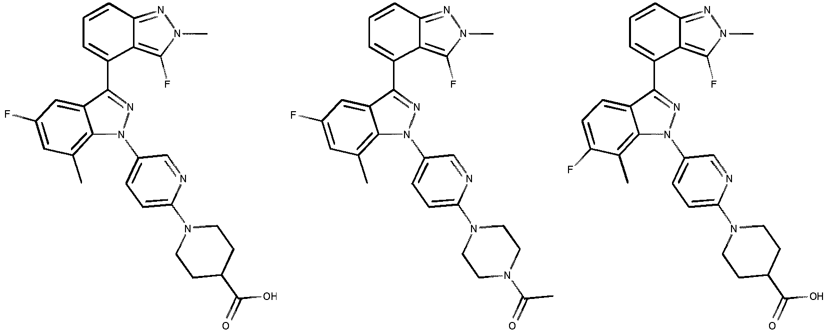 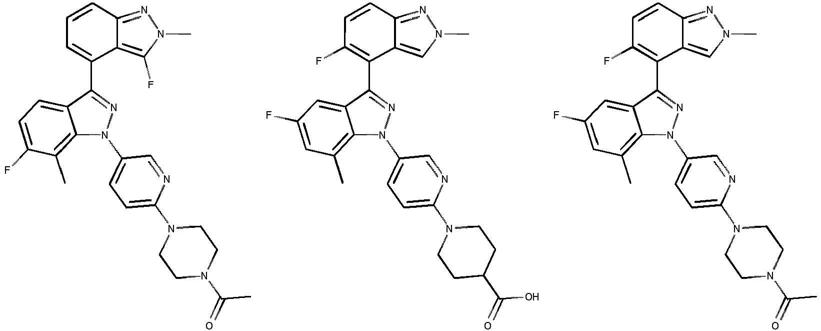 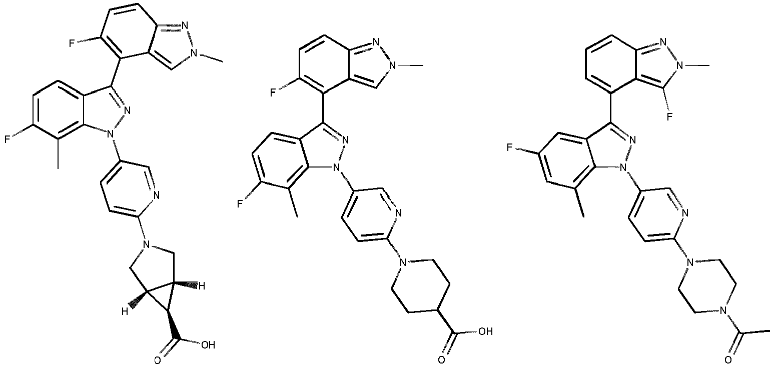 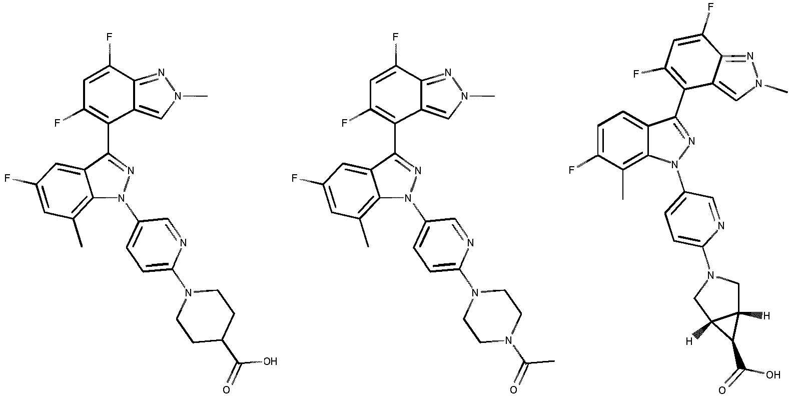 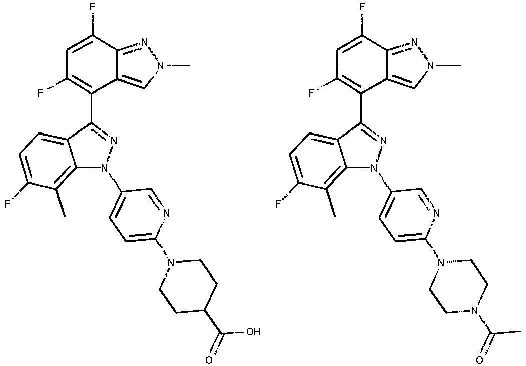 or a pharmaceutically acceptable salt thereof.

In one embodiment, the invention relates to compounds of formula (I) in their salt free forms. In another embodiment, the invention relates to compounds of formula (I) in form of pharmaceutically acceptable salts.

Any and each of the definitions of B, D, R¹, R², R³, R^{4a}, R^{4b}, R^{4c}, R^{4d}, R⁵, R^{5.1}, R^{5.2}, R^{5.3}, R⁶, R⁷, R⁸, R⁹, R¹⁰, R¹¹, X and Y identified above for formula (I) may be combined with each other.

In one aspect, the invention relates to a pharmaceutical composition comprising at least one compound according to formula (I) or a pharmaceutically acceptable salt thereof and a pharmaceutically acceptable carrier.

It is found that compounds of formula (I) or pharmaceutically acceptable salts thereof may be useful in the prevention and/or for the treatment of diseases and/or conditions wherein the modulation of STING is of therapeutic benefit. Thus, in another aspect, the invention relates to a compound of formula (I) or a pharmaceutically acceptable salt thereof or a pharmaceutical composition comprising at least one compound of formula (I) for use as a medicament. In one aspect, the invention relates to compounds of formula (I),a pharmaceutically acceptable salt thereof or a pharmaceutical composition comprising at least one of these compounds for use in the treatment of feline or canine cancer.

In another aspect, the compounds in accordance with the invention show an interaction with dog STING (dSTING) reflected by a shift in Tₘ of > 15 °C, more preferably > 20 °C, and even more preferably > 25 °C, as determined by DSF.

In a preferred embodiment, the compounds in accordance with the present invention show an interaction with dSTING, as determined by DSF (dDSF) and also induce cytokine secretion in dog whole blood (dWB).

In a more preferred embodiment, the compounds in accordance with the invention show a combination of a high dDSF and low dWB.

### METHOD OF TREATMENT

In one aspect, the invention relates to the use of a compound of formula (I), a pharmaceutically acceptable salt thereof or a pharmaceutical composition comprising at least one of these compounds in a method of treating a disease.

In particular, the compounds of general formula (I) or salts thereof are useful in the prevention and/or for the treatment of diseases and/or conditions in mammals, for example in cats, mice, swines and dogs, wherein the modulation of STING is of therapeutic benefit. Furthermore, due to their activity the compounds of the present invention are suitable as vaccine adjuvants.

Diseases and conditions associated with or modulated by STING embrace, but are not limited to inflammation, allergic or autoimmune diseases, for example allergic rhinitis or asthma, infectious diseases or cancer.

Autoimmune diseases include, but are not limited to systemic lupus erythematosus, psoriasis, insulin-dependent diabetes mellitus (IDDM), dermatomyositis and Sjogren's syndrome (SS).

The compounds of the invention may be used to treat inflammation of any tissue and organs of the body, including but not limited to musculoskeletal inflammation, vascular inflammation, neural inflammation, digestive system inflammation, ocular inflammation, inflammation of the reproductive system, and other inflammation.

Examples of musculoskeletal inflammation which may be treated with compounds of the invention include arthritis (including, for example, osteoarthritis, rheumatoid arthritis, psoriatic arthritis, ankylosing spondylitis, acute and chronic infectious arthritis, arthritis associated with gout and pseudogout, and juvenile idiopathic arthritis), tendonitis, synovitis, tenosynovitis, bursitis, fibrositis (fibromyalgia), epicondylitis, myositis, and osteitis (including, for example, Paget's disease, osteitis pubis, and osteitis fibrosa cystic). Examples of ocular inflammation which may be treated with the compounds of the invention include blepharitis, blepharochalasis, conjunctivitis, dacryoadenitis, keratitis, keratoconjunctivitis sicca (dry eye), scleritis, trichiasis, and uveitis.

Examples of inflammation of the nervous system which may be treated with the compounds of the invention include encephalitis, Guillain-Barre syndrome, meningitis, neuromyotonia, narcolepsy, multiple sclerosis, myelitis and schizophrenia.

Examples of inflammation of the vasculature or lymphatic system which may be treated with the compounds of the invention include arthrosclerosis, arthritis, phlebitis, vasculitis, and lymphangitis. Examples of inflammatory conditions of the digestive system which may be treated with the compounds of the invention include cholangitis, cholecystitis, enteritis, enterocolitis, gastritis, gastroenteritis, inflammatory bowel disease (such as Crohn's disease and ulcerative colitis), ileitis, and proctitis.

Examples of inflammatory conditions of the reproductive system which may be treated with the compounds of the invention include cervicitis, chorioamnionitis, endometritis, epididymitis, omphalitis, oophoritis, orchitis, salpingitis, tubo-ovarian abscess, urethritis, vaginitis, vulvitis, and vulvodynia.

The compounds may be used to treat autoimmune conditions having an inflammatory component. Such conditions include acute disseminated alopecia universalis, Behcet's disease, Chagas' disease, chronic fatigue syndrome, dysautonomia, encephalomyelitis, ankylosing spondylitis, aplastic anemia, hidradenitis suppurativa, autoimmune hepatitis, autoimmune oophoritis, celiac disease, Crohn's disease, diabetes mellitus type 1, giant cell arteritis, Goodpasture's syndrome, Grave's disease, Guillain-Barre syndrome, Hashimoto's disease, Henoch-Schonlein purpura, Kawasaki's disease, lupus erythematosus, microscopic colitis, microscopic polyarteritis, mixed connective tissue disease, multiple sclerosis, myasthenia gravis, opsoclonus myoclonus syndrome, optic neuritis, Ord's thyroiditis, pemphigus, polyarteritis nodosa, polymyalgia, rheumatoid arthritis, Reiter's syndrome, Sjogren's syndrome, temporal arteritis, Wegener's granulomatosis, warm autoimmune haemolytic anemia, interstitial cystitis, lyme disease, morphea, psoriasis, sarcoidosis, scleroderma, ulcerative colitis, and vitiligo.

The compounds may be used to treat T-cell mediated hypersensitivity diseases having an inflammatory component. Such conditions include contact hypersensitivity, contact dermatitis (including that due to poison ivy), urticaria, skin allergies, respiratory allergies (hayfever, allergic rhinitis) and gluten-sensitive enteropathy (Celiac disease).

Other inflammatory conditions which may be treated with the compounds include, for example, appendicitis, dermatitis, dermatomyositis, endocarditis, fibrositis, gingivitis, glossitis, hepatitis, hidradenitis suppurativa, iritis, laryngitis, mastitis, myocarditis, nephritis, otitis, pancreatitis, parotitis, pericarditis, peritonitis, pharyngitis, pleuritis, pneumonitis, prostatitis, pyelonephritis, and stomatitis, transplant rejection (involving organs such as kidney, liver, heart, lung, pancreas (e.g., islet cells), bone marrow, cornea, small bowel, skin allografts, skin homografts, and heart valve xenografts, serum sickness, and graft vs host disease), acute pancreatitis, chronic pancreatitis, acute respiratory distress syndrome, Sexary's syndrome, congenital adrenal hyperplasis, nonsuppurative thyroiditis, hypercalcemia associated with cancer, pemphigus, bullous dermatitis herpetiformis, severe erythema multiforme, exfoliative dermatitis, seborrheic dermatitis, seasonal or perennial allergic rhinitis, bronchial asthma, contact dermatitis, atopic dermatitis, drug hypersensitivity reactions, allergic conjunctivitis, keratitis, herpes zoster ophthalmicus, iritis and oiridocyclitis, chorioretinitis, optic neuritis, symptomatic sarcoidosis, fulminating or disseminated pulmonary tuberculosis chemotherapy, idiopathic thrombocytopenic purpura in adults, secondary thrombocytopenia in adults, acquired (autoimmune) haemolytic anemia, leukaemia and lymphomas in adults, acute leukaemia of childhood, regional enteritis, autoimmune vasculitis, multiple sclerosis, chronic obstructive pulmonary disease, solid organ transplant rejection, sepsis.

Preferred treatments include treatment of transplant rejection, rheumatoid arthritis, psoriatic arthritis, multiple sclerosis. Type 1 diabetes, asthma, inflammatory bowel disease, systemic lupus erythematosis, psoriasis, chronic pulmonary disease, and inflammation accompanying infectious conditions (e.g., sepsis).

In one aspect the disease or condition to be treated using compounds of the invention is cancer. Examples of cancer diseases and conditions in which compounds of formula (I), or salts or solvates thereof may have potentially beneficial anti-tumor effects include, but are not limited to, cancers of the lung, bone, pancreas, skin, brain, head, neck, uterus, ovaries, stomach, colon, colorectal, breast, esophagus, small intestine, bowel, endocrine system, thyroid gland, parathyroid gland, adrenal gland, urethra, prostate, penis, testes, ureter, bladder, kidney or liver, bile duct; urothelial cancer; rectal cancer; cancer of the anal region; carcinomas of the fallopian tubes, endometrium, cervix, vagina, vulva, renal pelvis, renal cell; sarcoma; sarcoma of soft tissue; myxoma; rhabdomyoma; fibroma; lipoma; teratoma; cholangiocarcinoma; hepatoblastoma; angiosarcoma; hemagioma; hepatoma; fibrosarcoma; chondrosarcoma; myeloma; chronic or acute leukemia; lymphocytic lymphomas; primary CNS lymphoma; neoplasms of the CNS; spinal axis tumours; squamous cell carcinomas; synovial sarcoma; malignant pleural mesotheliomas; brain stem glioma; pituitary adenoma; bronchial adenoma; chondromatous hanlartoma; mesothelioma; Hodgkin's Disease or a combination of one or more of the foregoing cancers.

Preferred cancers, which may be treated with compounds according to the invention, are skin, lung, e.g. small-cell lung cancer, non-small cell lung cancer, liver, pancreas, colon, colorectal, brain, breast, ovary, prostate, kidney, bladder, bile duct, endometrium, thyroid gland, cervix, stomach, head, neck, sarcoma, sarcoma of soft tissue, esophagus, headand-neck-cancer, rectal and urothelial cancer, as well as lymphoma.

The new compounds may be used for the prevention, palliative, curative or semi-curative, short-term or long-term treatment of the above-mentioned diseases, optionally also in combination with surgery, radiotherapy or other "state-of-the-art" compounds, such as e.g. cytostatic or cytotoxic substances, cell proliferation inhibitors, anti-angiogenic substances, steroids, antibodies, nanobodies, cancer-targeting agents, viruses, including but not limited to oncolytic viruses, or immunogenic cell death inducers.

The new compounds may also be used for the prevention, palliative, curative or semi-curative, short-term or long-term treatment of the above-mentioned diseases by combining different administration routes, e.g. intravenous, intratumoral, subcutaneous, inhalative, oral etc. for the compounds, optionally also in combination with surgery, radiotherapy or other "state-of-the-art" compounds, such as e.g. cytostatic or cytotoxic substances, cell proliferation inhibitors, anti-angiogenic substances, steroids, antibodies, nanobodies, cancer-targeting agents, viruses, including but not limited to oncolytic viruses, or immunogenic cell death inducers. Merely as an example of surgery, partial or complete tumor excision may be combined with the compounds of the invention. Merely as an example of radiotherapy, external beam radiotherapy may be combined with the compounds of the invention.

In their role as adjuvants, in certain embodiments the present compounds and compositions may be used as adjuvants in a therapeutic or prophylactic strategy employing vaccine(s). Thus, the compounds of the present invention, or salts thereof, may be used together with one or more vaccines selected to stimulate an immune response to one or more predetermined antigens. The compounds of the present invention, or salts thereof, may be provided together with, or in addition to, such vaccines.

Such vaccine(s) can comprise inactivated or attenuated bacteria or viruses comprising the antigens of interest, purified antigens, live viral or bacterial delivery vectors recombinantly engineered to express and/or secrete the antigens, antigen presenting cell (APC) vectors comprising cells that are loaded with the antigens or transfected with a composition comprising a nucleic acid encoding the antigens, liposomal antigen delivery vehicles, or naked nucleic acid vectors encoding the antigens. This list is not meant to be limiting. By way of example, such vaccine(s) may also comprise an inactivated tumor cell or an oncolytic virus that expresses and secretes one or more of GM-CSF, CCL20, CCL3, IL-12p70, FLT-3 ligand, cytokines.

Accordingly, the present invention relates to a compound of general formula (I) for use as a medicament, e.g., for treating feline or canine cancer, or as vaccine adjuvants for use in, e.g., swines.

In a preferred aspect, the invention relates to a compound of formula (I), a pharmaceutically acceptable salt thereof or a pharmaceutical composition comprising at least one compound of formula (I) for use in the treatment of feline or canine cancer.

In one embodiment, the invention relates to the compound or a pharmaceutically acceptable salt thereof or a pharmaceutical composition comprising at least one of the compounds for use in the treatment of canine cancer, wherein the canine cancer is selected from osteosarcoma (OSA), oral melanoma, B-cell lymphoma, urothelial carcinoma (UC), hemangiosarcoma, mast cell tumor, soft tissue sarcoma, squamous cell carcinoma, T-cell lymphoma, mammary gland adenocarcinoma and anal sac carcinoma.

In another embodiment, the invention relates to the compound or a pharmaceutically acceptable salt thereof or a pharmaceutical composition comprising at least one of the compounds for use in the treatment of feline cancer, wherein the feline cancer is selected from B-cell and/or T-cell lymphoma, squamous cell carcinoma, mammary gland adenocarcinoma, mast cell tumors and injection site sarcoma.

In a further aspect the present invention relates to a method of treatment and/or prevention of the above-mentioned diseases and conditions which comprises administering to a subject an effective amount of a compound of formula (I), a pharmaceutically acceptable salt thereof or a pharmaceutical composition comprising at least one compound of formula (I).

In a particularly preferred embodiment, the present invention relates to a method of treating feline or canine cancer which comprises administering an effective amount of a compound of formula (I), a pharmaceutically acceptable salt thereof or a pharmaceutical composition comprising at least one compound of formula (I) to a feline or canine.

In a more preferred embodiment, the present invention relates to a method of treating canine cancer which comprises administering an effective amount of a compound of formula (I), a pharmaceutically acceptable salt thereof or a pharmaceutical composition comprising at least one compound of formula (I) to a canine, wherein the canine cancer is selected from canine cancer is selected from osteosarcoma (OSA), oral melanoma, B-cell lymphoma, urothelial carcinoma (UC), hemangiosarcoma, mast cell tumor, soft tissue sarcoma, squamous cell carcinoma, T-cell lymphoma, mammary gland adenocarcinoma and anal sac carcinoma.

In another more preferred embodiment, the present invention relates to a method of treating feline cancer which comprises administering an effective amount of a compound of formula (I), a pharmaceutically acceptable salt thereof or a pharmaceutical composition comprising at least one compound of formula (I) to a feline, wherein the feline cancer is selected from B-cell and/or T-cell lymphoma, squamous cell carcinoma, mammary gland adenocarcinoma, mast cell tumors and injection site sarcoma.

In a further aspect, the present invention relates to a compound of general formula (I) for use in the treatment and/or prevention of above-mentioned cancers, before or after tumor excision and/or radiotherapy.

In a further aspect, the present invention relates to the use of a compound of general formula (I) for the preparation of a medicament for the treatment and/or prevention of above- mentioned diseases and conditions.

In another aspect, the present invention relates to a method of treating canine or feline cancer which comprises administering an effective amount of a compound of formula (I), a pharmaceutically acceptable salt thereof or a pharmaceutical composition comprising at least one compound of formula (I) to a canine or feline in combination with radiotherapy.

### PHARMACEUTICAL COMPOSITIONS

In another aspect of the present invention, pharmaceutical compositions comprising at least one of the above-mentioned compounds are provided.

The pharmaceutical compositions may be formulated in a way that they are suitable for the administration of therapeutically effective amounts of said compounds. Suitable preparations for administering the compounds of formula (I) will be apparent to those with ordinary skill in the art and include for example tablets, pills, capsules, suppositories, lozenges, troches, solutions, syrups, elixirs, sachets, injectable solutions (subcutaneously, intravenously, intramuscularly, intra-peritoneal, intra-tumorally and peri-tumorally), inhalables, infusions, elixirs, emulsions, and powders. Furthermore, the compounds according to the invention may be administered via targeted delivery platforms, for example such targeted delivery platforms may be antibody-drug conjugates, nanobody-drug conjugates, peptide-drug conjugates, virus-like particles, or nanoparticle formulations.

Suitable tablets may be obtained, for example, by mixing one or more compounds according to formula I with known excipients, for example inert diluents, carriers, disintegrants, adjuvants, surfactants, binders and/or lubricants.

For the purposes of this disclosure, the pharmaceutical compositions may be administered by a variety of means, including non-parenterally, parenterally, by inhalation spray, topically, nasally, orally, or rectally in formulations containing pharmaceutically acceptable carriers, adjuvants and vehicles. The pharmaceutical compositions of the disclosure may be administered in the form of a sterile injectable preparation, such as a sterile injectable aqueous or oleaginous suspension.

### COMBINATION THERAPY

The compounds of the invention may be used on their own or may be combined with one or more further therapeutic agent(s).

In a further aspect, the invention provides a method of treatment of a disease or condition in which modulation of STING is beneficial comprising administering a therapeutically effective amount of a combination comprising a compound of formula (I), or a pharmaceutically acceptable salt thereof, and at least one further therapeutic agent.

In a further aspect, the invention provides a method of treatment of inflammation, allergic or autoimmune diseases, infectious diseases or cancer comprising administering a therapeutically effective amount of a combination comprising a compound of formula (I), or a pharmaceutically acceptable salt thereof, and at least one further therapeutic agent.

The actual pharmaceutically effective amount or therapeutic dosage will of course depend on factors known by those skilled in the art such as age and weight of the patient, route of administration and severity of disease. In any case the combination will be administered at dosages and in a manner which allows a pharmaceutically effective amount to be delivered based upon patient's unique condition.

In certain embodiments, the compounds and compositions thereof described herein are administered in conjunction with one or more additional compositions including vaccines intended to stimulate an immune response to one or more predetermined antigens; adjuvants; CTLA-4 and PD-1 pathway antagonists, lipids, liposomes, chemotherapeutic agents, immunomodulatory cell lines, cancer-targeting agents, immunogenic cell-death inducers, immuno-modulating agents, wherein the immunomodulating agents may be understood as agents of a general activation-modulation type in general as well as agents modulating and/or increasing the frequency of a certain immune cell subtype, etc..

The compounds and compositions thereof described herein may be administered before, after, and/or simultaneously with an additional therapeutic or prophylactic composition or modality.

The compounds, compositions, including any combinations with one or more additional therapeutic agent(s), according to the invention may be administered by mucosal (e.g. oral, sublingual, vaginal, nasal, cervical, etc.), intra-tumoral, intra-peritoneal, peri-tumoral, transdermal, inhalative, or parenteral (e.g. subcutaneous, intravenous, intramuscular, intraarterial, intradermal, intrathecal and epidural administrations) route.

Furthermore, the compounds, compositions, including any combinations with one or more additional therapeutic agents, according to the invention may be administered via targeted delivery platforms, for example such targeted delivery platforms can be antibody-drug conjugates, nanobody-drug conjugates, peptide-drug conjugates, virus-like particles, or nanoparticles.

Of the possible methods of administration, intra-peritoneal, intra-tumoral, peri-tumoral, subcutaneous, inhalative or intravenous administration is preferred. The compounds, compositions, including any combinations with one or more additional therapeutic agents, according to the invention may also be administered before, after, and/or simultaneously by a combination of different methods of administration. Simply by way of an example, an inhalative or intravenous administration may be followed by an intratumoral or peri-tumoral administration or an intra-tumoral or peri-tumoral administration may be followed by an inhalative or intravenous administration. Additionally, such an administration of the compounds via different routes may be before or after additional therapeutic step, such as tumor excision or radiotherapy.

In a particularly preferred embodiment, a compound of the invention, a pharmaceutically acceptable salt thereof or a pharmaceutical composition comprising at least one compound of the invention is used in combination with radiotherapy. Simply by way of an example, the compounds of the invention may be administered after radiotherapy. Furthermore, the compounds of the invention may be given by intravenous administration after radiotherapy. Furthermore, the compounds of the invention may be given by intravenous administration after tumor excision. Furthermore, the compounds of the invention may be given by intra-tumoral administration after radiotherapy. Furthermore, the compounds of the invention may be given by peri-tumoral administration after radiotherapy. Furthermore, the compounds of the invention may be given by inhalative administration after tumor excision. Furthermore, the compounds of the invention may be given by intravenous administration, followed by intra-tumoral administration, and both administrations take place after radiotherapy. Furthermore, the compounds of the invention may be given by intra-tumoral administration, followed by intravenous administration, and both administrations take place after radiotherapy. Furthermore, the compounds of the invention may be given by intravenous administration, followed by peri-tumoral administration, and both administrations take place after radiotherapy. Furthermore, the compounds of the invention may be given by peri-tumoral administration, followed by intravenous administration, and both administrations take place after radiotherapy.

Methods for co-administration with an additional therapeutic agent are well known in the art.

Because of the adjuvant properties of the compounds of the present invention, their use may also combined with other therapeutic modalities including other vaccines, adjuvants, antigen, antibodies, and immune modulators.

In addition to the compounds of the present invention and compositions thereof described herein, the compositions or methods of the present invention may further comprise one or more additional substances which, because of their nature, can act to stimulate or otherwise utilize the immune system to respond to the cancer antigens present on the targeted tumor cell(s).

The compounds of the present invention can be used in combination with an immune checkpoint inhibitor, such as an immune checkpoint inhibitor selected from the group consisting of a CTLA-4 pathway antagonist, a PD-1 pathway antagonist, a Tim-3 pathway antagonist, a Vista pathway antagonist, a BTLA pathway antagonist, a LAG-3 pathway antagonist, or a TIGIT pathway antagonist.

The compounds of the present invention can be used in combination with an immunooncological agonist in combination with a T-cell receptor agonist, or in combination with a TNF receptor superfamily agonist or antagonist.

The compounds of the present invention can be used in combination with therapeutic antibodies or therapeutic nanobodies. In some embodiments, the mechanism of action of the therapeutic antibody is Antibody-Dependent Cell-Mediated Cytotoxicity (ADCC).

In additional embodiments of the methods described herein, the compounds of the present invention are used in combination with chemotherapeutic agents (e.g. small molecule pharmaceutical compounds) as known to the skilled person. Thus the methods further involve administering to the subject an effective amount of one or more chemotherapeutic agents as an additional treatment or a combination treatment.

Additional pharmacologically active substance(s) which can also be used together/in combination with the compound of formula **(I)** - or a pharmaceutically acceptable salt thereof - (including all individual embodiments or generic subsets of compounds **(I))** or in the medical uses, uses, methods of treatment and/or prevention as herein (above and below) disclosed include, without being restricted thereto: hormones, hormone analogues and antihormones (e.g. tamoxifen, toremifene, raloxifene, fulvestrant, megestrol acetate, flutamide, nilutamide, bicalutamide, aminoglutethimide, cyproterone acetate, finasteride, buserelin acetate, fludrocortisone, fluoxymesterone, medroxyprogesterone, octreotide); aromatase inhibitors (e.g. anastrozole, letrozole, liarozole, vorozole, exemestane, atamestane); LHRH agonists and antagonists (e.g. goserelin acetate, luprolide); inhibitors of growth factors and/or of their corresponding receptors (growth factors are for example: platelet derived growth factor (PDGF), fibroblast growth factor (FGF), vascular endothelial growth factor (VEGF), epidermal growth factor (EGF), insuline-like growth factors (IGF), human epidermal growth factor (HER, *e.g*. HER2, HER3, HER4),) and/or their corresponding receptors; inhibitors are for example (*anti*-)growth factor antibodies, (*anti*-)growth factor receptor antibodies and tyrosine kinase inhibitors, such as for example afatinib, dacomitinib, canertinib, neratinib, avitinib, poziotinib, AV 412, PF-6274484, HKI 357, olmutinib, osimertinib, almonertinib, nazartinib, lazertinib, pelitinib, erlotinib, gefitinib, icotinib, sapitinib, lapatinib, varlitinib, vandetanib, TAK-285, AEE788, BMS599626/AC-480, GW 583340, necitumumab, panitumumab, cetuximab, amivantanab, pertuzumab, trastuzumab, trastuzumab emtansine, or inhibitors of mutant EGFR, an inhibitor of HER2 with exon 20 mutations, and hepatocyte growth factor (HGF, c-MET, e.g. emibetuzumab, amivantanab, savolitinib, cabozantinib, foretinib); antimetabolites (*e.g*. methotrexate, raltitrexed, 5-fluorouracil (5-FU), capecitabine, floxuridine, gemcitabine, mercaptopurine, thioguanine, cladribine, pentostatin, cytarabine (ara C), fludarabine, combination of trifluridine and tipiracil ( = TAS102)); antitumor antibiotics (*e.g*. anthracyclins such as doxorubicin, doxil (pegylated liposomal doxorubicin hydrochloride), myocet (nonpegylated liposomal doxorubicin), daunorubicin, epirubicin and idarubicin, mitomycin-C, bleomycin, dactinomycin, plicamycin, streptozocin); platinum derivatives (*e.g*. cisplatin, oxaliplatin, carboplatin); alkylation agents (*e.g*. estramustin, meclorethamine, melphalan, chlorambucil, busulphan, dacarbazin, cyclophosphamide, ifosfamide, temozolomide, nitrosoureas such as for example carmustin and lomustin, thiotepa); antimitotic agents (*e.g.* Vinca alkaloids e.g. vinblastine, vindesin, vinorelbin and vincristine; and taxanes such as paclitaxel, docetaxel, nab-paclitaxel (Abraxane)); angiogenesis inhibitors (*e.g*. tasquinimod, bevacizumab), tubuline inhibitors; DNA synthesis inhibitors, PARP inhibitors, topoisomerase inhibitors (*e.g*. epipodophyllotoxins such as for example etoposide and etopophos, teniposide, amsacrin, topotecan, irinotecan, mitoxantrone); serine/threonine kinase inhibitors (*e.g*. PDK 1 inhibitors, Raf inhibitors, A-Raf inhibitors, B-Raf inhibitors, C-Raf inhibitors, mTOR inhibitors (e.g. rapamycin, temsirolimus, everolimus, ridaforolimus, zotarolimus, sapanisertib, Torin 1, dactosilib, GDC-0349, vs-5584; vistusertib; AZD8055), mTORC1/2 inhibitors, PI3K inhibitors, PI3Kα inhibitors (e.g. alpelisib, serabelisib, GDC-0077, HH-CYH33, AMG 511, buparlisib, dactolisib, pictilisib, taselisib), dual mTOR/PI3K inhibitors, STK 33 inhibitors, AKT inhibitors, PLK 1 inhibitors, inhibitors of CDK4/6 (e.g. palbociclib, ribociclib, abemaciclib, trilaciclib, PF-06873600), Aurora kinase inhibitors); tyrosine kinase inhibitors (*e.g.* PTK2/FAK inhibitors); protein protein interaction inhibitors (*e.g.* IAP inhibitors/SMAC mimetics, MCL-1 (e.g. AZD-5991, AMG-176, AMG-397, S64315, S63845, A-1210477), MDM2, MDM2/MDMX); MEK inhibitors (e.g. trametinib, cobimetinib, binimetinib, selumetinib, refametinib); SOS1-inhibitor (*i.e.* a compound that modulates/inhibits the GEF functionality of SOS1, e.g. by binding to SOS1 and preventing protein-protein interaction between SOS1 and a (mutant) Ras protein, *e.g.* KRAS; *e.g.* BAY-293), an inhibitor of GDP-loaded or GTP-loaded RAS and/or of any mutants thereof (i.e. a compound that modulates/inhibits the functionality of (mutant) RAS protein by, *e.g*., binding to GDP-loaded or GTP-loaded (mutant) RAS protein, *e.g*. KRAS, NRAS and/or HRAS, preferably KRAS); an irreversible inhibitor of KRAS G12C (AMG-510, MRTX849, ARS-324, GDC-6036); a reversible or irreversible binder to GDP-loaded (mutant) KRAS; a reversible or irreversible binder to GTP-loaded (mutant) KRAS; ALK inhibitors (e.g. crizotinib, alectinib, entrectinib, brigatinib, ceritinib); ERK inhibitors; FLT3 inhibitors; BRD4 inhibitors; IGF-1R inhibitors; TRAILR2 agonists; Bcl-xL inhibitors; Bcl-2 inhibitors (*e.g*. venetoclax, obatoclax, navitoclax, oblimersen); Bcl-2/Bcl-xL inhibitors; ErbB receptor inhibitors; BCR-ABL inhibitors; ABL inhibitors; Src inhibitors (e.g. dasatinib, ponatinib, bosutinib, vandetanib, KX-01, saracatinib, KX2-391, SU 6656, WH-4-023); rapamycin analogs (*e.g*. everolimus, temsirolimus, ridaforolimus, sirolimus); androgen synthesis inhibitors; androgen receptor inhibitors; DNMT inhibitors; HDAC inhibitors; ANG1/2 inhibitors; histone deacetylase inhibitor; an inhibitor of IL6; inhibitor of JAK and/or any mutants thereof; an inhibitor of A-Raf and/or B-Raf and/or C-Raf and/or any mutants thereof (encorafenib, dabrafenib, vemurafenib, PLX-8394, RAF-709 (= example 131 in WO 2014/151616), LXH254, sorafenib, LY-3009120 (= example 1 in WO 2013/134243), lifirafenib, TAK-632, agerafenib, CCT196969, RO5126766, RAF265); an inhibitor of a receptor tyrosine kinase (RTK) and/or of any mutants thereof; an inhibitor of SHP2 and/or of any mutants thereof ( *e.g*. SHP099, TNO155, RMC-4550, RMC-4630, IACS-13909); CYP17 inhibitors; radiopharmaceuticals; proteasome inhibitors (*e.g*. carfilzomib); immunotherapeutic agents such as immune checkpoint inhibitors (*e.g*. CTLA4, PD1, PD-L1, PD-L2, LAG3, SIRPalpha-antibodies, and TIM3 binding molecules/immunoglobulins (ipilimumab, nivolumab, pembrolizumab, tislelizumab, atezolizumab, avelumab, durvalumab, pidilizumab, PDR-001 (= spartalizumab), AMG-404, ezabenlimab, sintilimab, camrelizumab, toribalimab, tislelizumab, ); ADCC (antibody-dependent cell-mediated cytotoxicity) enhancers (*e.g*. anti-CD33 antibodies, anti-CD37 antibodies, anti-CD20 antibodies); T-cell engagers, e.g. PSMA x CD3, B7H6/CD3 (as e.g. disclosed in WO2021/064137), DLL3/CD3 (as e.g. disclosed in WO2019/234220), *e.g.* bi-specific T-cell engagers (BiTEs^{®}) like *e.g.* CD3 x BCMA, CD3 x CD33, CD3 x CD19 ), cancer vaccines, MDM2-inhibitors, oncolytic viruses and various chemotherapeutic agents such as amifostin, anagrelid, clodronat, filgrastin, interferon, interferon alpha, leucovorin, procarbazine, levamisole, mesna, mitotane, pamidronate and porfimer. The compounds of the present invention can be used in combination with an OX40 agonist, an ICOS-ligand, a CD27 agonist, a GITR agonist, a Toll like receptor agonist.

In a preferred embodiment, additional pharmacologically active substance(s) which can also be used together/in combination with the compound of formula (I) - or a pharmaceutically acceptable salt thereof - (including all individual embodiments or generic subsets of compounds (I)) or in the medical uses, uses, methods of treatment and/or prevention as herein (above and below) disclosed include check-point inhibitors (ipilimumab, nivolumab, pembrolizumab, tislelizumab, atezolizumab, avelumab, durvalumab, pidilizumab, PDR-001 (= spartalizumab), AMG-404, ezabenlimab, sintilimab, camrelizumab, toribalimab, tislelizumab), taxanes (paclitaxel, docetaxel, nab-paclitaxel (Abraxane)), T-cell-engagers e.g. PSMA x CD3, B7H6/CD3 (as e.g. disclosed in WO2021/604137), DLL3/CD3 (as e.g. disclosed in WO2019/234220), e.g. bi-specific T-cell engagers (BiTEs^{®}) like e.g. CD3 x BCMA, CD3 x CD33, CD3 x CD19, cancer vaccines, MDM2-inhibitors, and oncolytic viruses.

In additional embodiments of the methods described herein, the compounds of the present invention are used in combination with chemotherapeutic agents and/or additional agents e.g. cancer-targeting therapies, for treating the indications as described in the methods herein. Thus the methods further involve administering to the subject an effective amount of one or more cancer-targeting agents as an additional treatment or a combination treatment.

In additional embodiments the methods described herein, the compounds of the present invention are used in combination with chemotherapeutic agents and/or additional agents for treating the indications as described in the methods herein and/or additional therapies such as radiotherapy and/or tumor excision.

In yet another aspect, the present invention relates a method for treating a disease or condition associated with or modulated by STING in a patient that includes the step of administering to a patient in need of such treatment a therapeutically effective amount of a compound of the present invention in combination with a therapeutically effective amount of one or more additional therapeutic agents described hereinbefore.

The use of the compound according to the invention in combination with the additional therapeutic agent may take place simultaneously or at staggered times.

The compound according to the invention and the one or more additional therapeutic agents may both be present together in one formulation or separately in two identical or different formulations, for example as a so-called kit-of-parts.

Thus, in a further aspect, the present invention provides a combination comprising a compound of general formula (I), and at least one further therapeutic agent.

A further aspect of the present invention is to provide a pharmaceutical composition comprising a compound of formula (I), or a pharmaceutically acceptable salt thereof, and at least one further therapeutic agent and one or more of pharmaceutically acceptable excipients.

In a further aspect, the invention provides a combination comprising a compound of formula (I), or a pharmaceutically acceptable salt thereof, and at least one further therapeutic agent for use in therapy.

In a further aspect, the invention provides a combination comprising a compound of formula (I), or a pharmaceutically acceptable salt thereof, and at least one further therapeutic agent for use in the treatment of a disease or condition in which modulation of STING is beneficial.

In a further aspect the invention provides a combination comprising a compound of formula (I), or a pharmaceutically acceptable salt thereof, and at least one further therapeutic agent for use in the treatment of cancer, e.g., canine or feline cancer.

In another aspect, this invention relates to a pharmaceutical composition which comprises a compound according to the invention and one or more additional therapeutic agent(s) described hereinbefore and hereinafter, optionally together with one or more inert carriers and/or diluents.

Other features and advantages of the present invention will become apparent from the following more detailed Examples which illustrate, by way of example, the principles of the invention.

### CHEMICAL SYNTHESIS

### LIST OF ABBREVIATIONS

| | |
|---|---|
| aq | Aqueous |
| Boc | tert-Butoxycarbonyl |
| DCM | Dichloromethane |
| DIPEA | Diisopropylethylamine (Hünig's base) |
| DMF | Dimethylformamide |
| DME | Dimethoxyethane |
| DMSO | Dimethylsulfoxide |
| PdCl₂dppf | [1,1'-bis-(diphenylphosphino)-ferrocenyl]-dichloro-palladium(II) |
| EtOH | Ethanol |
| EtOAc | Ethyl Acetate |
| h | Hours |
| HPLC | High Pressure Liquid Chromatography |
| iPrOH | Isopropanol |
| K₂CO₃ | Potassium carbonate |
| KOAc | Potassium acetate |
| LDA | Lithium Diisopropylamide |
| LiCl | Lithium chloride |
| MeCN | Acetonitrile |
| Me₃OBF₄ | Trimethyloxonium tetrafluoroborate |
| MeOH | Methanol |
| MgSO₄ | Magnesium sulfate |
| MS | Mass spectrometry |
| MeTHF | 2-Methyltetrahydrofuran |
| NaCl | Sodium chloride |
| NaHCO₃ | Sodium hydrogen carbonate (sodium bicarbonate) |
| NaOH | Sodium hydroxide |
| Na₂SO₄ | Sodium sulfate |
| NFSI | N-Fluorobenzenesulfonimide |
| NH₃ | Ammonia |
| NH₄Cl | Ammonium chloride |
| NMP | N-Methylpyrrolidone |
| NMR | Nuclear Magnetic Resonance |
| ppm | Part per million |
| RT | Room temperature |
| TEA | Triethylamine |
| TFA | Trifluoroacetic acid |
| THF | Tetrahydrofuran |
| TMSCl | Trimethylsilyl chloride |

Other features and advantages of the present invention will become apparent from the following more detailed examples which exemplarily illustrate the principles of the invention without restricting its scope.

### GENERAL

Unless stated otherwise, all the reactions are carried out in commercially obtainable apparatuses using methods that are commonly used in chemical laboratories. Starting materials that are sensitive to air and/or moisture are stored under protective gas and corresponding reactions and manipulations there with are carried out under protective gas (nitrogen or argon). The compounds according to the invention are named in accordance with IUPAC guidelines. If a compound is to be represented both by a structural formula and by its nomenclature, in the event of a conflict the structural formula is decisive.

### Chromatography

Thin layer chromatography is carried out on ready-made TLC plates of silica gel 60 on glass (with fluorescence indicator F-254) made by Merck.

A Biotage Isolera Four apparatus is used for automated preparative NP chromatography together with Interchim Puri Flash columns (50 µm, 12 - 300 g) or glass columns filled with silica gel made by Millipore (Granula Silica Si-60A 35-70 µm).

Preparative RP HPLC is carried out with columns made by Waters (Sunfire C18, 10 µm, 30x100 mm Part. No. 186003971 or X-Bridge C18, 10 µm, 30x100 mm Part. No. 186003930). The compounds are eluted using either different gradients of H2O/acetonitrile or H2O/MeOH, where 0.1% TFA is added to the water, or with different gradients utilizing a basic aqueous buffer solution (1 L water contains 5 mL of an ammonium hydrogen carbonate solution (158 g per 1 L H2O) and 2 mL ammonia (7 mol/I solution in MeOH)) instead of the water-TFA-mixture.

The analytical HPLC (reaction monitoring) of intermediate compounds is carried out with columns made by Waters and Phenomenex. The analytical equipment is also provided with a mass detector in each case.

### HPLC mass spectroscopy/UV spectrometry

The retention times/MS-ESI+ for characterizing the example compounds according to the invention are determined using an HPLC-MS apparatus (high performance liquid chromatography with mass detector) e.g. made by Agilent. Compounds that elute at the injection peak are given the retention time tR = 0.

### Analytical HPLC Methods:

### Acidic Method

| | |
|---|---|
| HPLC: | Agilent 1260 Infinity II |
| MS: | Agilent LC/MS (G6125B) |
| Column: | Sunfire C18 2.5 µm, 3.0x30 mm |
| Eluent: | A: 0.1% TFA (v/v) in H2O; B: MeCN (HPLC grade) |
| Detection: MS: | Positive and negative mode |
| Column temp.: | 60 °C |
| Gradient: | 0.00 - 0.20 min: 3% B (Flow 2.2 ml/min) |
| | 0.20 - 1.20 min: 3% to 100% B (Flow 2.2 ml/min) |
| | 1.20 - 1.25 min: 100% B (Flow 3.0 ml/min) |
| | 1.25 - 1.40 min: 100% B (Flow 3.0 ml/min) |

### Basic Method

| | |
|---|---|
| HPLC: | Agilent 1260 Infinity II |
| MS: | Agilent LC/MS (G6125B) |
| Column: | X-Bridge C18, 2.5 µm, 3.0x30 mm |
| Eluant: | A: 0.1% NH4OH (v/v) in H2O; B: MeCN (HPLC grade) |
| Detection: | MS: Positive and negative mode |
| Column temp.: | 60 °C |
| Gradient: | 0.00 - 0.20 min: 3% B (Flow 2.2 ml/min) |
| | 0.20 - 1.20 min: 3% to 100% B (Flow 2.2 ml/min) |
| | 1.20 - 1.25 min: 100% B (Flow 3.0 ml/min) |
| | 1.25 - 1.40 min: 100% B (Flow 3.0 ml/min) |

### Preparative HPLC Methods:

### Acidic Method

| | |
|---|---|
| HPLC: | Agilent 1260 Infinity II |
| MS: | Agilent LC/MS (G6125B) |
| Column: | Sunfire C18 10 µm, 30x300 mm |
| Eluent: | A: 0.1% TFA (v/v) in H2O; B: MeCN (HPLC grade) |
| Detection: | MS: Positive and negative mode |
| Flow: | 50 ml/min |
| Column temp.: | 40 °C |

### Basic Method

| | |
|---|---|
| HPLC: | Agilent 1260 Infinity II |
| MS: | Agilent LC/MS (G6125B) |
| Column: | X-Bridge C18, 10 µm, 30x300 mm |
| Eluant: | A: 0.1% NH4OH (v/v) in H2O; B: MeCN (HPLC grade) |
| Detection: | MS: Positive and negative mode |
| Flow: | 50 ml/min |
| Column temp.: | 40 °C |

### PREPARATION OF THE COMPOUNDS ACCORDING TO THE INVENTION

The compounds according to the present invention and their intermediates may be obtained using methods of synthesis which are known to the one skilled in the art and described in the literature of organic synthesis. These methods are intended as an illustration of the invention, without restricting its subject matter and the scope of the compounds claimed to these examples. Preferably, the compounds are obtained in analogous fashion to the methods of preparation explained more fully hereinafter, in particular as described in the experimental section. In some cases, the order in carrying out the reaction steps may be varied. Variants of the reaction methods that are known to the one skilled in the art but not described in detail here may also be used.

The general processes for preparing the compounds according to the invention will become apparent to the one skilled in the art studying the following schemes. Starting materials may be prepared by methods that are described in the literature or herein, or may be prepared in an analogous or similar manner. Any functional groups in the starting materials or intermediates may be protected using conventional protecting groups. These protecting groups may be cleaved again at a suitable stage within the reaction sequence using methods familiar to the one skilled in the art.

One method for the preparation of compounds of formula (I) is exemplified in Scheme I: Indazoles **B** can be synthesized from ortho-methyl aniline derivatives **A.** Subsequent iodination leads to 3-iodo-indazoles **C.** Intermediates **D** can be obtained e.g. by Chan-Lam coupling utilizing (6-fluoropyridin-3-yl)boronic acid. Conversion into intermediates **F** can be achieved e.g. *via* Suzuki coupling with intermediates **E.** Finally, compounds of formula (I) are synthesized, e.g., by nucleophilic aromatic substitution. The products are isolated by conventional means and preferably purified by chromatography.

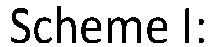

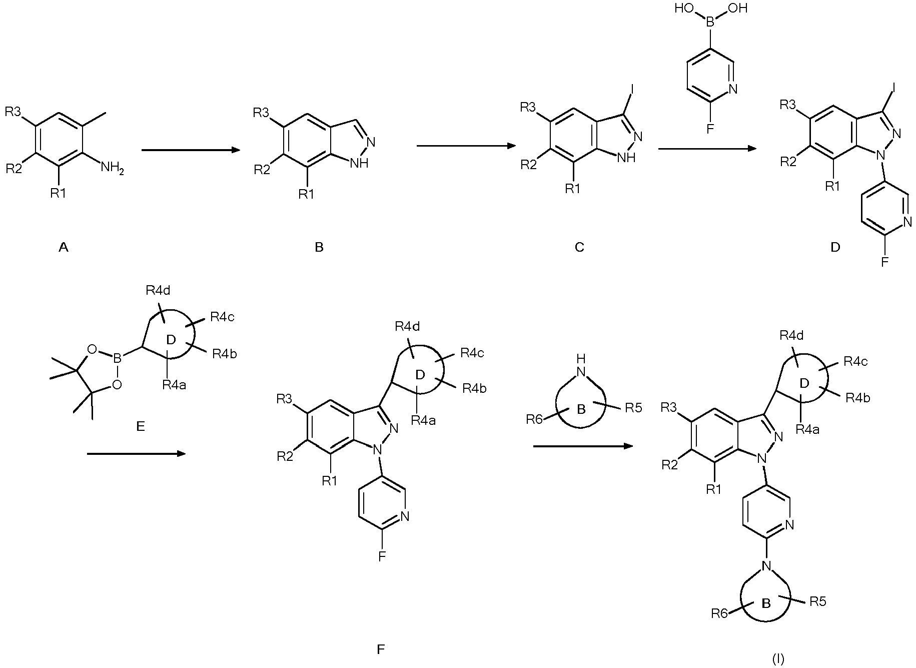

A second method for the preparation of compounds of formula (I) is exemplified in Scheme II: Intermediates **D** can be converted to intermediates **G** via various methods, e.g., by nucleophilic aromatic substitution. Intermediates **G** can be converted to compounds of formula (I) can be achieved, e.g., *via* Suzuki coupling with intermediates **E.** The products are isolated by conventional means and preferably purified by chromatography.

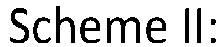

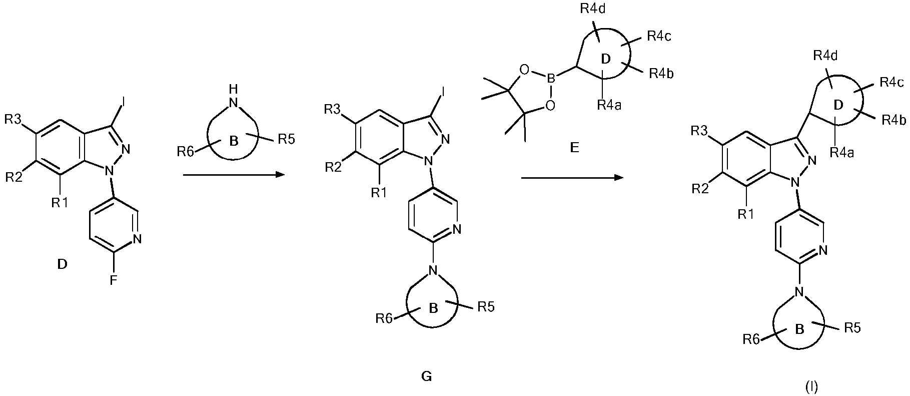

A third method for the preparation of compounds of formula (I) is exemplified in Scheme III: (6-fluoropyridin-3-yl)boronic acid can be converted to intermediates **H** via various methods, e.g. by nucleophilic aromatic substitution. Intermediates **G** can be obtained from intermediate **H,** e.g., by Chan-Lam coupling. Intermediates **G** can be converted to compounds of formula (I) can be achieved, e.g., via Suzuki coupling with intermediates **E.** The products are isolated by conventional means and preferably purified by chromatography.

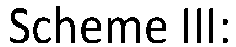

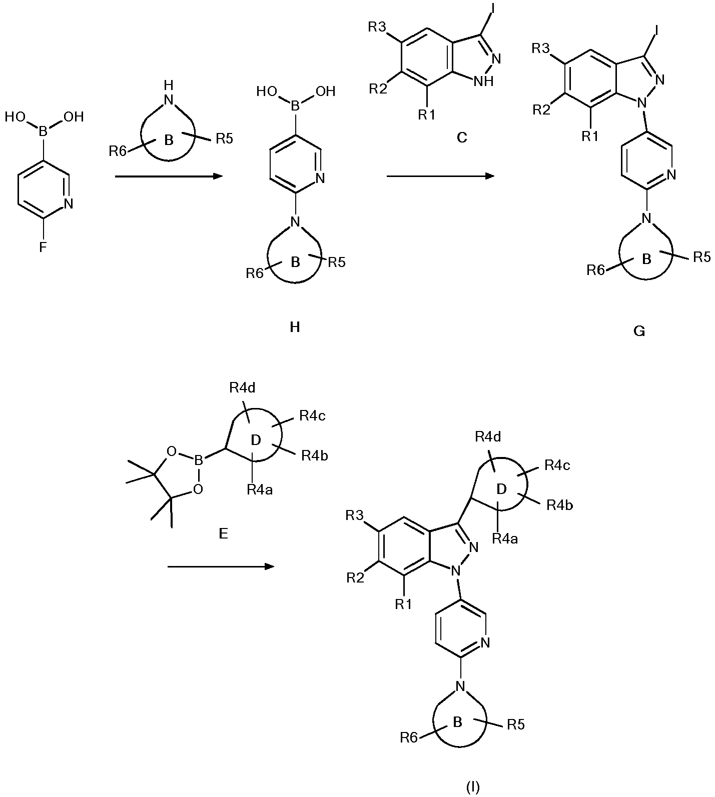

### PREPARATION OF INTERMEDIATES

### Intermediate 1

### 4-Bromo-3-fluoro-2-methyl-2H-indazole

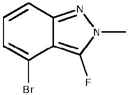

To a solution of 4-bromo-2-methyl-2H-indazole (100 mg, 0.46 mmol) in MeCN (1 ml) was added 1-chloromethyl-4-fluoro-1,4-diazoniabicyclo[2.2.2]octane-bis-(tetrafluoroborate) (Selectfluor, 204 mg, 0.55 mmol) and the mixture was heated at 80°C using a microwave for 2 h. The crude reaction mixture was purified by preparative HPLC (acidic method). Two products were isolated, i.e., the title compound (4-bromo-3-fluoro-2-methyl-2H-indazole, 9 mg) and the regioisomer (4-bromo-7-fluoro-2-methyl-2H-indazole, 7 mg).

Alternatively, to a solution of 4-bromo-2-methyl-2H-indazole (1 g, 4.5 mmol) in anhydrous THF (20 ml) cooled to -78°C was added a solution of LDA (2M in THF, 5.23 ml, 10.5 mmol) and stirred at this temperature for 30 min. Then N-fluorobenzenesulfonimide (NFSI, 2.93 g, 9.2 mmol) was added and allowed to slowly warm to RT and stirred for 2.5 h. The reaction was carefully quenched with water, diluted with DMF, acidified with TFA and purified by preparative HPLC (acidic method). The title compound was isolated (371 mg).

1H NMR (400 MHz, DMSO-d6) δ ppm 4.03 (d, J=1.90 Hz, 3 H) 7.15 (dd, J=8.68, 7.16 Hz, 1 H) 7.27 (d, J=7.22 Hz, 1 H) 7.50 (dd, J=8.68, 1.84 Hz, 1 H).

### Intermediate 2

### (3-Fluoro-2-methyl-2H-indazol-4-yl)boronic acid

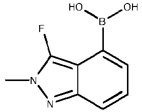

A mixture of intermediate 1 (445 mg, 1.94 mmol), bis-(neopentyl glycolato)diboron (543 mg, 2.33 mmol), and potassium acetate (763 mg, 7.77 mmol) in dioxane (10 ml) was degassed and an nitrogen atmosphere was maintained. To the mixture was added [1,1'-bis-(diphenylphosphino)-ferrocenyl]-dichloropalladium(II) DCM complex (79 mg, 0.097 mmol) and it was heated at 85 °C for 2 h. To the reaction was added MeOH and then filtered. The filtrate was diluted with water and was purified by preparative HPLC (acidic method) to give the titled compound (200 mg).

1H NMR (400 MHz, DMSO-d6) δ ppm 4.01 (d, J=1.77 Hz, 3 H), 7.12 (dd, J=6.72, 2.79 Hz, 6 H), 7.35 (dd, J=8.81, 6.91 Hz, 6 H), 7.53 (dd, J=8.81, 1.71 Hz, 6 H).

### Intermediate 3

### 1-(6-Fluoropyridin-3-yl)-3-iodo-7-methyl-1H-indazole

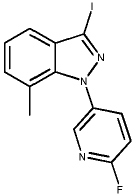

To a stirred reaction of 3-iodo-7-methyl-1H-indazole (16 g, 62 mmol) in DCM (200 ml) was added copper acetate (16.89 g, 93 mmol), pyridine (9.80 g, 124 mmol), and 6-fluoropyridine-3-boronic acid (14.85 g, 105 mmol), and then the reaction mixture was stirred at RT for 72 h. The reaction was filtered through celite, the filtrate was concentrated and the crude product was purified using silica chromatography (EtOAc:Hexane) to give the title compound (13 g).

1H NMR (400 MHz, DMSO-d6) δ ppm 2.09 (s, 3 H), 7.25 (m, 1 H), 7.34 (m, 1 H), 7.42 (m, 2 H), 8.28 (ddd, J=8.65, 7.07, 2.79 Hz, 1 H), 8.53 (d, J=2.15 Hz, 1 H).

### Intermediate 4

### Methyl (1R,5S,6R)-3-[5-(3-iodo-7-methyl-1H-indazol-1-yl)pyridin-2-yl]-3-azabicyclo[3.1.0]hexane-6-carboxylate

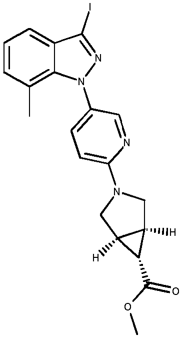

To a mixture of 1-(6-fluoropyridin-3-yl)-3-iodo-7-methyl-1H-indazole (Intermediate 3, 1g, 2.83 mmol) and methyl exo-3-azabicyclo[3.1.0]hexane-6-carboxylate hydrochloride (1.037g, 5.66 mmol) in NMP (6 ml) was added DIPEA (1.974 ml, 11.61 mmol). The reaction was heated at 80 °C for 16 h. The reaction was allowed to cool and added to water. The precipitate formed was collected by filtration and dried under vacuum. The solid was purified by silica chromatography (Cyclohexane:EtOAc) to give the title compound (1.14 g).

1H NMR (400 MHz, DMSO-d6) δ ppm 1.61 (t, J=3.04 Hz, 1 H), 2.08 (s, 3 H), 2.26 (br s, 2 H), 3.55 (dd, J=9.19, 1.58 Hz, 2 H), 3.63 (s, 3 H), 3.83 (d, J=10.90 Hz, 2 H), 6.57 (d, J=8.87 Hz, 1 H), 7.18 (m, 1 H), 7.25 (d, J=6.84 Hz, 1 H), 7.35 (d, J=7.98 Hz, 1 H), 7.67 (dd, J=8.87, 2.66 Hz, 1 H), 8.21 (d, J=2.53 Hz, 1 H).

### Intermediate 5

### Methyl (1R,5S,6R)-3-(5-{3'-fluoro-2',7-dimethyl-1H,2'H-[3,4'-biindazol]-1-yl}pyridin-2-yl)-3-azabicyclo[3.1.0]hexane-6-carboxylate

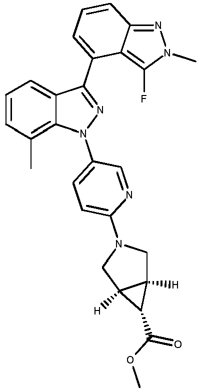

A mixture of intermediate 4 (90 mg, 0.19 mmol), intermediate 2 (39 mg, 0.2 mmol), sodium carbonate (60 mg, 0.57 mmol) in dioxane (2 ml) and water (0.5 ml) was degassed, and a nitrogen atmosphere was maintained. To the mixture was added [1,1'-bis-(diphenylphosphino)-ferrocenyl]-dichloro-palladium(II) (PdCl₂dppf, 6.9 mg, 0.009mmol) and the mixture was heated at 100°C for 45 min. To the reaction was added DMF and the reaction mixture was then filtered. The filtrate was diluted with water, acidified with TFA and was purified by preparative HPLC (acidic method) to give the title compound (46 mg).

1H NMR (400 MHz, DMSO-d6) δ ppm 1.65 (t, J=3.04 Hz, 1 H), 2.19 (s, 3 H), 2.29 (br s, 2 H), 3.64 (s, 4 H), 3.60 (m, 2 H), 3.86 (d, J=11.03 Hz, 2 H), 4.02 (d, J=1.77 Hz, 3 H), 6.69 (d, J=9.00 Hz, 1 H), 7.20 (m, 1 H), 7.25 (m, 1 H), 7.41 (m, 1 H), 7.48 (m, 1 H), 7.56 (dd, J=8.74, 1.01 Hz, 1 H), 7.83 (m, 2 H), 8.33 (d, J=2.41 Hz, 1 H).

### Intermediate 6

### 3'-Fluoro-1-(6-fluoropyridin-3-yl)-2',7-dimethyl-1H,2'H-3,4'-biindazole

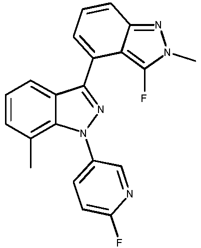

A mixture of intermediate 3 (365 mg, 1.03 mmol), intermediate 2 (200 mg,1.03 mmol), and sodium carbonate (328 mg, 3.1 mmol) in dioxane (8 ml) and water (2 ml) was degassed and a nitrogen atmosphere was maintained. To the mixture was added [1,1'-bis-(diphenylphosphino)-ferrocenyl]-dichloro-palladium(II) (PdCl₂dppf, 38 mg, 0.05mmol) and the mixture was heated at 100°C for 2.5h. To the reaction was added DMF and the reaction mixture was then filtered. The filtrate was diluted with water, acidified with TFA and was purified by preparative HPLC (acidic method) to give the title compound (260 mg).

1H NMR (400 MHz, DMSO-d6) δ ppm 2.07 (s, 1 H), 2.18 (s, 3 H), 4.03 (d, J=1.65 Hz, 3 H), 7.26 (m, 1 H), 7.32 (m, 1 H), 7.44 (m, 2 H), 7.52 (d, J=6.72 Hz, 1 H), 7.59 (dd, J=8.68, 1.33 Hz, 1 H), 7.89 (d, J=7.98 Hz, 1 H), 8.35 (t, J=7.81 Hz, 1 H), 8.60 (d, J=2.53 Hz, 1 H).

### Intermediate 7

### (7-Fluoro-2-methyl-2H-indazol-4-yl)boronic acid

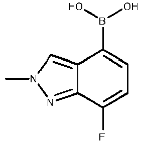

Using the method described for Intermediate 2: 4-bromo-7-fluoro-2-methyl-2H-indazole (100 mg) gave the title compound (104 mg).

### Intermediate 8

### 7'-Fluoro-1-(6-fluoropyridin-3-yl)-2',7-dimethyl-1H,2'H-3,4'-biindazole

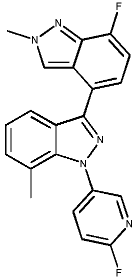

Using the method described for Intermediate 5: Intermediate 3 (120 mg) and Intermediate 7 (89 mg) gave the title compound (90 mg).

### Intermediate 9

### 2',7-Dimethyl-1H,2'H-3,4'-biindazole

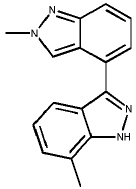

A mixture of 2-methyl-7-(4,4,5,5-tetramethyl-1,3,2-dioxaborolan-2-yl)-2H-indazole (CAS: 845751-67-9: 5.5 g, 21.3 mmol), and K₂CO₃ (6.69 g, 48.44 mmol) in EtOH (60 ml) was degassed and maintained under an Argon atmosphere. To this was added water (12.5 ml), followed by bis(di-tert-butyl(4-dimethylaminophenyl)phosphine)dichloropalladium(II) ((AmPhos)2*PdCl₂: 343 mg, 0.49 mmol) and then 3-iodo-7-methyl-1H-indazole (CAS: 847906-27-85 g, 19.4 mmol) was added. The reaction was heated at 75 °C for 1 h. N-Acetyl-L-cysteine (3.16 g, 19.38 mmol) in water (65ml) was added. The reaction mixture was allowed to cool and the solid formed was collected by filtration to give the title compound (4.68 g).

1H NMR (400 MHz, DMSO-d6) δ ppm 2.60 (s, 3 H), 4.25 (s, 3 H), 7.14 (t, J=7.17 Hz, 1 H), 7.21 (d, J=6.84 Hz, 1 H), 7.39 (dd, J=8.62, 6.97 Hz, 1 H), 7.63 (d, J=8.74 Hz, 1 H), 7.73 (d, J=6.59 Hz, 1 H), 7.98 (d, J=7.98 Hz, 1 H), 8.70 (s, 1 H), 13.39 (s, 1 H).

### Intermediate 10

### 5'-Fluoro-2',7-dimethyl-1H,2'H-3,4'-biindazole

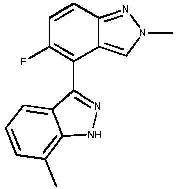

To a solution of Intermediate 9 (400 mg, 1.53 mmol) in dioxane (5 ml) and water (5 ml) was added NFSI (981 mg, 3.05 mmol) and stirred at 80 °C for 16 h. To the reaction was added DMF and then filtered. The filtrate was diluted with water, acidified with TFA and was purified by preparative HPLC (acidic method) to give the title compound (60 mg) and the regioisomer (7'-fluoro-2',7-dimethyl-1H,2'H-3,4'-biindazole, 30 mg) and the difluorinated compound (5',7',-difluoro-2',7-dimethyl-1H,2'H-3,4'-biindazole, 40 mg).

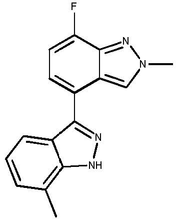

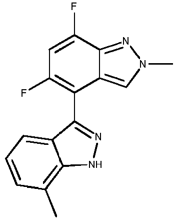

### Intermediate 11

### 5'-Fluoro-1-(6-fluoropyridin-3-yl)-2',7-dimethyl-1H,2'H-3,4'-biindazole

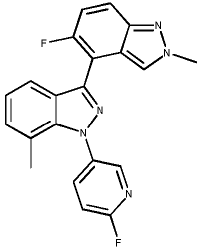

To a solution of intermediate 10 (60 mg, 0.21 mmol) and (6-fluoropyridin-3-yl)boronic acid (90.5 mg, 0.64 mmol) in DCM (5 ml) was added pyridine (51.8µl, 0.64 mmol) followed by copper(II) acetate (58.3 mg, 0.32 mmol) and the mixture was stirred at RT for 16 h. The reaction was diluted with sat. NaHCO₃(aq) and extracted with DCM, the organic phases were combined, dried over Na₂SO₄, filtered and concentrated under reduced pressure. The residue was purified by preparative HPLC (acidic method) to give the title compound (49 mg).

### Intermediate 12

### 5',7'-Difluoro-2',7-dimethyl-1H,2'H-3,4'-biindazole

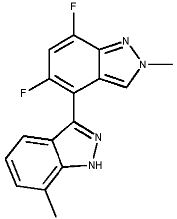

The Intermediate 12 was isolated from the synthesis of Intermediate 10 and the synthesis is described therein and gave the title compound (40 mg).

Alternatively,
To a solution of 7'-fluoro-2',7-dimethyl-1H,2'H-3,4'-biindazole (960 mg, 3.43 mmol) in MeCN (30 mL) was added SelectFluor (971 mg, 2.74 mmol) and stirred at RT for 45 min. Additional SelectFluor (260 mg) was added at stirred at RT for 40 min. The reaction was concentrated and redissolved with DMF, basified with aq. NH3. sol. and purified over preparative. HPLC (Basic) and gave the desired product (100 mg, 10%).

The following by-product (8 mg, 3',5',7'-trifluoro-2',7-dimethyl-1H,2'H-3,4'-biindazole) was also isolated.

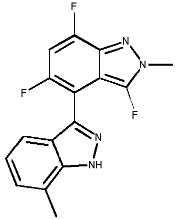

### Intermediate 13

### 5',7'-Difluoro-1-(6-fluoropyridin-3-yl)-2',7-dimethyl-1H,2'H-3,4'-biindazole

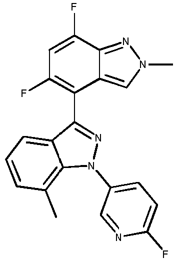

Using the method described for Intermediate 11: Intermediate 12 (40 mg) and (6-fluoropyridin-3-yl)boronic acid (38 mg) gave the title compound (18 mg).

Alternatively, to a solution of intermediate 8 (960 mg, 3.425 mmol) in MeCN (30 ml) was added SelectFluor (970.6 mg, 2.74 mmol) and stirred for 30 mins. An additional portion of SelectFluor (260 mg) was added and stirred for 40 mins. The reaction was concentrated and purified by preparative HPLC (basic) to give the titled compound (300 mg, 29%).
HPLC (Basic): Rt = 1.182 min ((M+H)+ 299.0)

Also isolated were the by-products:
5,5',7'-Trifluoro-1-(6-fluoropyridin-3-yl)-2',7-dimethyl-1H,2'H-3,4'-biindazole (100 mg, 10%) 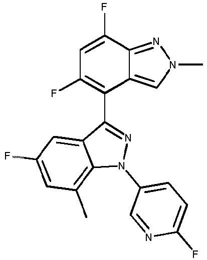
3',5',7'-Trifluoro-1-(6-fluoropyridin-3-yl)-2',7-dimethyl-1H,2'H-3,4'-biindazole (80 mg, 8%) 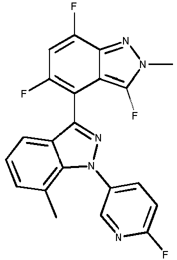
HPLC (Acidic): Rt = 1.011 min ((M+H)+ 317.0)

### Intermediate 14

### 3'-Fluoro-2',7-dimethyl-1-[6-(piperazin-1-yl)pyridin-3-yl]-1H,2'H-3,4'-biindazole

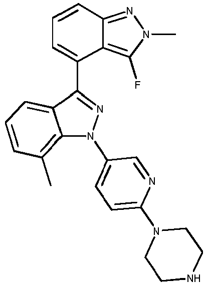

Using the method described for Example 2: Intermediate 6 (65 mg, 0.17 mmol) and piperazine (45.2 µl, 0.52 mmol) gave the title compound (35 mg) after preparative HPLC (basic method).

1H NMR (400 MHz, DMSO-d6) δ ppm 2.19 (s, 3 H), 2.82 (m, 4 H), 3.53 (m, 4 H), 4.02 (d, J=1.65 Hz, 3 H), 6.95 (d, J=9.00 Hz, 1 H), 7.19 (m, 1 H), 7.24 (m, 1 H), 7.41 (m, 1 H), 7.49 (m, 1 H), 7.55 (d, J=9.51 Hz, 1 H), 7.76 (dd, J=9.00, 2.66 Hz, 1 H), 7.84 (d, J=7.98 Hz, 1 H), 8.32 (d, J=2.66 Hz, 1 H).

### Intermediate 15

### Methyl (1R,5S,6R)-3-(5-iodopyridin-2-yl)-3-azabicyclo[3.1.0]hexane-6-carboxylate

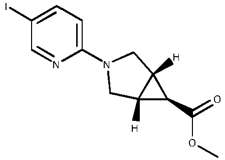

A mixture of 2-fluoro-5-iodopyridine (6.0 g, 26.91 mmol), methyl (1R,5S,6R)-3-azabicyclo[3.1.0]hexane-6-carboxylate hydrochloride (5.7 g, 32.29 mmol) and potassium carbonate (8.2 g, 59.20 mmol) in NMP (20 ml) under an Argon atmosphere was heated and stirred at 120 °C for 16 h. The reaction was allowed to cool and poured into water and it was stirred for 1 h. The solid formed was collected by filtration and dried under vacuum at 50 °C and gave the title compound (7.08 g).

1H NMR (400 MHz, DMSO-d6) δ ppm 1.52 (t, J=3.04 Hz, 2 H), 2.20 (m, 4 H), 3.40 (dt, J=10.96, 1.55 Hz, 4 H), 3.61 (s, 6 H), 3.69 (d, J=10.77 Hz, 4 H), 6.36 (d, J=8.62 Hz, 2 H), 7.72 (dd, J=8.81, 2.34 Hz, 2 H), 8.21 (d, J=1.77 Hz, 2 H)

### Intermediate 16

### 5-Fluoro-3-iodo-7-methyl-1H-indazole

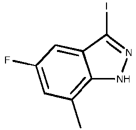

To a solution of 5-fluoro-7-methyl-1H-indazole (CAS: 1427377-45-4, 4 g, 26.64 mmol) in DMF (24 ml) and water (6 ml) was added potassium phosphate (8.48 g, 39.96 mmol). To this was added iodine (7.44 g, 29.30 mmol) slowly portionwise. The reaction was stirred at 30 °C for 1 h. To the reaction was then added a solution of sodium metabisulfite (7.6 g) in water (88 ml) and the mixture was stirred for 2.5 h. The formed solid was collected by filtration and the solid was washed with water. The solid was dried at 45 °C for 16 h.

1H NMR (400 MHz, DMSO-d6) δ ppm 6.98 (dd, J=8.62, 2.28 Hz, 2 H), 7.16 (br d, J=9.76 Hz, 2 H), 13.69 (br s, 2 H)

### Intermediate 17

### 3',5-Difluoro-2',7-dimethyl-1H,2'H-3,4'-biindazole

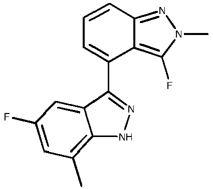

Using the method described for Intermediate 5: Intermediate 16 (100 mg, 0.33 mmol) and 3-fluoro-2-methyl-4-(4,4,5,5-tetramethyl-1,3,2-dioxaborolan-2-yl)indazole (111 mg, 0.39 mmol) gave the title compound (54 mg).

1H NMR (400 MHz, DMSO-d6) δ ppm 2.59 (s, 3 H), 4.04 (d, J=1.77 Hz, 3 H), 7.14 (m, 1 H), 7.38 (m, 2 H), 7.46 (dd, J=9.31, 2.09 Hz, 1 H), 7.52 (m, 1 H), 13.55 (m, 1 H)

### Intermediate 18

### 6-Fluoro-3-iodo-7-methyl-1H-indazole

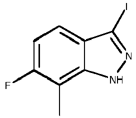

Using the method described for Intermediate 16: 6-fluoro-7-methyl-1H-indazole (CAS 1427395-91-2, 4.3 g, 28.64 mmol) gave the title compound (7.55 g).

1H NMR (400 MHz, DMSO-d6) δ ppm 2.41 (m, 3 H), 7.05 (t, J=9.38 Hz, 1 H), 7.27 (dd, J=8.74, 4.82 Hz, 1 H), 13.67 (s, 1 H)

### Intermediate 19

### 6-Fluoro-1-(6-fluoropyridin-3-yl)-3-iodo-7-methyl-1H-indazole

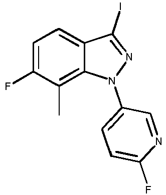

Using the method described for Intermediate 11: Intermediate 18 (5 g, 17.39 mmol) and 2-fluoropyridine-5- boronic acid (3.75 g, 26.08 mmol) gave the title compound (3.35 g) after recrystallization in EtOH.

1H NMR (400 MHz, DMSO-d6) δ ppm 1.95 (d, J=1.77 Hz, 3 H), 7.23 (t, J=9.38 Hz, 1 H), 7.44 (m, 2 H), 8.30 (ddd, J=8.68, 7.03, 2.79 Hz, 1 H), 8.54 (d, J=2.15 Hz, 1 H)

### Intermediate 20

### Methyl (1R,5S,6R)-3-[5-(6-fluoro-3-iodo-7-methyl-1H-indazol-1-yl)pyridin-2-yl]-3-azabicyclo[3.1.0]hexane-6-carboxylate

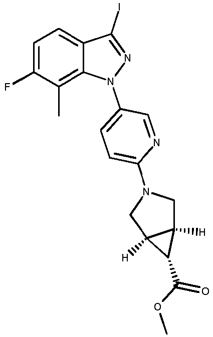

Using the method described for Intermediate 4: Intermediate 19 (2.0 g, 5.39 mmol) and methyl exo-3-azabicyclo[3.1.0]hexane-6-carboxylate hydrochloride (1.48g, 8.08 mmol) gave the title compound (2.33 g).

1H NMR (400 MHz, DMSO-d6) δ ppm 1.61 (t, J=2.98 Hz, 1 H), 1.95 (d, J=1.27 Hz, 3 H), 2.26 (br s, 2 H), 3.55 (m, 2 H), 3.63 (s, 3 H), 3.83 (d, J=10.90 Hz, 2 H), 6.58 (d, J=8.87 Hz, 1 H), 7.17 (t, J=9.24 Hz, 1 H), 7.37 (dd, J=8.81, 5.01 Hz, 1 H), 7.69 (dd, J=9.00, 2.66 Hz, 1 H), 8.23 (d, J=2.53 Hz, 1 H)

### Intermediate 21

### 4-Bromo-7-fluoro-1H-indazole

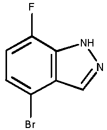

A solution of 6-bromo-2,3-difluorobenzaldehyde (180 g, 0.82 mol, 1 equiv.) in DME (900 mL) and hydrazine hydrate (900 mL) was stirred for 40 h at 90 °C. The resulting mixture was concentrated under reduced pressure. The residue was triturated using n-hexane (500 mL). The mixture was filtered, and the filter cake was washed with n-hexane (200 mL). This resulted into 4-bromo-7-fluoro-2H-indazole (140 g) as a light-yellow solid.

### Intermediate 22

### 4-Bromo-7-fluoro-2-methyl-2H-indazole

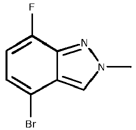

To a solution of 4-bromo-7-fluoro-2H-indazole (Intermediate 21, 40 g, 186.9 mmol, 1 equiv) in EtOAc (800 mL) was added Me₃OBF₄ (41.5 g, 280.4 mmol, 1.5 equiv.) under nitrogen atmosphere at 0°C. The mixture was stirred for 16 h at room temperature. The reaction was quenched by water (500 mL). The resulting mixture was extracted with EtOAc (3 x 500 mL). The combined organic layers were washed with brine (1x800 mL) and dried over anhydrous Na₂SO₄. After filtration, the filtrate was concentrated under reduced pressure. The residue was purified by silica gel column chromatography, eluted with Petroleum Ether:EtOAc (10:1 to 4:1) to afford 4-bromo-7-fluoro-2-methyl-2H-indazole (30 g) as a white solid.

1H NMR (300 MHz, DMSO-d6) δ 8.52 (d, J = 2.7 Hz, 1H), 7.21 (dd, J = 8.0, 3.7 Hz, 1H), 7.00 (dd, J = 11.5, 7.9 Hz, 1H), 4.22 (s, 3H).

### Intermediate 23

### 7-Fluoro-2-methyl-4-(4,4,5,5-tetramethyl-1,3,2-dioxaborolan-2-yl)-2H-indazole

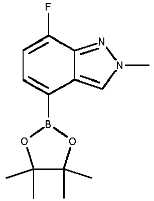

To a solution of Intermediate 22 (500 mg, 2.14 mmol) and bis(pinacolato)diboron (832 mg, 3.21 mmol) in dioxane (5.0 ml) was added potassium acetate (840 mg 8.56 mmol) and the mixture was degassed with Argon. To the reaction mixture was added [1,1'-bis-(diphenylphosphino)-ferrocenyl]-dichloro-palladium(II) (PdCl₂dppf, 175 mg, 0.21 mmol) and heated at 90 °C for 16 h. The reaction was allowed to cool to RT. The reaction was diluted with water and the organics were extracted with EtOAc. The organic layers were combined, washed with brine, dried over MgSO₄, filtered and concentrated under reduced pressure. The title compound was isolated (487 mg) after silica chromatography (Cyclohexane:EtOAc).

1H NMR (400 MHz, DMSO-d6) δ ppm 1.33 (s, 12 H), 4.23 (s, 3 H), 7.02 (m, 1 H), 7.42 (dd, J=7.35, 4.94 Hz, 1 H), 8.45 (d, J=2.91 Hz, 1 H)

### Intermediate 24

### 5-Fluoro-1-(6-fluoropyridin-3-yl)-3-iodo-7-methyl-1H-indazole

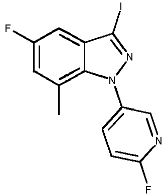

Using the method described for Intermediate 11: Intermediate 16 (10 g, 33.7 mmol) and 2-fluoropyridine-5- boronic acid (11.87 g, 84.22 mmol) gave the title compound (3.3 g) after recrystallization in EtOH.

1H NMR (400 MHz, DMSO-d6) δ ppm 2.09 (s, 3 H), 7.18 (m, 1 H), 7.31 (dd, J=9.89, 1.39 Hz, 1H), 7.43 (dd, J=8.62, 3.04 Hz, 1H), 8.29 (m, 1 H), 8.53 (d, J=2.15 Hz, 1 H)

### Intermediate 25

### Methyl (1R,5S,6R)-3-[5-(5-fluoro-3-iodo-7-methyl-1H-indazol-1-yl)pyridin-2-yl]-3-azabicyclo[3.1.0]hexane-6-carboxylate

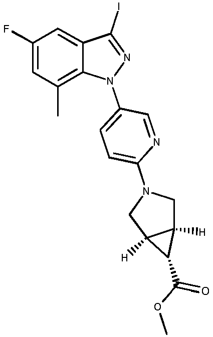

Using the method described for Intermediate 4: Intermediate 24 (2.0 g, 5.39 mmol) and methyl exo-3-azabicyclo[3.1.0]hexane-6-carboxylate hydrochloride (1.97g, 10.78 mmol) gave the title compound (2.56 g).

1H NMR (400 MHz, DMSO-d6) δ ppm 1.61 (t, J=3.04 Hz, 1 H), 2.08 (s, 3 H), 2.26 (br s, 2 H), 3.55 (m, 2 H), 3.63 (s, 3 H), 3.83 (d, J=10.90 Hz, 2 H), 6.57 (d, J=9.00 Hz, 1 H), 7.10 (dd, J=8.24, 2.03 Hz, 1 H), 7.22 (dd, J=9.89, 1.39 Hz, 1 H), 7.68 (dd, J=8.93, 2.72 Hz, 1 H), 8.22 (d, J=2.53 Hz, 1 H)

### Intermediate 26

### 4-Bromo-3,7-difluoro-2-methyl-2H-indazole

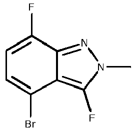

To a stirred solution of 4-bromo-7-fluoro-2-methylindazole (10 g, 43.7 mmol, 1 equiv.) in DMF (200 mL) was added SelectFluor (30.93 g, 87.3 mmol, 2.0 equiv.) at room temperature. The resulting mixture was stirred for 16 h at 60 °C. The mixture was purified by reversed-phase flash chromatography with the following conditions: column, C18 silica gel; mobile phase, 0.5% NH₃·H2O in water, MeCN 50% to 75% gradient in 20 min; detector, UV 254 nm to afford 4-bromo-3,7-difluoro-2-methylindazole (3 g) as a white solid.

1H NMR (400 MHz, DMSO-d, ppm) δ: 7.21 (dd, J = 7.9, 3.5 Hz, 1H), 7.03 (dd, J = 11.6, 7.9 Hz, 1H), 4.06 (d, J = 2.0 Hz, 3H).

### Intermediate 27

### 3,7-Difluoro-2-methyl-4-(4,4,5,5-tetramethyl-1,3,2-dioxaborolan-2-yl)-2H-indazole

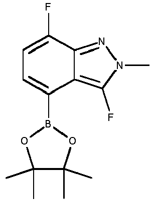

Using the method described for Intermediate 23: Intermediate 26 (35 mg, 0.14 mmol) gave the title compound (47 mg).

### Intermediate 28

### 5,5'-Difluoro-2',7-dimethyl-1H,2'H-3,4'-biindazole

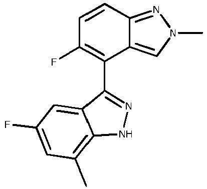

To a solution of Intermediate 9 (1.2 g, 4.58 mmol) in dioxane (5 ml) and water (5 ml) was added N-fluorobenzenesulfonimide (4.42 gm 13.72 mmol) and heated in a sealed vessel at 90 °C for 16 h. Numerous fluorinated compounds were isolated: 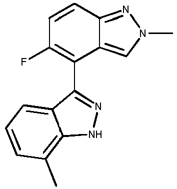 40 mg
HPLC (Acidic): Rt = 0.992 min ((M+H)+ 281.0) 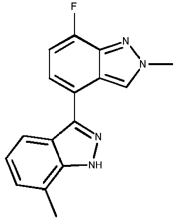 14 mg
HPLC (Acidic): Rt = 1.022 min ((M+H)+ 281.0) 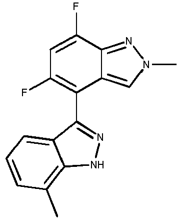 30 mg
HPLC (Acidic): Rt = 1.061 min ((M+H)+ 299.0) 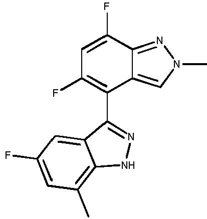 5 mg
HPLC (Acidic): Rt = 1.061 min ((M+H)+ 317.0) 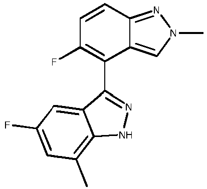 Titled compound: 7 mg
HPLC (Acidic): Rt = 1.061 min ((M+H)+ 299.0)

### Intermediate 29

### 5,5',7'-Trifluoro-2',7-dimethyl-1H,2'H-3,4'-biindazole

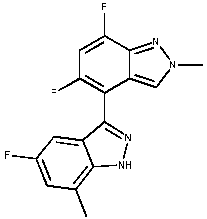

The synthesis of this intermediate is described as for Intermediate 28, and 5 mg were isolated.

HPLC (Acidic): Rt = 1.061 min ((M+H)+ 317.0)

### Intermediate 30

### 4-Bromo-6-fluoro-2-methyl-2H-indazole

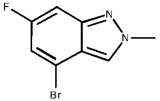

To a stirred solution of 4-bromo-6-fluoro-2H-indazole (4.1 g, 19.1 mmol, 1 equiv.) in EtOAc (40 mL) was added trimethyloxonium tetrafluoroborate (3.38 g, 22.9 mmol, 1.2 equiv.) in portions at 0 °C. The reaction solution was stirred overnight at room temperature. The resulting mixture was diluted with water (40 mL) and extracted with EtOAc (3 x 100 mL). The combined organic layers were washed with brine (1x100 mL) and dried over anhydrous Na₂SO₄. After filtration, the filtrate was concentrated under reduced pressure. The residue was purified by silica gel column chromatography, eluted with Petroleum Ether / EtOAc (10:1) to afford 4-bromo-6-fluoro-2-methylindazole (3.7 g) as white solid.

1H NMR (400 MHz, Chloroform-d) δ 7.93 (s, 1H), 7.27 (ddd, J = 9.6, 2.0, 1H), 7.12 (dd, J = 8.8, 2.0 Hz, 1H), 4.22 (s, 3H).

### Intermediate 31

### 6-Fluoro-2-methyl-4-(4,4,5,5-tetramethyl-1,3,2-dioxaborolan-2-yl)-2H-indazole

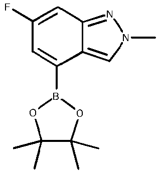

To a stirred solution of 4-bromo-6-fluoro-2-methylindazole (2.6 g, 11.4 mmol, 1 equiv.) and bis(pinacolato)diboron (4.32 g, 17.1 mmol, 1.5 equiv.) in 1,4-dioxane (26 mL) were added potassium acetate (3.34 g, 34.053 mmol, 3 equiv.) and Pd(dppf)Cl₂ (0.83 g, 1.14 mmol, 0.1 equiv.) at room temperature under nitrogen atmosphere. The resulting mixture was stirred for 4 h at 80 °C under nitrogen atmosphere. The resulting mixture was concentrated under reduced pressure. The residue was purified by silica gel column chromatography, eluted with Petroleum Ether / THF (15:1) to afford a crude product. The crude product was further purified by trituration with n-Hexane (5 mL) to afford 6-fluoro-2-methyl-4-(4,4,5,5-tetramethyl-1,3,2-dioxaborolan-2-yl)indazole (1.56 g) as white solid.

1H NMR (400 MHz, Chloroform-d) δ 8.26 (s, 1H), 7.44-7.38 (m, 2H), 4.24 (s, 3H), 1.41 (s, 12H).

### Intermediate 32

### 1-(6-fluoropyridin-3-yl)-3-iodo-7-methyl-1H-indazole

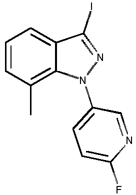

To a stirred solution of 3-iodo-7-methylindazole (5.0 g, 19.4 mmol, 1 equiv.) in DCM (75 mL) was added 2 (6-fluoropyridin-3-yl)boronic acid (6.83g, 48.4 mmol), copper(II) acetate (5.28g, 29.1 mmol) and pyridine (5.47 mL) and the reaction was stirred at RT for 16h. The reaction was concentrated under reduced pressure and redissolved in EtOAc and stirred with 2 mol/L aq. NaOH-sol. (half saturated with NaCl) and the precipitate formed was removed by filtration. The EtOAc phase was washed with 2 mol/L aq. NaOH-sol. (half saturated with NaCl), then washed with 2 x 2 mol/L aq. HCl-sol. (half saturated with NaCl). The organic layer was dried over Na₂SO₄, filtered and evaporated to dryness. The crude product was heated under reflux conditions in 100 mL EtOH (clear brown solution) and slowly cooled to RT overnight, the resulting precipitate was collected by filtration, washed three timed with 5 mL cold EtOH and dried in a vacuum oven to give a white solid (3.80 g).

¹H NMR (400 MHz, DMSO-*d*₆) δ ppm 2.09 (s, 3 H), 7.25 (m, 1 H), 7.34 (m, 1 H), 7.42 (m, 2 H), 8.28 (m, 1 H), 8.53 (d, *J*=2.41 Hz, 1 H).

### Intermediate 33 and Intermediate 34

### Methyl 2-[5-(3-iodo-7-methyl-1H-indazol-1-yl)pyridin-2-yl]-2-azabicyclo[2.2.2]octane-5-carboxylate

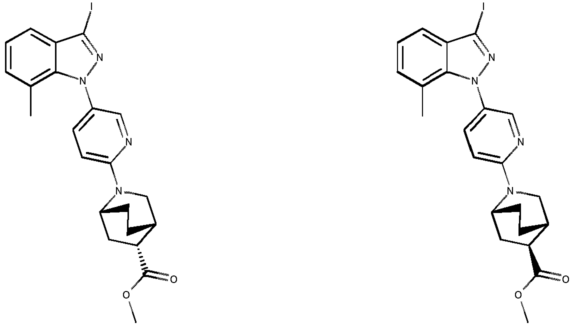

### Racemic Intermediate 33 Racemic Intermediate 34

To a solution of 2-tert-butyl 5-methyl 2-azabicyclo[2.2.2]octane-2,5-dicarboxylate (723 mg, 2.55 mmol) was added TFA (2 mL) and stirred at RT for 30mins. The reaction was concentrated under reduced pressure. To the residue was added a solution of Intermediate 32 (600 mg, 1.7 mmol, 1 equiv.), DIPEA (780 µL, 4.59 mmol) in anhydrous NMP (8 mL). The resulting mixture was stirred for 16 h at 140 °C. The resulting mixture was acidified with TFA and purified by preparative HPLC (acidic) to give a mixture of Intermediate 33 (240 mg) and Intermediate 34 (200 mg).
Intermediate 33
   HPLC (Acidic): Rt = 1.568 min ((M+H)+ 503)
Intermediate 34
   HPLC (Acidic): Rt = 1.596 min ((M+H)+ 503)

### Intermediate 35 and Intermediate 36

### Methyl (1R,4S,5R)-2-[5-(3-iodo-7-methyl-1H-indazol-1-yl)pyridin-2-yl]-2-azabicyclo[2.2.2]octane-5-carboxylate

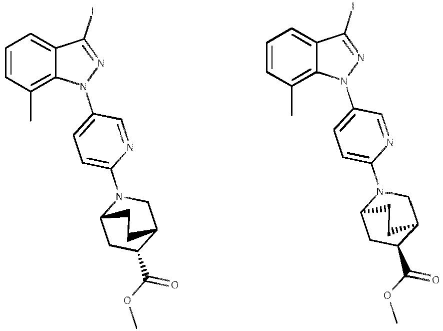

Intermediate 33 (270 mg) was subjected to chiral preparative HPLC and Intermediate 35 (93 mg) and Intermediate 36 (78 mg) was isolated.

### Chiral Preparative HPLC

| | |
|---|---|
| HPLC: | Sepiatec PrepSFC100 |
| Column: | CHIRAL ART^{®} Cellulose-SC_20 x 250 mm_5µm |
| Eluent: | A: 65% scCO2; B: 35% MeOH+20mM NH₃ |
| Detection: | UV: 254 nm |
| Column temp.: | 40 °C |
| Flow: | 60 ml/min |
| Gradient: | isocratic |

### Intermediate 37 and Intermediate 38

### Methyl (1R,4S,5S)-2-[5-(3-iodo-7-methyl-1H-indazol-1-yl)pyridin-2-yl]-2-azabicyclo[2.2.2]octane-5-carboxylate

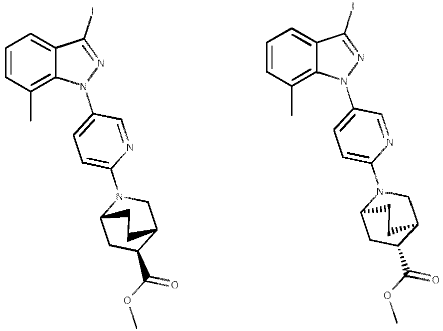

Intermediate 34 (200 mg) was subjected to chiral preparative HPLC and Intermediate 37 (51.6 mg) and Intermediate 38 (103 mg) was isolated.

### Chiral Preparative HPLC

| | |
|---|---|
| HPLC: | Sepiatec PrepSFC100 |
| Column: | CHIRAL ART^{®} Cellulose-SC_20 x 250 mm_5µm |
| Eluent: | A: 60% scCO2; B: 40% MeOH+20mM NH₃ |
| Detection: | UV: 254 nm |
| Column temp.: | 40 °C |
| Flow: | 60 ml/min |
| Gradient: | isocratic |

### Intermediate 39

### tert-butyl (1S,4S)-5-[5-(3-iodo-7-methyl-1H-indazol-1-yl)pyridin-2-yl]-2,5-diazabicyclo[2.2.1]heptane-2-carboxylate

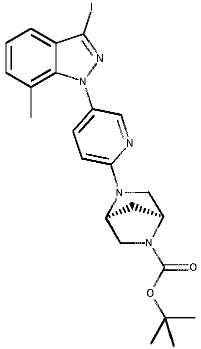

To a stirred solution of Intermediate 32 (500 mg, 1.42 mmol, 1 equiv.) in anhydrous NMP (5 mL) was added DIPEA (366 mg, 2.83 mmol) and tert-butyl (1S,4S)-2,5-diazabicyclo-[2.2.1]heptane-2-carboxylate (434 mg, 2.12 mmol).The resulting mixture was stirred for 15 h at 80 °C. The reaction mixture was diluted with 50 ml water and extracted 2 times with EtOAc. The organics were combined and were washed with water, 10 % LiCl solution, sat. NaCl(aq), dried over MgSO₄, filtered and concentrated under reduced pressure. The desired compound (607 mg, 81%) was isolated after silica chromatography (Cyclohexane:EtOAc).

¹H NMR (400 MHz, DMSO-*d*₆) δ ppm 1.39 (m, 9 H), 1.97 (br s, 2 H), 2.10 (s, 3 H), 3.24 (m, 1 H), 3.38 (m, 2 H), 3.57 (br d, *J*=6.84 Hz, 1 H), 4.49 (br d, *J*=13.31 Hz, 1 H), 4.89 (br s, 1 H), 6.67 (br d, *J*=8.87 Hz, 1 H), 7.19 (m, 1 H), 7.26 (d, *J*=6.97 Hz, 1 H), 7.35 (d, *J*=7.98 Hz, 1 H), 7.68 (dd, *J*=8.87, 2.66 Hz, 1 H), 8.22 (d, *J*=2.53 Hz, 1 H).

### Intermediate 40

### 1-{6-[(1S,4S)-2,5-diazabicyclo[2.2.1]heptan-2-yl]pyridin-3-yl}-3-iodo-7-methyl-1H-indazole trifluoroacetic acid salt

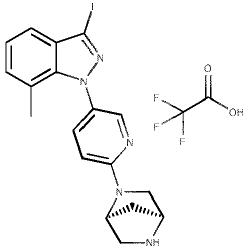

To a stirred solution of Intermediate 39 (632 mg, 1.19 mmol, 1 equiv.) in anhydrous DCM (7 mL) was added TFA (3 mL) and stirred for 1 h at RT. The reaction mixture was concentrated under reduced pressure and used directly in the next step (513 mg, 79%).

### Intermediate 41

### 1-[(1S,4S)-5-[5-(3-iodo-7-methyl-1H-indazol-1-yl)pyridin-2-yl]-2,5-diazabicyclo[2.2.1]heptan-2-yl]ethan-1-one

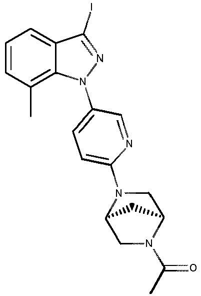

To a stirred solution of Intermediate 40 (510 mg, 0.935 mmol) in anhydrous MeTHF (5 mL) was added DIPEA (971 µL, 5.61 mmol) and acetic anhydride (133 µL, 1.4 mmol). The resulting mixture was stirred for 1 h at RT. The reaction mixture was diluted DCM and washed with 50 ml water. The organics were dried over MgSO₄, filtered and concentrated under reduced pressure. The desired compound was isolated after silica chromatography (MeOH:EtOAc).

HPLC (Basic): Rt = 2.05 min ((M+H)+ 474.0)

### Intermediate 42

### tert-butyl (1R,4R)-5-[5-(3-iodo-7-methyl-1H-indazol-1-yl)pyridin-2-yl]-2,5-diazabicyclo[2.2.1]heptane-2-carboxylate

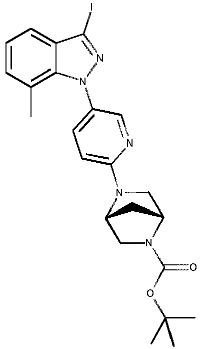

To a stirred solution of Intermediate 32 (500 mg, 1.42 mmol, 1 equiv.) in anhydrous NMP (5 mL) was added DIPEA (366 mg, 2.83 mmol) and tert-butyl (1R,4R)-2,5-diazabicyclo-[2.2.1]heptane-2-carboxylate (434 mg, 2.12 mmol).The resulting mixture was stirred for 15 h at 80 °C. The reaction mixture was diluted with 50 ml water and extracted 2 times with EtOAc. The organics were combined and were washed with water, 10 % LiCl solution, sat. NaCl(aq), dried over MgSO₄, filtered and concentrated under reduced pressure. The desired compound (637 mg, 85%) was isolated after silica chromatography (Cyclohexane:EtOAc).

¹H NMR (400 MHz, DMSO-*d*₆) δ ppm 1.39 (m, 9 H), 1.97 (m, 2 H), 2.10 (s, 3 H), 3.25 (m, 1 H), 3.36 (m, 2 H), 3.58 (br d, *J*=6.59 Hz, 1 H), 4.49 (br d, *J*=13.31 Hz, 1 H), 4.89 (br s, 1 H), 6.67 (br d, *J*=8.74 Hz, 1 H), 7.18 (t, *J*=7.32 Hz, 1 H), 7.26 (d, *J*=6.97 Hz, 1 H), 7.35 (d, *J*=7.98 Hz, 1 H), 7.68 (dd, *J*=8.87, 2.66 Hz, 1 H), 8.22 (d, *J*=2.53 Hz, 1 H).

### Intermediate 43

### tert-butyl (1R,4R)-5-[5-(3-iodo-7-methyl-1H-indazol-1-yl)pyridin-2-yl]-2,5-diazabicyclo[2.2.1]heptane-2-carboxylate

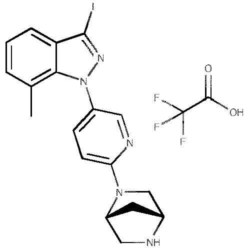

To a stirred solution of Intermediate 39 (632 mg, 1.19 mmol, 1 equiv.) in anhydrous DCM (7 mL) was added TFA (3 mL) and stirred for 1 h at RT. The reaction mixture was concentrated under reduced pressure and used directly in the next step (513 mg, 79%).

### Intermediate 44

### tert-butyl (1R,4R)-5-[5-(3-iodo-7-methyl-1H-indazol-1-yl)pyridin-2-yl]-2,5-diazabicyclo[2.2.1]heptane-2-carboxylate

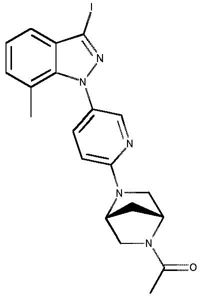

To a stirred solution of Intermediate 43 (510 mg, 0.935 mmol) in anhydrous MeTHF (5 mL) was added DIPEA (971 µL, 5.61 mmol) and acetic anhydride (133 µL, 1.4 mmol). The resulting mixture was stirred for 1 h at RT. The reaction mixture was diluted DCM and washed with 50 ml water. The organics were dried over MgSO₄, filtered and concentrated under reduced pressure. The desired compound was isolated after silica chromatography (MeOH:EtOAc).

HPLC (Acidic): Rt = 0.874 min ((M+H)+ 474.0)

### Intermediate 45

### 5-fluoro-2-methyl-4-(4,4,5,5-tetramethyl-1,3,2-dioxaborolan-2-yl)-2H-indazole

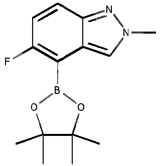

To a stirred solution of 4-bromo-5-fluoro-2-methylindazole (WO2022221556 A1, 1.00 g, 4.32 mmol) and bis(pinacolato)diboron (1.7 g, 6.48 mmol) in dioxane (10 mL) was added KOAc (1.697 g, 17.29 mmol) and then degassed by bubbling Argon throught the solution. Then [1,1'-bis(diphenylhosphino)ferrocene]dichloropalladium(II) complex with DCM (1:1) (353 mg, 0.432 mmol) was added and the reaction heated and stirred for 16h at at 90 °C. The reaction mixture was diluted with 50 ml water and extracted with EtOAc. The organics were combined and were washed with water, sat. NaCl(aq), dried over MgSO₄, filtered and concentrated under reduced pressure. The desired compound (939 mg, 79%) was isolated after silica chromatography (Cyclohexane:EtOAc).

HPLC (Acidic): Rt = 1.022 min ((M+H)+ 277.2)

### Intermediate 46 and Intermediate 47

### methyl (1S,4R,5R)-2-[5-(3-iodo-7-methyl-1H-indazol-1-yl)pyridin-2-yl]-2-azabicyclo[2.2.1]heptane-5-carboxylate methyl (1R,4S,5S)-2-[5-(3-iodo-7-methyl-1H-indazol-1-yl)pyridin-2-yl]-2-azabicyclo[2.2.1]heptane-5-carboxylate

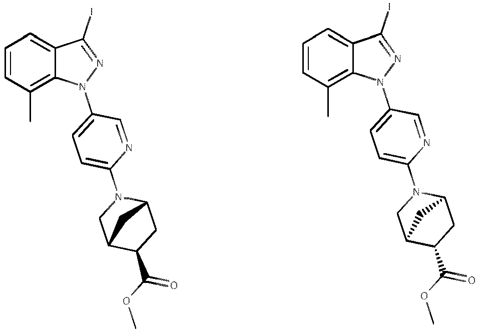

To a stirred solution of Intermediate 32 (260 mg, 0.74 mmol) in anhydrous NMP (3 mL) was added DIPEA (338 µL, 1.99 mmol) and (rel)-methyl (1S,4R,5R)-2-azabicyclo[2.2.1]heptane-5-carboxylate hydrochloride (155 mg, 0.81 mmol).The resulting mixture was stirred for 15 h at 95 °C. The reaction mixture was acidified with TFA and purified by preparative HPLC (acidic) and gave the titled product which was purified by chiral HPLC chromatography and gave Intermediate 46 (110 mg) and Intermediate 47 (112 mg).

### Chiral Preparative HPLC

| | |
|---|---|
| HPLC: | Sepiatec PrepSFC100 |
| Column: | Lux^{®} Cellulose-3_21.2 x 250 mm_5µm |
| Eluent: | A: 85% scCO2; B: 15% MeOH+20mM NH₃ |
| Detection: | UV: 254 nm |
| Column temp.: | 40 °C |
| Flow: | 60 ml/min |
| Gradient: | isocratic |

### Intermediate 48

### 6'-fluoro-1-(6-fluoropyridin-3-yl)-2',7-dimethyl-1H,2'H-3,4'-biindazole

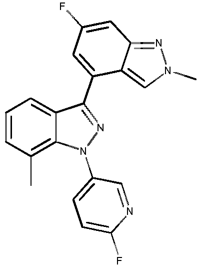

To a stirred solution of Intermediate 3 (1 g, 2.832 mmol) in dioxane (4 mL) and water (1 mL) was added 6-fluoro-2-methyl-4-(4,4,5,5-tetramethyl-1,3,2-dioxaborolan-2-yl)indazole (Intermediate 31, 0.957 mg, 3.4 mmol) and K₂CO₃ (1.174 g, 8.5 mmol) and degassed with bubbling Argon through the solution. Then Pd(dppf)Cl₂ (0.104 g, 0.14 mmol) was added and the reaction heated at 100 °C for 3h. The reaction mixture was diluted with EtOAc and filtered over celite. The remaining organic layer was washed twice with 1 mol/L aq. NaOH-sol. 100 mg active charcoal were added to the organic layer and the organic layer was dried over Na₂SO₄, filtered and concentrated under reduced pressure. The desired compound was isolated after silica chromatography (MeOH:EtOAc). The crude product was heated under reflux conditions in 35 mL iPrOH and slowly cooled to RT over 3 h, the precipitate was collected by filtration (800 mg; 75%).

HPLC (Acidic): Rt = 1.133 min ((M+H)+ 376.2)

### Intermediate 49 and Intermediate 50

### 2-tert-butyl 5-methyl (1S,4R,5R)-2-azabicyclo[2.2.1]heptane-2,5-dicarboxylate and

### 2-tert-butyl 5-methyl ()-2-azabicyclo[2.2.1]heptane-2,5-dicarboxylate

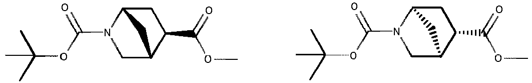

To a stirred mixture of methyl (rel-1S,4R,5R)-2-azabicyclo[2.2.1]heptane-5-carboxylate hydrohloride (Enamine, 100 mg, 0.522 mmol) in DCM (3 mL) was added TEA (147 µL, 1.044 mmol) and Boc-anhydride (125.3 mg, 0.574 mmol). The resulting mixture was stirred for 30 min at RT. The reaction mixture was concentrated DCM and gave the titled compound (105 mg, 79%) after preparative HPLC chromatography (Basic).

### Intermediate 51 and Intermediate 52

### (1S,4R,5R)-2-[(tert-butoxy)carbonyl]-2-azabicyclo[2.2.1]heptane-5-carboxylic acid and

### (1R,4S,5S)-2-[(tert-butoxy)carbonyl]-2-azabicyclo[2.2.1]heptane-5-carboxylic acid

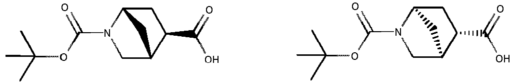

To a stirred solution of Intermediate 49 and Intermediate 50 (70 mg, 0.274 mmol) in DMF (1 mL) was added aqueous NaOH (4M, 343 µL, 1.37 mmol). The resulting mixture was stirred for 2 h at RT. The reaction mixture was acidified with TFA and the titled compound (44 mg, 67%) was isolated after preparative HPLC chromatography (Acidic).

Intermediate 51 and Intermediate 52 (60 mg) was subjected to chiral preparative HPLC and Intermediate 51 (16 mg) and Intermediate 52 (26 mg) was isolated.

### Chiral Preparative HPLC

| | |
|---|---|
| HPLC: | Sepiatec PrepSFC100 |
| Column: | Chiralpak^{®} IG_10 x 250 mm_5µm |
| Eluent: | A: 90% scCO2; B: 10% MeOH+20mM NH₃ |
| Detection: | UV: 220 nm |
| Column temp.: | 40 °C |
| Flow: | 15 ml/min |
| Gradient: | isocratic |

Intermediate 51: ¹H NMR (400 MHz, DMSO-*d*₆) δ ppm 1.40 (m, 11 H), 1.79 (m, 2 H), 2.50 (m, 2 H), 2.69 (br s, 1 H), 2.94 (d, *J*=9.63 Hz, 1 H), 3.13 (m, 1 H), 4.07 (br d, *J*=12.93 Hz, 1 H), 12.21 (br s, 1 H)

### Intermediate 53

### (1S,4R,5R)-2-azabicyclo[2.2.1]heptane-5-carboxylic acid

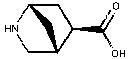

To a stirred solution of Intermediate 51 (12.3 mg, 0.051 mmol) in DCM (2 mL) was added TFA (500 µL) and stirred for 1 h at RT. The reaction mixture was concentrated under reduced pressure and used directly in the next step.

### Intermediate 54

### (1R,4S,5S)-2-azabicyclo[2.2.1]heptane-5-carboxylic acid

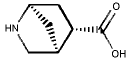

To a stirred solution of Intermediate 52 (26 mg, 0.108 mmol) in DCM (2 mL) was added TFA (500 µL) and stirred for 1 h at RT. The reaction mixture was concentrated under reduced pressure and used directly in the next step.

HPLC (Acidic): Rt = 0.085 min ((M+H)+ 142.0)

### Intermediate 55

### (3-bromo-2,5,6-trifluorophenyl)trimethylsilane

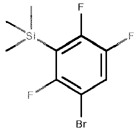

To a stirred solution of 1-bromo-2,4,5-trifluorobenzene (400 g, 1.89 mol, 1 equiv) in THF (4000 mL) was added dropwise LDA (1.14 L, 2.27 mol, 1.2 equiv, 2 mol/L) at -78 °C under nitrogen. The resulting mixture was stirred for 2 h at -78 °C. The mixture was added dropwise TMSCI (534 g, 4.914 mol, 2.6 equiv) at -78 °C and stirred for additional 0.5 h at - 78 °C. The mixture was allowed to room temperature and stirred for 0.5 h at room temperature. The mixture was quenched by ice water (2 L), extracted with EtOAc (2L x 3). The organic layer was dried over Na₂SO₄, filtrated, concentrated under vacuum. The residue was purified by silica gel column chromatography, eluted with PE: EA (10:1) to afford (3-bromo-2,5,6-trifluorophenyl)trimethylsilane (400 g, 74.48%) as a light yellow oil.

### Intermediate 56

### 2-bromo-3,5,6-trifluoro-4-(trimethylsilyl)benzaldehyde

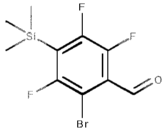

To a stirred solution of (3-bromo-2,5,6-trifluorophenyl)trimethylsilane (400 g, 1.418 mol, 1 equiv) in THF (4000 mL) was added dropwise LDA (851 mL, 1.7 mol, 1.2 equiv, 2 mol/L) at -78 °C under nitrogen. The resulting mixture was stirred for 1 h at -78 °C. Into the mixture was added dropwise DMF (517 g, 7.09 mol, 5 equiv) at -78 °C and stirred for additional 0.5 h at -78 °C. The resulting mixture was quenched by sat. NH₄Cl (1 L), extracted with EtOAc (2L x3). The organic phase was dried over Na₂SO₄, filtrated, concentrated under vacuum. The residue was purified by silica gel column chromatography, eluted with Petroleum Ether: EtOAc (10:1) to afford (3-bromo-2,5,6-trifluorophenyl)trimethylsilane (350 g, 79.55%) as a light yellow oil.

### Intermediate 57

### {[2-bromo-3,5,6-trifluoro-4-(trimethylsilyl)phenyl]methylidene}(methoxy)amine

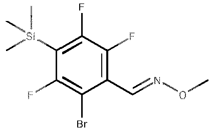

To a stirred solution of 2-bromo-3,5,6-trifluoro-4-(trimethylsilyl)benzaldehyde (300 g, 964.094 mmol, 1 equiv) and O-methylhydroxylamine hydrochloride (88.57 g, 1060.503 mmol, 1.1 equiv) in DME (3000 mL) was added K₂CO₃ (199.86 g, 1446.141 mmol, 1.5 equiv). The resulting mixture was stirred for 2 h at 50°C. The mixture was allowed to cool down to room temperature and diluted with EtOAc (1L). The resulting mixture was washed with water (1L). The organic layer was concentrated under vacuum to afford crude product (E)-([2-bromo-3,5,6-trifluoro-4-(trimethylsilyl)phenyl] methylidene-(methoxy)amine (300 g, brown oil). The crude product was used in the next step directly without further purification.

### Intermediate 58

### 4-bromo-5,7-difluoro-1H-indazole

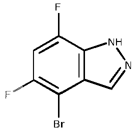

A solution of (E)-([2-bromo-3,5,6-trifluoro-4-(trimethylsilyl)phenyl]methylidene-(methoxy)amine (300 g, crude) in hydrazine hydrate (1500 mL) and DME (1500 mL) was stirred for 16 h at 80 °C. The resulting mixture was diluted with EtOAc (1L), and washed with water (1 L). The organic layers were dried over anhydrous Na₂SO₄. After filtration, the filtrate was concentrated under reduced pressure. The residue was purified by silica gel column chromatography, eluted with Petroleum Ether: EtOAc (1:1) to afford 4-bromo-5,7-difluoro-2H-indazole (41 g, 19.95%) as a white solid.

### Intermediate 59

### 4-bromo-5,7-difluoro-2-methyl-2H-indazole

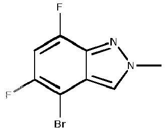

To a stirred solution of 4-bromo-5,7-difluoro-2H-indazole (40 g, 171.662 mmol, 1 equiv) in EA (800 mL) was added trimethyloxonium tetrafluoroborate (38.09 g, 257.493 mmol, 1.5 equiv) at 0 °C. The resulting mixture was stirred for 16 h at room temperature. The reaction mixture was diluted with and EtOAc (500 mL), and washed with water (500 mL) The organic layer was concentrated under reduced pressure. The residue was purified by silica gel column chromatography, eluted with Petroleum Ether: EtOAc (5:1) to afford 4-bromo-5,7-difluoro-2-methylindazole (27 g, 63.67%) as a white solid and 4-bromo-5,7-difluoro-1-methyl-1H-indazole (1.5 g, 3.53%) as a white solid.

1H NMR (400 MHz, Chloroform-δ) δ 8.00 (d, J = 2.5 Hz, 1H), 6.90 (t, J = 9.7 Hz, 1H), 4.27 (s, 3H).

### Intermediate 60

### 5,7-difluoro-2-methyl-4-(4,4,5,5-tetramethyl-1,3,2-dioxaborolan-2-yl)-2H-indazole

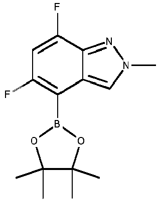

To a solution of 4-bromo-5,7-difluoro-2-methylindazole (20 g, 80.958 mmol, 1 equiv) and bis(pinacolato)diboron (41.12 g, 161.916 mmol, 2 equiv) in dioxane (400 mL) were added KOAc (15.89 g, 161.916 mmol, 2 equiv) and Pd(dppf)Cl₂.DCM (6.59 g, 8.096 mmol, 0.1 equiv). After stirring for 16 h at 90 °C under a nitrogen atmosphere, the resulting mixture was concentrated under reduced pressure. The residue was purified by silica gel column chromatography, eluted with Petroleum Ether: EtOAc (5:1) to afford 5,7-difluoro-2-methyl-4-(4,4,5,5-tetramethyl-1,3,2-dioxaborolan-2-yl)indazole (5.1336 g, 21.56%) as a white solid.

1H NMR (400 MHz, Chloroform-δ) δ 8.28 (d, J = 2.7 Hz, 1H), 6.79 (t, J = 10.3 Hz, 1H), 4.27 (s, 3H), 1.42 (s, 12H).

### Intermediate 61

### tert-butyl (1S,4S)-5-(5-{5',7'-difluoro-2',7-dimethyl-1H,2'H-[3,4'-biindazol]-1-yl}pyridin-2-yl)-2,5-diazabicyclo[2.2.1]heptane-2-carboxylate

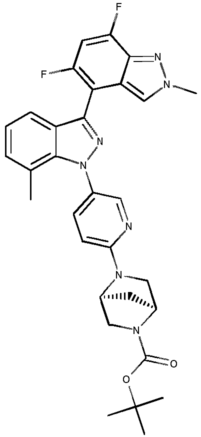

To a mixture of Intermediate 13 (15 mg, 0.038 mmol) and (1S,4S)-2-Boc-2,5-diazabicyclo[2.2.1]heptane (24 mg, 0.114 mmol) in MeCN (1.5 ml) was added DIPEA (24.6 mg, 0.191 mmol) and the mixture was heated at 105 °C for 16 h. The reaction was allowed to cool and then concentrated and used directly in the next step without further purification.

HPLC (Acidic): Rt = 1.075 min ((M+H)+ 572.2)

### Intermediate 62

### 1-{6-[(1S,4S)-2,5-diazabicyclo[2.2.1]heptan-2-yl]pyridin-3-yl}-5',7'-difluoro-2',7-dimethyl-1H,2'H-3,4'-biindazole

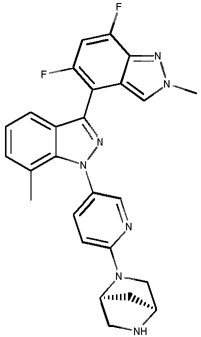

To a stirred solution of Intermediate 61 (12.3 mg, 0.051 mmol) in DCM (2 mL) was added TFA (500 µL) and stirred for 1 h at RT. The reaction mixture was concentrated under reduced pressure and used directly in the next step.

HPLC (Acidic): Rt = 0.877 min ((M+H)+ 472.2)

### Intermediate 63

### tert-butyl (1R,4R)-5-(5-{5',7'-difluoro-2',7-dimethyl-1H,2'H-[3,4'-biindazol]-1-yl}pyridin-2-yl)-2,5-diazabicyclo[2.2.1]heptane-2-carboxylate

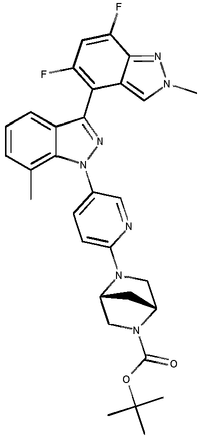

To a mixture of Intermediate 13 (10 mg, 0.025 mmol) and (1R,4R)-2-Boc-2,5-diazabicyclo[2.2.1]heptane (15.6 mg, 0.076 mmol) in MeCN (1.5 ml) was added DIPEA (16.4 mg, 0.127 mmol) and the mixture was heated at 105 °C for 16 h. The reaction was allowed to cool and then concentrated and used directly in the next step without further purification.

HPLC (Acidic): Rt = 1.077 min ((M+H)+ 572.2)

### Intermediate 64

### 1-{6-[(1R,4R)-2,5-diazabicyclo[2.2.1]heptan-2-yl]pyridin-3-yl}-5',7'-difluoro-2',7-dimethyl-1H,2'H-3,4'-biindazole

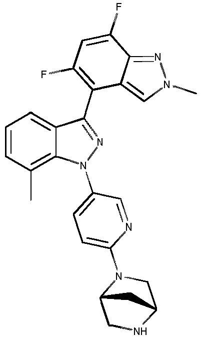

To a stirred solution of Intermediate 63 (17.4 mg, 0.020 mmol) in DCM (2 mL) was added TFA (500 µL) and stirred for 1 h at RT. The reaction mixture was concentrated under reduced pressure and used directly in the next step.

HPLC (Acidic): Rt = 0.871 min ((M+H)+ 472.2)

### Intermediate 65

### 1-(6-{2,5-diazabicyclo[2.2.2]octan-2-yl}pyridin-3-yl)-5',7'-difluoro-2',7-dimethyl-1H,2'H-3,4'-biindazole

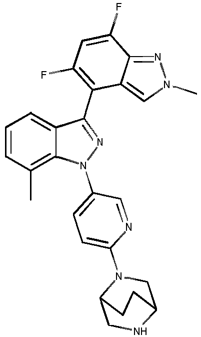

To a mixture of Intermediate 13 (10 mg, 0.025 mmol) and 2,5-diazabicyclo [2.2.2]octane dihydrochloride (9.9 mg, 0.051 mmol) in NMP (1.5 ml) was added DIPEA (16.4 mg, 0.127 mmol) and the mixture was heated at 105 °C for 3 h. The reaction was allowed to cool and used directly in the next step without further purification.

HPLC (Acidic): Rt = 0.904 min ((M+H)+ 486.2)

### Intermediate 66

### 3',7'-difluoro-1-(6-fluoropyridin-3-yl)-2',7-dimethyl-1H,2'H-3,4'-biindazole

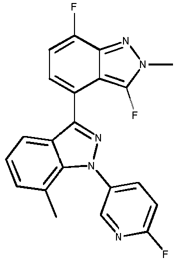

Using the method described for Intermediate 11: Intermediate 3 (500 mg) and Intermediate 27 (517 mg) gave the title compound (435 mg).

¹H NMR (400 MHz, DMSO-*d*₆) δ ppm 2.18 (s, 3 H), 4.07 (d, *J*=1.90 Hz, 3 H), 7.25 (m, 2 H), 7.32 (m, 1 H), 7.46 (m, 2 H), 7.88 (d, *J*=7.98 Hz, 1 H), 8.34 (ddd, *J*=8.68, 7.03, 2.79 Hz, 1 H), 8.59 (d, *J*=2.41 Hz, 1 H).

### Intermediate 67

### 5-Fluoro-1-(6-fluoropyridin-3-yl)-3-iodo-7-methyl-1H-indazole

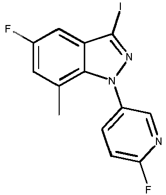

Using the method described for Intermediate 11: Intermediate 16 (10 g, 35.86 mmol) and 2-fluoropyridine-5- boronic acid (12.89 g, 89.66 mmol) gave the title compound (7.12 g, 57%) after recrystallization in EtOH.

¹H NMR (400 MHz, DMSO-*d*₆) δ ppm 2.09 (s, 3 H), 7.18 (dd, J=8.11, 2.28 Hz, 1 H), 7.31 (dd, *J*=9.89, 1.39 Hz, 1 H), 7.43 (dd, J=8.62, 3.04 Hz, 1 H), 8.29 (m, 1 H), 8.53 (d, *J*=2.15 Hz, 1 H).

### Intermediate 68

### 3',5-difluoro-1-(6-fluoropyridin-3-yl)-2',7-dimethyl-1H,2'H-3,4'-biindazole

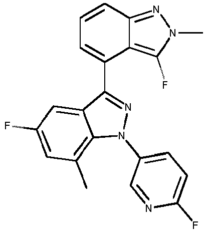

Using the method described for Intermediate 11: Intermediate 67 (1 g) and 3-fluoro-2-methyl-4-(4,4,5,5-tetramethyl-1,3,2-dioxaborolan-2-yl)indazole (920 mg) gave the title compound (200 mg) after recrystallization from EtOH.

¹H NMR (400 MHz, DMSO-*d*₆) δ ppm 2.18 (s, 3 H), 4.03 (d, *J*=1.77 Hz, 3 H), 7.30 (dd, *J*=9.76, 1.39 Hz, 1H), 7.41 (m, 1 H), 7.46 (dd, *J*=8.68, 2.98 Hz, 1 H), 7.51 (d, *J*=6.72 Hz, 1 H), 7.58 (dd, *J*=8.74, 1.27 Hz, 1 H), 7.65 (dd, *J*=8.74, 2.15 Hz, 1 H), 8.36 (ddd, *J*=8.65, 7.07, 2.79 Hz, 1 H), 8.61 (d, *J*=2.28 Hz, 1 H).

### Intermediate 69

### Methyl 1-(5-{3',5-difluoro-2',7-dimethyl-1H,2'H-[3,4'-biindazol]-1-yl}pyridin-2-yl)piperidine-4-carboxylate

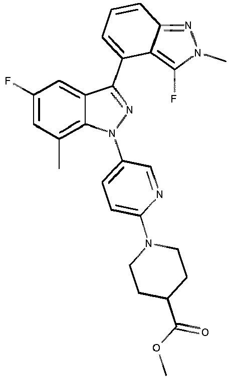

Using the method described for Intermediate 4: Intermediate 68 (60 mg, 0.153 mmol) and methyl piperidine-4-carboxylate hydrochloride (67 mg, 0.458 mmol) gave the title compound (60 mg).

¹H NMR (400 MHz, DMSO-*d*₆) δ ppm 1.62 (m, 1 H), 1.94 (br dd, *J*=13.24, 3.10 Hz, 1 H), 2.19 (s, 1H), 2.70 (m, 1H), 3.10 (m, 1 H), 3.64 (s, 3 H), 4.02 (d, *J*=1.65 Hz, 3 H), 4.30 (m, 1 H), 7.03 (d, *J*=9.13 Hz, 1 H), 7.22 (dd, *J*=9.82, 1.33 Hz, 1 H), 7.40 (m, 1 H), 7.47 (m, 1 H), 7.57 (ddd, *J*=15.18, 8.78, 1.52 Hz, 1 H), 7.79 (dd, *J*=9.06, 2.72 Hz, 1 H), 8.34 (d, *J*=2.66 Hz, 1 H)

### Intermediate 70

### 3',6-difluoro-1-(6-fluoropyridin-3-yl)-2',7-dimethyl-1H,2'H-3,4'-biindazole

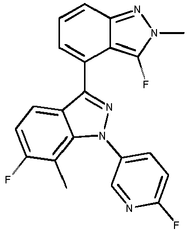

Using the method described for Intermediate 11: Intermediate 19 (1 g) and 3-fluoro-2-methyl-4-(4,4,5,5-tetramethyl-1,3,2-dioxaborolan-2-yl)indazole (920 mg) gave the title compound (766 mg) after recrystallization from isopropanol.

¹H NMR (400 MHz, DMSO-*d*₆) δ ppm 2.05 (m, 3 H), 4.03 (d, *J*=1.77 Hz, 3 H), 7.23 (t, *J*=9.38 Hz, 1 H), 7.42 (m, 1 H), 7.47 (dd, *J*=8.62, 3.04 Hz, 1 H), 7.51 (d, *J*=6.59 Hz, 1 H), 7.60 (dd, *J*=8.68, 1.20 Hz, 1H), 7.91 (dd, *J*=8.87, 4.94 Hz, 1 H), 8.37 (ddd, *J*=8.68, 7.03, 2.79 Hz, 1 H), 8.62 (d, *J*=2.28 Hz, 1 H).

### Intermediate 71

### 5',5-difluoro-1-(6-fluoropyridin-3-yl)-2',7-dimethyl-1H,2'H-3,4'-biindazole

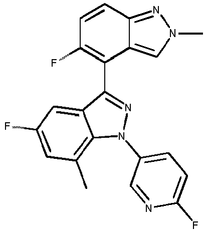

Using the method described for Intermediate 11: Intermediate 67 (70 mg) and Intermediate 45 (90 mg) gave the title compound (73 mg) after preparative HPLC purification (Acidic).

### Intermediate 72

### 5',6-difluoro-1-(6-fluoropyridin-3-yl)-2',7-dimethyl-1H,2'H-3,4'-biindazole

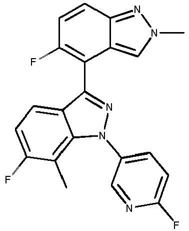

Using the method described for Intermediate 11: Intermediate 19 (70 mg) and Intermediate 45 (90 mg) gave the title compound (72 mg) after preparative HPLC purification (Acidic).

HPLC (Acidic): Rt = 1.127 min ((M+H)+ 394.2)

### Intermediate 73

### 5,5',7'-trifluoro-2',7-dimethyl-1H,2'H-3,4'-biindazole

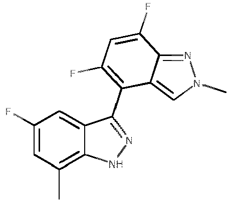

This Intermediate was isolated in the synthesis of Intermediate 13 - see experimental for Intermediate 13.

### Intermediate 74

### 5,5',7'-trifluoro-1-(6-fluoropyridin-3-yl)-2',7-dimethyl-1H,2'H-3,4'-biindazole

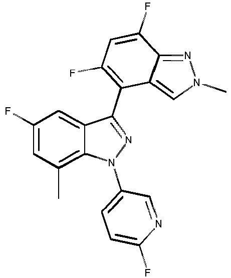

Using the method described for Intermediate 11: Intermediate 73 (180 mg) and 2-fluoropyridine-5- boronic acid (200 mg) gave the title compound (140 mg) after preparative HPLC purification (Acidic).

¹H NMR (400 MHz, DMSO-*d*₆) δ ppm 2.18 (s, 3 H), 4.21 (s, 3 H), 7.30 (br d, *J*=9.89 Hz, 1 H), 7.40 (m, 2 H), 7.48 (dd, *J*=8.68, 2.98 Hz, 1 H), 8.46 (m, 1 H), 8.64 (d, *J*=2.79 Hz, 1 H), 8.72 (d, *J*=2.53 Hz, 1 H).

### Intermediate 75

### 6,7'-difluoro-2',7-dimethyl-1H,2'H-3,4'-biindazole

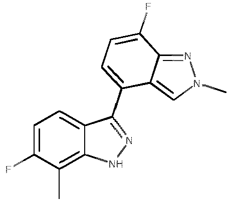

Using the method described for Intermediate 9: Intermediate 18 (1000 mg) and Intermediate 23 (1078 mg) gave the title compound (965 mg) after preparative HPLC purification (Acidic).

¹H NMR (400 MHz, DMSO-*d*₆) δ ppm 2.49 (d, *J*=1.65 Hz, 3 H), 4.27 (s, 3 H), 7.07 (t, *J*=9.31 Hz, 1 H), 7.15 (dd, *J*=11.53, 7.73 Hz, 1 H), 7.64 (dd, *J=*7.79*,* 4.12 Hz, 1 H), 7.96 (dd, *J*=8.87, 4.82 Hz, 1 H), 8.78 (d, *J*=2.79 Hz, 1 H), 13.47 (s, 1 H)

### Intermediate 76

### 5',6,7'-trifluoro-1-(6-fluoropyridin-3-yl)-2',7-dimethyl-1H,2'H-3,4'-biindazole

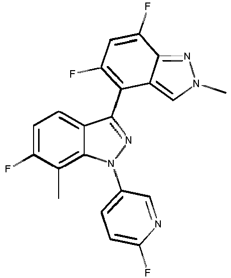

To a solution of intermediate 75 (900 mg, 3.017 mmol) in MeCN (6 ml) and DMF (6 ml) was added SelectFluor (962 mg, 2.72 mmol) and stirred for 90 mins. The reaction was concentrated and purified by silica chromatography (DCM:MeOH) to give the titled compound (180 mg, 19%).

¹H NMR (400 MHz, DMSO-d₆) δ ppm 2.49 (d, *J*=1.77 Hz, 3 H), 4.22 (s, 3 H), 7.04 (t, *J*=9.38 Hz, 1 H), 7.33 (t, *J*=11.15 Hz, 1 H), 7.58 (dt, *J*=8.84, 4.39 Hz, 1 H), 8.54 (d, *J*=2.79 Hz, 1 H), 13.66 (s, 1 H)

The following by-product was also isolated:
3',6,7'-trifluoro-1-(6-fluoropyridin-3-yl)-2',7-dimethyl-1H,2'H-3,4'-biindazole (35 mg, 4%) 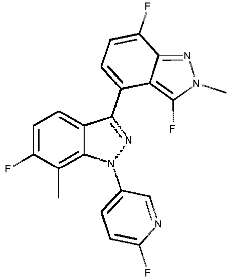
¹H NMR (400 MHz, DMSO-*d*₆) δ ppm 2.47 (m, 3 H), 4.07 (d, *J*=1.90 Hz, 3 H), 7.04 (t, *J*=9.31 Hz, 1 H), 7.19 (dd, *J*=11.53, 7.73 Hz, 1 H), 7.34 (dd, *J=*7.73*,* 4.18 Hz, 1 H), 7.72 (dd, *J*=8.87, 4.82 Hz, 1H), 13.53 (s, 1 H)

### Intermediate 77 and Intermediate 78

### methyl (1R,4S,5S)-2-(5-{3'-fluoro-2',7-dimethyl-1H,2'H-[3,4'-biindazol]-1-yl}pyridin-2-yl)-2-azabicyclo[2.2.1]heptane-5-carboxylate; methyl (1S,4R,5R)-2-(5-{3'-fluoro-2',7-dimethyl-1H,2'H-[3,4'-biindazol]-1-yl}pyridin-2-yl)-2-azabicyclo[2.2.1]heptane-5-carboxylate

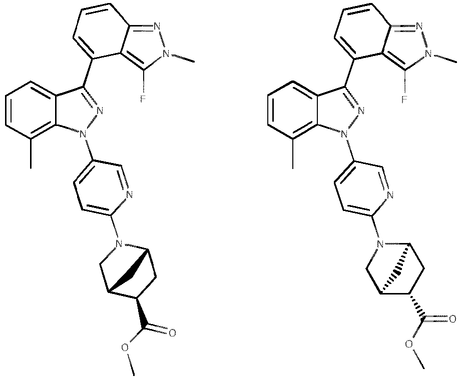

Using the method described for Intermediate 4: Intermediate 6 (45 mg, 0.12 mmol) and methyl -rac (15,4R,5R)-2-azabicyclo[2.2.1]heptane-5-carboxylate hydrochloride (57.4 mg, 0.3 mmol) gave the title compound (55 mg).
HPLC (Acidic): Rt = 0.909 min ((M+H)+ 511.2)
Chiral preparative HPLC and Intermediate 77 (17.5 mg) and Intermediate 78 (15.7 mg) was isolated.

### Chiral Preparative HPLC

| | |
|---|---|
| HPLC: | Sepiatec PrepSFC50_2 |
| Column: | CHIRAL ART^{®} Amylose-C_neo_20 x 250 mm_5µm |
| Eluent: | A: 60% scCO2; B: 40% MeOH+20mM NH₃ |
| Detection: | UV: 220 nm |
| Column temp.: | 40 °C |
| Flow: | 12 ml/min |
| Gradient: | isocratic |

### Intermediate 79

### (3,5-difluoro-2-methyl-2H-indazol-4-yl)boronic acid

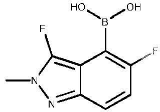

To a solution of intermediate 45 (100 mg, 0.355 mmol) in MeCN (6 ml) was added SelectFluor (962 mg, 2.72 mmol) and stirred for 40 mins. The reaction was concentrated and purified by silica chromatography (DMC:MeOH) to give the titled compound (13 mg, 17%).

HPLC (Acidic): Rt = 0.547 min ((M+H)+ 213.0)

### Intermediate 80

### (3,6-difluoro-2-methyl-2H-indazol-4-yl)boronic acid

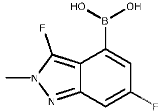

To a solution of intermediate 31 (50 mg, 0.177 mmol) in MeCN (2 ml) was added SelectFluor (75.4 mg, 0.213 mmol) and stirred for 30 mins. The reaction was concentrated and purified by silica chromatography (DCM:MeOH) to give the titled compound (10 mg, 27%).

HPLC (Acidic): Rt = 0.665 min ((M+H)+ 213.0)

### Intermediate 81

### 7'-fluoro-2',7-dimethyl-1H,2'H-3,4'-biindazole

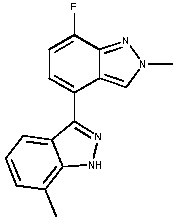

Using the method described for Intermediate 9: 3-iodo-7-methyl-1H-indazole (1000 mg) and Intermediate 23 (1201 mg) gave the title compound (960 mg) after crystallization from water at 70 °C.

¹H NMR (400 MHz, DMSO-*d*₆) δ ppm 2.59 (s, 3 H), 4.28 (s, 3 H), 7.15 (m, 2 H), 7.21 (m, 1 H), 7.66 (dd, *J=*7.79*,* 4.12 Hz, 1 H), 7.95 (d, *J*=8.11 Hz, 1 H), 8.81 (d, *J*=2.79 Hz, 1 H), 13.37 (s, 1 H).

### Intermediate 82

### 3',5',7'-trifluoro-2',7-dimethyl-1H,2'H-3,4'-biindazole

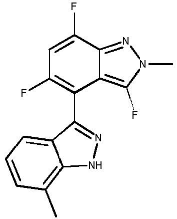

To a solution of intermediate 81 (300 mg, 3.425 mmol) in MeCN (30 ml) was added SelectFluor (970.6 mg, 2.74 mmol) and stirred for 45 mins. An additional portion of SelectFluor was added (260 mg) and stirred for 40 min. The reaction was concentrated and purified by preparative HPL C (Basic) to give the titled compound (8 mg, 1%).

HPLC (Acidic): Rt = 1.011 min ((M+H)+ 317.0)

Also isolated were:
5',7'-difluoro-2',7-dimethyl-1H,2'H-3,4'-biindazole (300 mg, 29%) 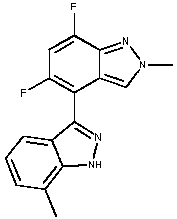
5,5',7'-trifluoro-2',7-dimethyl-1H,2'H-3,4'-biindazole (100 mg, 10%)

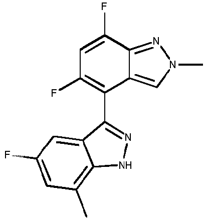

### Intermediate 83

### 5'-chloro-2',7-dimethyl-1H,2'H-3,4'-biindazole

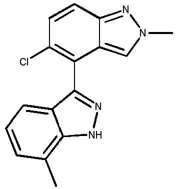

To a solution of intermediate 9 (300 mg, 1.144 mmol) in DMF (5 ml) was added N-chlorosuccinimide (170 mg, 1.258 mmol) and stirred for 3.5 h at 75 °C. The reaction was concentrated and purified by preparative HPL C (Basic) and mixture of two regioisomers 195 mg).

### Preparative HPLC

| | |
|---|---|
| HPLC: | Torus-2-PIC |
| Column: | Torus-2-PIC , 5µm, 30 x 150 mm |
| Eluent: | A: scCO2; B: MeOH+20mM NH₃ |
| Detection: | UV: 220 nm |
| Column temp.: | 40 °C |
| Flow: | 150 ml/min |
| Gradient: | 20 to 80% MeOH in 4 min |
| Back-Pressure: | 1886 psi |
| Intermediate 83 (67 mg, 33%). | |

¹H NMR (400 MHz, DMSO-*d*₆) δ ppm 2.60 (s, 3 H), 4.13 (s, 3 H), 7.05 (dd, *J*=8.11, 6.97 Hz, 1 H), 7.18 (d, *J*=6.84 Hz, 1 H), 7.35 (d, *J*=8.11 Hz, 1 H), 7.40 (d, *J*=9.00 Hz, 1 H), 7.71 (dd, *J*=9.12, 0.89 Hz, 1 H), 8.06 (s, 1 H), 13.50 (s, 1 H).

Also isolated was:
3'-chloro-2',7-dimethyl-1H,2'H-3,4'-biindazole (74 mg, 37%) 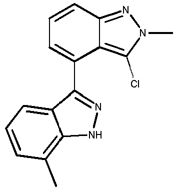
¹H NMR (400 MHz, DMSO-*d*₆) δ ppm 2.58 (s, 3 H), 4.13 (s, 3 H), 7.04 (dd, *J*=8.05, 6.91 Hz, 1 H), 7.17 (d, *J*=6.97 Hz, 1 H), 7.26 (dd, *J*=6.84, 0.76 Hz, 1 H), 7.42 (m, 2 H), 7.70 (dd, *J*=8.62, 0.76 Hz, 1H), 13.36 (s, 1 H)

### Intermediate 84

### 5'-chloro-1-(6-fluoropyridin-3-yl)-2',7-dimethyl-1H,2'H-3,4'-biindazole

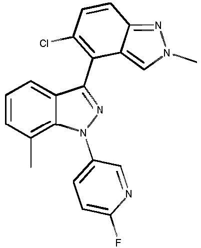

Using the method described for Intermediate 11: Intermediate 83 (67 mg) and 2-fluoropyridine-5- boronic acid (79 mg) gave the title compound (80 mg) after preparative HPLC purification (Acidic).

¹H NMR (400 MHz, DMSO-*d*₆) δ ppm 2.20 (s, 3 H), 4.15 (s, 3 H), 7.21 (dd, *J*=8.05, 7.03 Hz, 1 H), 7.31 (d, *J*=6.97 Hz, 1 H), 7.46 (m, 2 H), 7.51 (d, *J*=7.98 Hz, 1 H), 7.77 (dd, *J*=9.12, 0.89 Hz, 1 H), 8.26 (s, 1 H), 8.46 (ddd, *J*=8.62, 7.10, 2.79 Hz, 1 H), 8.71 (d, *J*=2.16 Hz, 1 H)

### EXAMPLES

### Example 1

### (1R,5S,6R)-3-(5-{3"-Fluoro-2",7-dimethyl-1H,2"H-[3,4"-biindazol]-1-yl}pyridin-2-yl)-3-azabicyclo[3.1.0]hexane-6-carboxylic acid

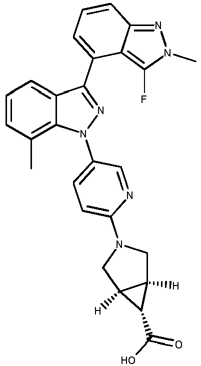

To a solution of Intermediate 5 (55 mg, 0.11 mmol) in EtOH (4 ml) was added a solution of NaOH (aq, 4M(aq), 110.8 µl, 0.44 mmol) and stirred at 80 °C for 2.5 h. The reaction mixture was allowed to cool and then concentrated. The residue was purified by preparative HPLC (acidic method) and the title compound was isolated (24 mg).

1H NMR (400 MHz, DMSO-d6) δ ppm 1.49 (t, J=3.04 Hz, 1 H), 2.19 (s, 3 H), 2.23 (br s, 2 H), 3.58 (m, 2 H), 3.85 (d, J=10.90 Hz, 2 H), 3.94 (s, 1 H), 4.02 (d, J=1.77 Hz, 3 H), 6.67 (d, J=9.00 Hz, 1 H), 7.22 (m, 2 H), 7.41 (m, 1 H), 7.48 (m, 1 H), 7.55 (dd, J=8.68, 1.08 Hz, 1 H), 7.80 (dd, J=9.00, 2.66 Hz, 1 H), 7.85 (d, J=7.98 Hz, 1 H), 8.32 (d, J=2.53 Hz, 1 H).

### Example 2

### 1-(5-{3"-Fluoro-2",7-dimethyl-1H,2"H-[3,4"-biindazol]-1-yl}pyridin-2-yl)piperidine-4-carboxylic acid

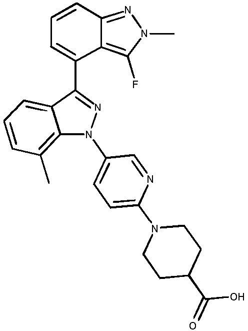

To a mixture of Intermediate 6 (40 mg, 0.11 mmol) and methyl piperidine carboxylate (46.7 mg, 0.32 mmol) in NMP (1.5 ml) was added DIPEA (55.1 mg, 0.43 mmol) and the mixture was heated at 140 °C for 16 h. The reaction was allowed to cool. Then NaOH (4M(aq), 0.5 ml) was added and the mixture was stirred for 2 h at RT. The reaction mixture was acidified with the addition of TFA, diluted with DMF and purified by preparative HPLC (acidic method) to give the title compound (39 mg).

1H NMR (400 MHz, DMSO-d6) δ ppm 1.60 (m, 2 H), 1.93 (br dd, J=13.24, 3.10 Hz, 2 H), 2.20 (s, 3 H), 2.58 (m, 1 H), 3.10 (m, 2 H), 4.03 (d, J=1.52 Hz, 3 H), 4.30 (br d, J=13.31 Hz, 2 H), 7.03 (d, J=9.12 Hz, 1 H), 7.22 (m, 2 H), 7.41 (m, 1 H), 7.49 (m, 1 H), 7.56 (d, J=8.62 Hz, 1 H), 7.79 (dd, J=9.00, 2.66 Hz, 1 H), 7.85 (d, J=7.98 Hz, 1 H), 8.34 (d, J=2.66 Hz, 1 H).

### Example 3

### (1R,5S,6R)-3-(5-{7"-Fluoro-2",7-dimethyl-1H,2"H-[3,4"-biindazol]-1-yl}pyridin-2-yl)-3-azabicyclo[3.1.0]hexane-6-carboxylic acid

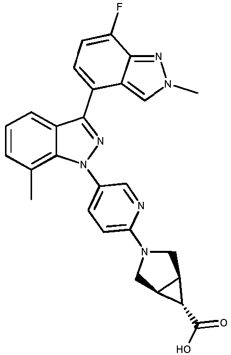

Using the method described for Example 2: Intermediate 8 (40 mg) with methyl exo-3-azabicyclo[3.1.0]hexane-6-carboxylate hydrochloride (49 mg) gave the title compound (15 mg).

1H NMR (400 MHz, DMSO-d6) δ ppm 1.49 (t, J=3.04 Hz, 1 H), 2.18 (s, 3 H), 2.24 (br s, 2 H), 3.59 (m, 2 H), 3.87 (d, J=11.03 Hz, 2 H), 4.09 (m, 1 H), 4.23 (s, 3 H), 6.69 (d, J=9.00 Hz, 1 H), 7.22 (m, 3 H), 7.71 (dd, J=7.79, 4.12 Hz, 1 H), 7.86 (dd, J=8.93, 2.60 Hz, 1 H), 8.06 (d, J=7.10 Hz, 1 H), 8.39 (d, J=2.53 Hz, 1 H), 8.72 (d, J=2.79 Hz, 1 H).

### Example 4

### 1-(5-{7"-Fluoro-2",7-dimethyl-1H,2"H-[3,4"-biindazol]-1-yl}pyridin-2-yl)piperidine-4-carboxylic acid

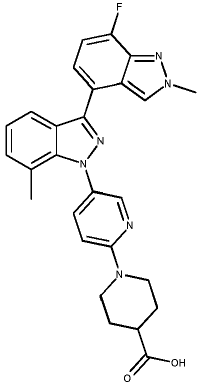

Using the method described for Example 2: Intermediate 8 (40 mg) with methyl piperidine carboxylate (47 mg) gave the title compound (41 mg).

1H NMR (400 MHz, DMSO-d6) δ ppm 1.62 (m, 2 H), 1.94 (br dd, J=13.24, 2.98 Hz, 2 H), 2.06 (m, 1 H), 2.19 (s, 3 H), 2.59 (m, 1 H), 3.11 (m, 2 H), 4.24 (s, 3 H), 4.25 (br s, 1 H), 4.32 (br d, J=13.31 Hz, 2 H), 7.05 (d, J=9.12 Hz, 1 H), 7.22 (m, 3 H), 7.71 (dd, J=7.79, 4.12 Hz, 1 H), 7.85 (dd, J=9.06, 2.72 Hz, 1 H), 8.06 (d, J=7.35 Hz, 1 H), 8.40 (d, J=2.66 Hz, 1 H), 8.74 (d, J=2.79 Hz, 1 H).

### Example 5

### (1R,5S,6S)-3-(5-{5"-Fluoro-2",7-dimethyl-1H,2"H-[3,4"-biindazol]-1-yl}pyridin-2-yl)-3-azabicyclo[3.1.0]hexane-6-carboxylic acid

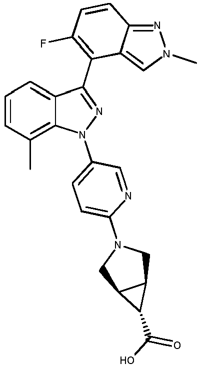

Using the method described for Example 2: Intermediate 11 (24 mg) with methyl exo-3-azabicyclo[3.1.0]hexane-6-carboxylate hydrochloride (29 mg) gave the title compound (16 mg).

1H NMR (400 MHz, DMSO-d6) δ ppm 1.50 (t, J=3.04 Hz, 1 H), 2.20 (s, 3 H), 2.24 (br s, 2 H), 3.60 (br d, J=10.52 Hz, 2 H), 3.87 (d, J=10.90 Hz, 2 H), 4.17 (s, 3 H), 6.70 (d, J=9.00 Hz, 1 H), 7.18 (m, 1 H), 7.25 (m, 1 H), 7.34 (dd, J=10.84, 9.31 Hz, 1 H), 7.67 (dd, J=8.05, 4.37 Hz, 1 H), 7.75 (dd, J=9.31, 4.12 Hz, 1 H), 7.91 (dd, J=9.00, 2.66 Hz, 1 H), 8.43 (d, J=4.18 Hz, 2 H), 8.42 (s, 1 H).

### Example 6

### 1-(5-{5"-Fluoro-2",7-dimethyl-1H,2"H-[3,4"-biindazol]-1-yl}pyridin-2-yl)piperidine-4-carboxylic acid

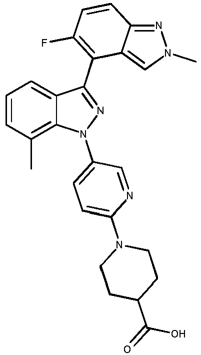

Using the method described for Example 2: Intermediate 11 (30 mg) with methyl piperidine carboxylate (35 mg) gave the title compound (22 mg).

1H NMR (400 MHz, DMSO-d6) δ ppm 1.61 (m, 2 H), 1.94 (br dd, J=13.24, 2.98 Hz, 2 H), 2.20 (m, 3 H), 2.58 (m, 1 H), 3.11 (m, 2 H), 4.10 (m, 1 H), 4.18 (m, 3 H), 4.31 (br d, J=13.18 Hz, 2 H), 7.05 (d, J=9.12 Hz, 1 H), 7.18 (m, 1 H), 7.25 (m, 1 H), 7.34 (dd, J=10.84, 9.31 Hz, 1 H), 7.67 (dd, J=7.92, 4.50 Hz, 1 H), 7.75 (m, 1 H), 7.88 (dd, J=9.13, 2.66 Hz, 1 H), 8.43 (m, 2 H).

### Example 7

### (1R,5S,6S)-3-(5-{5",7"-Difluoro-2",7-dimethyl-1H,2"H-[3,4"-biindazol]-1-yl}pyridin-2-yl)-3-zabicyclo[3.1.0]hexane-6-carboxylic acid

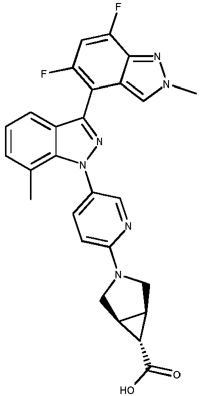

Using the method described for Example 2: Intermediate 13 (18 mg) with methyl exo-3-azabicyclo[3.1.0]hexane-6-carboxylate hydrochloride (21 mg) gave the title compound (7.5 mg).

1H NMR (400 MHz, DMSO-d6) δ ppm 1.45 (t, J=2.98 Hz, 1 H), 2.19 (m, 5 H), 3.55 (br d, J=10.52 Hz, 2 H), 3.85 (d, J=10.90 Hz, 2 H), 4.20 (s, 3 H), 6.61 (d, J=8.87 Hz, 1 H), 7.17 (m, 1 H), 7.24 (m, 1 H), 7.36 (t, J=11.03 Hz, 1 H), 7.65 (dd, J=8.05, 4.37 Hz, 1 H), 7.83 (dd, J=8.87, 2.66 Hz, 1 H), 8.39 (d, J=2.54 Hz, 1 H), 8.55 (d, J=2.79 Hz, 1 H).

### Example 8

### 1-[4-(5-{3"-Fluoro-2",7-dimethyl-1H,2"H-[3,4"-biindazol]-1-yl}pyridin-2-yl)piperazin-1-yl]ethan-1-one

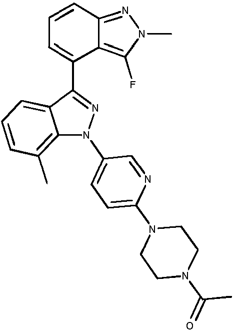

Using the method described for Example 2: Intermediate 6 (40 mg, 0.11 mmol) and 1-acetylpiperazine (41.4 mg, 0.32 mmol) gave the title compound (45 mg).

1H NMR (400 MHz, DMSO-d6) δ ppm 2.07 (s, 3 H), 2.20 (s, 3 H), 3.61 (s, 6 H), 3.70 (m, 2 H), 4.02 (m, 3 H), 7.03 (d, J=9.00 Hz, 1 H), 7.20 (m, 1 H), 7.25 (m, 1 H), 7.41 (m, 1 H), 7.49 (m, 1 H), 7.56 (d, J=8.74 Hz, 1 H), 7.84 (m, 2 H), 8.37 (d, J=2.66 Hz, 1 H).

### Example 9

### 4-(5-{3"-Fluoro-2",7-dimethyl-1H,2"H-[3,4"-biindazol]-1-yl}pyridin-2-yl)piperazine-1-carbaldehyde

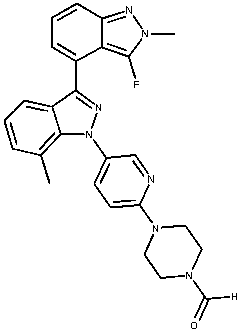

To a solution of Intermediate 14 (30 mg, 0.068 mmol) in THF (3 ml) was added N-formylsaccharin (28.3 mg, 0.136 mmol) and stirred at RT for 1 h. Another portion of N-formylsaccharin (28.3 mg, 0.136 mmol) was added and the mixture was stirred for 30 min at RT. The reaction mixture was diluted with a little water, acidified with the addition of TFA and purified by preparative HPLC (acidic method) to give the title compound (8 mg).

1H NMR (400 MHz, DMSO-d6) δ ppm 2.19 (s, 3 H), 3.53 (m, 4 H), 3.67 (m, 4 H), 4.02 (d, J=1.65 Hz, 3 H), 7.07 (d, J=9.00 Hz, 1 H), 7.20 (m, 1 H), 7.25 (m, 1 H), 7.41 (m, 1 H), 7.49 (m, 1 H), 7.56 (dd, J=8.74, 1.01 Hz, 1 H), 7.84 (m, 2 H), 8.13 (s, 1 H), 8.37 (d, J=2.66 Hz, 1 H)

### Example 10

### (1R,5S,6R)-3-(5-{3',5-Difluoro-2',7-dimethyl-1H,2'H-[3,4'-biindazol]-1-yl}pyridin-2-yl)-3-azabicyclo[3.1.0]hexane-6-carboxylic acid

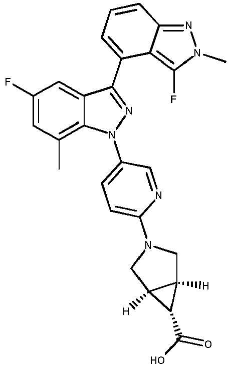

A mixture of intermediate 15 (62.6 mg, 0.18 mmol) and intermediate 17 (50 mg, 0.12 mmol), potassium carbonate (20.1 mg, 0.15 mmol), copper(I) iodide (3.5 mg, 0.02 mmol) and 2-methylquinolin-8-ol (5.8 mg, 0.04 mmol) in DMSO (1 ml) was heated at 100 °C for 16 h. The reaction was allowed to cool. Then NaOH (4M (aq), 0.5 ml) was added and stirred for 2 h at RT. The reaction mixture was acidified with the addition of TFA, diluted with DMF and purified by preparative HPLC (acidic method) to give the title compound (28 mg).

1H NMR (400 MHz, DMSO-d6) δ ppm 1.49 (t, J=3.04 Hz, 1 H), 2.19 (s, 3 H), 2.23 (br s, 2 H), 3.58 (m, 2 H), 3.85 (br d, J=10.90 Hz, 2 H), 4.02 (d, J=1.77 Hz, 3 H), 6.67 (d, J=9.00 Hz, 1 H), 7.22 (dt, J=9.76, 1.14 Hz, 1 H), 7.40 (m, 1 H), 7.47 (m, 1 H), 7.57 (m, 2 H), 7.81 (dd, J=8.93, 2.60 Hz, 1 H), 8.32 (d, J=2.53 Hz, 1 H)

### Example 11

### (1R,5S,6R)-3-(5-{3",6-Difluoro-2",7-dimethyl-1H,2"H-[3,4"-biindazol]-1-yl}pyridin-2-yl)-3-azabicyclo[3.1.0]hexane-6-carboxylic acid

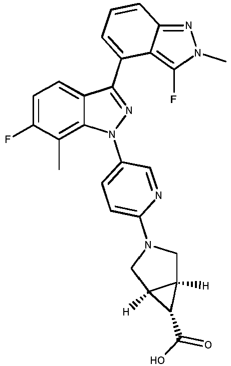

A mixture of intermediate 20 (70 mg, 0.14 mmol), 3-fluoro-2-methyl-4-(4,4,5,5-tetramethyl-1,3,2-dioxaborolan-2-yl)indazole (53 mg, 0.19 mmol), and sodium carbonate (45 mg, 0.43 mmol) in dioxane (2 ml) and water (0.5 ml) was degassed and a nitrogen atmosphere was maintained. To the mixture was added [1,1'-bis-(diphenylphosphino)-ferrocenyl]-dichloro-palladium(II) (PdCl₂dppf, 5.2 mg, 0.007 mmol) and the mixture was heated at 100 °C for 1.5 h. To the reaction was added DMF and then filtered. The filtrate was diluted with water and was purified by preparative HPLC (basic method) to give the title compound (56 mg).

1H NMR (400 MHz, DMSO-d6) δ ppm 1.47 (t, J=3.04 Hz, 1 H), 2.05 (d, J=1.52 Hz, 3 H), 2.21 (br s, 2 H), 3.56 (m, 2 H), 3.84 (d, J=10.90 Hz, 2 H), 4.02 (d, J=1.65 Hz, 3 H), 6.62 (d, J=9.00 Hz, 1 H), 7.15 (t, J=9.38 Hz, 1 H), 7.40 (m, 1 H), 7.47 (m, 1 H), 7.56 (dd, J=8.62, 1.01 Hz, 1 H), 7.76 (dd, J=8.87, 2.66 Hz, 1 H), 7.85 (dd, J=8.81, 5.01 Hz, 1 H), 8.31 (d, J=2.53 Hz, 1 H), 12.23 (br s, 1 H)

### Example 12

### (1R,5S,6R)-3-(5-{5,7"-Difluoro-2",7-dimethyl-1H,2"H-[3,4"-biindazol]-1-yl}pyridin-2-yl)-3-azabicyclo[3.1.0]hexane-6-carboxylic acid

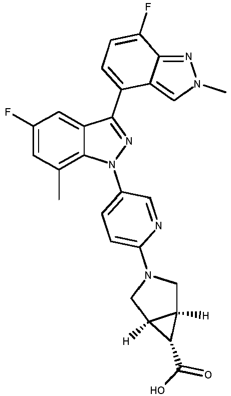

Using the method described for Example 11: Intermediate 25 (73 mg) and Intermediate 23 (81.9 mg) gave the title compound (45 mg).

1H NMR (400 MHz, DMSO-d6) δ ppm 1.48 (t, J=3.04 Hz, 1 H), 2.17 (s, 3 H), 2.24 (br s, 2 H), 3.59 (m, 2 H), 3.87 (d, J=10.90 Hz, 2 H), 4.23 (s, 3 H), 6.68 (d, J=9.00 Hz, 1 H), 7.16 (dd, J=11.47, 7.79 Hz, 2 H), 7.22 (dd, J=9.63, 1.27 Hz, 2 H), 7.68 (dd, J=7.86, 4.06 Hz, 2 H), 7.84 (m, 3 H), 8.39 (d, J=2.53 Hz, 1 H), 8.40 (m, 1 H), 8.71 (d, J=2.79 Hz, 1 H)

### Example 13

### (1R,5S,6R)-3-(5-{3",7"-Difluoro-2",7-dimethyl-1H,2"H-[3,4"-biindazol]-1-yl}pyridin-2-yl)-3-azabicyclo[3.1.0]hexane-6-carboxylic acid

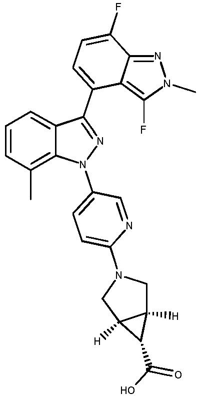

Using the method described for Example 11: Intermediate 4 (30 mg) and Intermediate 27 (18 mg) gave the title compound (15 mg).

HPLC (Acidic): Rt = 0.907 min ((M+H)+ 501.2)

### Example 14

### (1R,5S,6R)-3-(5-{5,5"-Difluoro-2",7-dimethyl-1H,2"H-[3,4"-biindazol]-1-yl}pyridin-2-yl)-3-azabicyclo[3.1.0]hexane-6-carboxylic acid

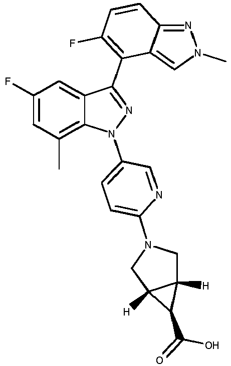

Using the method described for Example 10: Intermediate 15 (12 mg) and Intermediate 28 (7 mg) gave the title compound (5 mg).

HPLC (Acidic): Rt = 0.941 min ((M+H)+ 501.0)

### Example 15

### (1R,5S,6R)-3-(5-{5,5",7"-Trifluoro-2",7-dimethyl-1H,2"H-[3,4"-biindazol]-1-yl}pyridin-2-yl)-3-azabicyclo[3.1.0]hexane-6-carboxylic acid

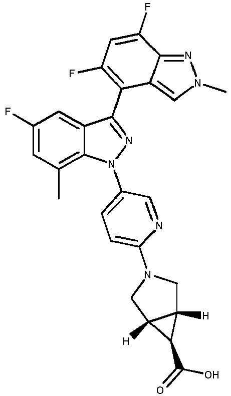

Using the method described for Example 10: Intermediate 15 (8.1 mg) and Intermediate 29 (5 mg) gave the title compound (5 mg).

HPLC (Acidic): Rt = 0.975 min ((M+H)+ 519.0)

The following examples were synthesized according to the methods described above for Example 1 to 15.

**Table 1**

| Example | Structure | Name |
|---|---|---|
| | | Analytics |
| Example 16 | 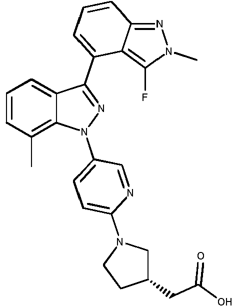 | 2-[(3S)-1-(5-{3"-fluoro-2",7-dimethyl-1H,2"H-[3,4"-biindazol]-1-yl}pyridin-2-yl)pyrrolidin-3-yl]acetic acid |
| | | HPLC (Acidic): Rt = 0.864 min ((M+H)+ 485) |
| | From Intermediate 6 | |
| Example 17 | 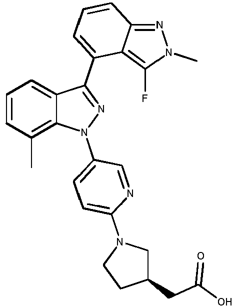 From Intermediate 6 | 2-[(3R)-1-(5-{3"-fluoro-2",7-dimethyl-1H,2"H-[3,4"-biindazol]-1-yl}pyridin-2-yl)pyrrolidin-3-yl]acetic acid |
| | | 1H NMR (400 MHz, DMSO-d6) δ ppm 1.75 (dq, J=12.32, 8.44 Hz, 1 H), 2.22 (s, 3 H), 2.47 (d, J=7.22 Hz, 2 H), 2.66 (m, 1 H), 3.18 (m, 1 H), 3.49 (dt, J=10.52, 7.79 Hz, 1 H), 3.65 (m, 1 H), 3.80 (m, 1 H), 4.02 (d, J=1.77 Hz, 3 H), 6.76 (d, J=9.13 Hz, 1 H), 7.21 (d, J=7.98 Hz, 1 H), 7.26 (m, 1 H), 7.41 (m, 1 H), 7.49 (m, 1 H), 7.56 (dd, J=8.68, 1.08 Hz, 1 H), 7.86 (m, 2 H), 8.34 (d, J=2.53 Hz, 1 H) |
| Example 18 | 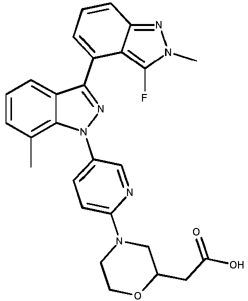 From Intermediate 6 | 2-[4-(5-{3"-fluoro-2",7-dimethyl-1H,2"H-[3,4"-biindazol]-1-yl}pyridin-2-yl)morpholin-2-yl]acetic acid (Mixture of stereoisomers) |
| | | HPLC (Acidic): Rt = 0.952 min ((M+H)+ 515.2) |
| Example 19 | 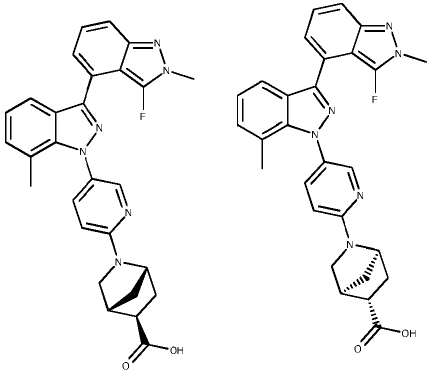 From Intermediate 6 | 2-(5-{3"-fluoro-2",7-dimethyl-1H,2"H-[3,4"-biindazol]-1-yl}pyridin-2-yl)-2-azabicyclo[2.2.1]heptane-5-carboxylic acid (Mixture of stereoisomers) |
| | | 1H NMR (400 MHz, DMSO-d6) δ ppm 1.67 (m, 1 H), 1.75 (m, 1 H), 1.97 (m, 2 H), 2.23 (s, 3 H), 2.65 (m, 1 H), 2.92 (br s, 1 H), 3.23 (br d, J=9.51 Hz, 1 H), 3.51 (dd, J=9.76, 3.42 Hz, 1 H), 3.88 (m, 1 H), 4.03 (d, J=1.77 Hz, 3 H), 4.68 (br s, 1 H), 6.77 (br d, J=7.98 Hz, 1 H), 7.22 (m, 1 H), 7.26 (m, 1 H), 7.41 (m, 1 H), 7.49 (m, 1 H), 7.56 (dd, J=8.68, 1.08 Hz, 1 H), 7.84 (m, 2 H), 8.32 (d, J=2.53 Hz, 1 H) |
| Example 20 | 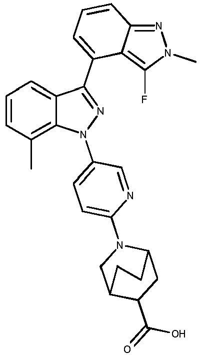 | 2-(5-{3'-fluoro-2',7-dimethyl-1H,2'H-[3,4'-biindazol]-1-yl}pyridin-2-yl)-2-azabicyclo[2.2.2]octane-5-carboxylic acid |
| Example 20-1 | Enantiomer 1: Suzuki reaction of Intermediate 35 with Intermediate 2 followed by a hydrolysis step | HPLC (Acidic): |
| | | Rt = 0.880 min ((M+H)+ 511.0) |
| Example 20-2 | Enantiomer 2: Suzuki reaction of Intermediate 36 with Intermediate 2 followed by a hydrolysis step | HPLC (Acidic): |
| | | Rt = 0.881 min ((M+H)+ 511.0) |
| Example 20-3 | Enantiomer 3: Suzuki reaction of Intermediate 37 with Intermediate 2 followed by a hydrolysis step | HPLC (Acidic): |
| | | Rt = 0.882 min ((M+H)+ 511.0) |
| Example 20-4 | Enantiomer 4: Suzuki reaction of Intermediate 38 with Intermediate 2 followed by a hydrolysis step | HPLC (Acidic): |
| | | Rt = 0.882 min ((M+H)+ 511.0) |
| Example 21 | 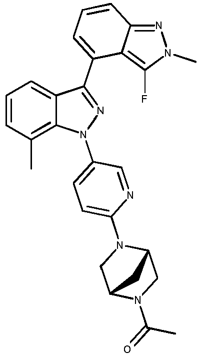 From Intermediate 41 | 1-[(1S,4S)-5-(5-{3"-fluoro-2",7-dimethyl-1H,2"H-[3,4"-biindazol]-1-yl}pyridin-2-yl)-2,5-diazabicyclo[2.2.1]heptan-2-yl]ethan-1-one |
| | | Suzuki reaction of Intermediate 41 with Intermediate 2 followed by a hydrolysis step |
| | | ¹H NMR (400 MHz, DMSO-d₆) δ ppm 1.86 - 2.08 (m, 5 H), 2.21 (d, *J*=5.32 Hz, 3 H), 3.53 (m, 4 H), 4.02 (m, 3 H), 4.79 (br d, *J*=46.64 Hz, 1 H), 5.00 (br d, *J*=38.40 Hz, 1 H), 6.79 (br dd, *J*=15.27, 9.06 Hz, 1 H), 7.23 (m, 2 H), 7.31 (d, *J*=5.83 Hz, 1 H), 7.41 (m, 1 H), 7.49 (m, 1 H), 7.56 (d, *J*=8.62 Hz, 1 H), 7.84 (m, 2 H), 8.34 (dd, *J*=4.37, 2.60 Hz, 1 H) |
| Example 22 | 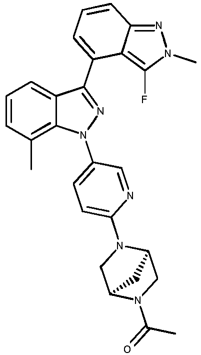 | 1-[(1R,4R)-5-(5-{3"-fluoro-2",7-dimethyl-1H,2"H-[3,4"-biindazol]-1-yl}pyridin-2-yl)-2,5-diazabicyclo[2.2.1]heptan-2-yl]ethan-1-one |
| | | Suzuki reaction of Intermediate 44 with Intermediate 2 followed by a hydrolysis step |
| | From Intermediate 44 | ¹H NMR (400 MHz, DMSO-*d*₆) δ ppm 1.86 - 2.08 (m, 5 H), 2.21 (d, *J*=5.20 Hz, 3 H), 3.54 (m, 4 H), 4.02 (d, J=1.39 Hz, 3 H), 4.79 (br d, *J*=46.64 Hz, 1 H), 4.99 (m, 1 H), 6.79 (br dd, *J*=14.89, 8.93 Hz, 1 H), 7.23 (m, 2 H), 7.46 (m, 3 H), 7.84 (m, 2 H), 8.34 (dd, *J*=4.31, 2.66 Hz, 1 H) |
| Example 23 | 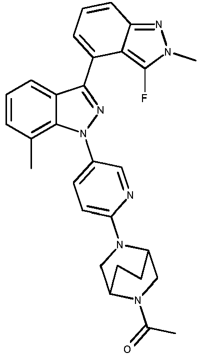 From Intermediate 6 | 1-[5-(5-{3"-fluoro-2",7-dimethyl-1H,2"H-[3,4"-biindazol]-1-yl}pyridin-2-yl)-2,5-diazabicyclo[2.2.2]octan-2-yl]ethan-1-one |
| | | HPLC (Acidic): |
| | | Rt = 0.912 min ((M+H)+ 510.2) |
| Example 24 | 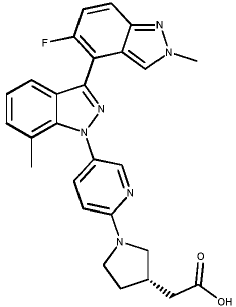 From Intermediate 11 | 2-[(3S)-1-(5-{5"-fluoro-2",7-dimethyl-1H,2"H-[3,4"-biindazol]-1-yl}pyridin-2-yl)pyrrolidin-3-yl]acetic acid |
| | | HPLC (Acidic): |
| | | Rt = 0.863 min ((M+H)+ 485.2) |
| Example 25 | 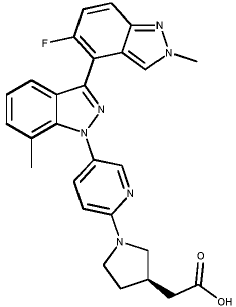 From Intermediate 11 | 2-[(3R)-1-(5-{5"-fluoro-2",7-dimethyl-1H,2"H-[3,4"-biindazol]-1-yl}pyridin-2-yl)pyrrolidin-3-yl]acetic acid |
| | | HPLC (Acidic): |
| | | Rt = 0.863 min ((M+H)+ 485.2) |
| Example 26 | 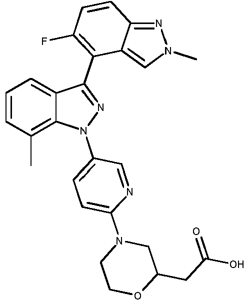 | |
| Example 26-1 | R-Isomer: | 2-[(2R)-4-(5-{5"-fluoro-2",7-dimethyl-1H,2"H-[3,4"-biindazol]-1-yl}pyridin-2-yl)morpholin-2-yl]acetic acid |
| | From Intermediate 11 | |
| | | HPLC (Acidic): |
| | | Rt = 0.996 min ((M+H)+ 501.2) |
| Example 26-2 | S-Isomer: | 2-[(2S)-4-(5-{5"-fluoro-2",7-dimethyl-1H,2"H-[3,4"-biindazol]-1-yl}pyridin-2-yl)morpholin-2-yl]acetic acid |
| | From Intermediate 11 | |
| | | HPLC (Acidic): |
| | | Rt = 0.996 min ((M+H)+ 501.2) |
| Example 27 | 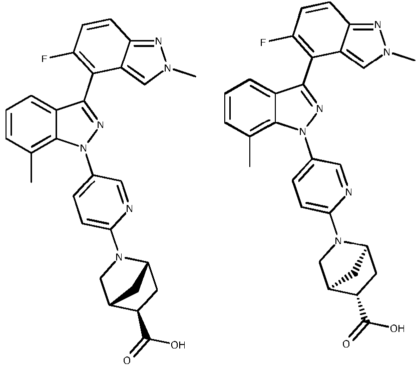 | (1S,4R,5R)-2-(5-{5"-fluoro-2",7-dimethyl-1H,2"H-[3,4"-biindazol]-1-yl}pyridin-2-yl)-2-azabicyclo[2.2.1]heptane-5-carboxylic acid |
| | | (1R,4S,5S)-2-(5-{5"-fluoro-2",7-dimethyl-1H,2"H-[3,4"-biindazol]-1-yl}pyridin-2-yl)-2-azabicyclo[2.2.1]heptane-5-carboxylic acid |
| Example 27-1 | Suzuki reaction of Intermediate 46 with Intermediate 45 followed by a hydrolysis step | ¹H NMR (400 MHz, DMSO-d₆) δ ppm 1.71 (m, 2 H), 1.98 (m, 2 H), 2.23 (s, 3 H), 2.65 (m, 1 H), 2.92 (br s, 1 H), 3.24 (br d, *J*=9.63 Hz, 1 H), 3.52 (dd, *J*=9.76, 3.42 Hz, 1 H), 4.18 (s, 3 H), 4.69 (br s, 1 H), 6.76 (br d, *J*=7.86 Hz, 1 H), 7.19 (dd, *J*=7.98, 7.10 Hz, 1 H), 7.25 (m, 1 H), 7.34 (dd, *J*=10.90, 9.25 Hz, 1 H), 7.67 (dd, *J*=8.05, 4.37 Hz, 1 H), 7.76 (ddd, *J*=9.25, 4.31, 0.76 Hz, 1 H), 7.92 (dd, *J*=9.00, 2.28 Hz, 1 H), 8.42 (m, 2 H) |
| Example 27-2 | Suzuki reaction of Intermediate 47 with Intermediate 45 followed by a hydrolysis step | ¹H NMR (400 MHz, DMSO-d₆) δ ppm 1.71 (m, 2 H), 1.98 (m, 2 H), 2.07 (s, 1 H), 2.23 (s, 3 H), 2.33 (m, 1 H), 2.65 (m, 1 H), 2.93 (br s, 1 H), 3.24 (br d, *J*=9.63 Hz, 1 H), 3.52 (dd, *J*=9.76, 3.42 Hz, 1 H), 4.17 (s, 4 H), 4.70 (br s, 1 H), 6.78 (br d, *J*=6.72 Hz, 1 H), 7.19 (dd, *J*=7.98, 7.10 Hz, 1 H), 7.26 (m, 1 H), 7.34(dd, *J*=10.90, 9.25 Hz, 1 H), 7.67 (dd, *J*=8.05, 4.37 Hz, 1 H), 7.76 (ddd, *J*=9.25, 4.31, 0.76 Hz, 1 H), 7.93 (dd, *J*=9.00, 2.15 Hz, 1 H), 8.42 (d, *J*=4.06 Hz, 2 H), 8.42 (s, 2 H) |
| Example 28 | 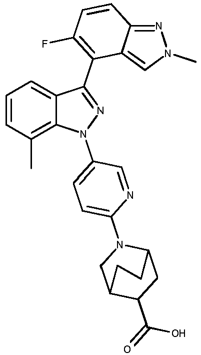 | (1R,4S,5R)-2-(5-{5"-fluoro-2",7-dimethyl-1H,2"H-[3,4"-biindazol]-1-yl}pyridin-2-yl)-2-azabicyclo-[2.2.2]octane-5-carboxylic acid |
| | | (15,4R,55)-2-(5-{5"-fluoro-2",7-dimethyl-1H,2"H-[3,4"-biindazol]-1-yl}pyridin-2-yl)-2-azabicyclo-[2.2.2]octane-5-carboxylic acid |
| | | (1R,4S,5S)-2-(5-{5"-fluoro-2",7-dimethyl-1H,2"H-[3,4"-biindazol]-1-yl}pyridin-2-yl)-2-azabicyclo-[2.2.2]octane-5-carboxylic acid |
| | | (1S,4R,5R)-2-(5-{5"-fluoro-2",7-dimethyl-1H,2"H-[3,4"-biindazol]-1-yl}pyridin-2-yl)-2-azabicyclo[2.2.2]octane-5-carboxylic acid |
| Example 28-1 | Enantiomer 1: Suzuki reaction of Intermediate 35 with Intermediate 45 followed by a hydrolysis step | HPLC (Acidic): |
| | | Rt = 0.892 min ((M+H)+ 511.2) |
| Example 28-2 | Enantiomer 2: Suzuki reaction of Intermediate 36 with Intermediate 45 followed by a hydrolysis step | HPLC (Acidic): |
| | | Rt = 0.892 min ((M+H)+ 511.2) |
| Example 28-3 | Enantiomer 3: Suzuki reaction of Intermediate 37 with Intermediate 45 followed by a hydrolysis step | HPLC (Acidic): |
| | | Rt = 0.895 min ((M+H)+ 511.2) |
| Example 28-4 | Enantiomer 4: Suzuki reaction of Intermediate 38 with Intermediate 45 followed by a hydrolysis step | HPLC (Acidic): |
| | | Rt = 0.893 min ((M+H)+ 511.2) |
| Example 29 | 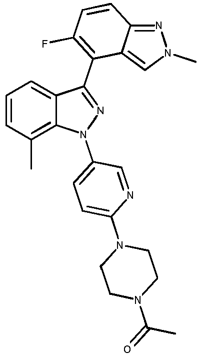 From Intermediate 11 | 1-[4-(5-{5"-fluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 0.976 min ((M+H)+ 484.2) |
| Example 30 | 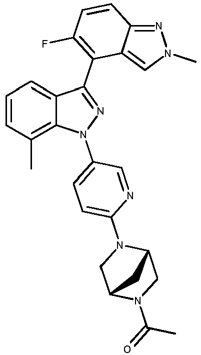 From Intermediate 41 | 1-[(1S,4S)-5-(5-{5"-fluoro-2",7-dimethyl-1H,2"H-[3,4"-biindazol]-1-yl}pyridin-2-yl)-2,5-diazabicyclo[2.2.1]heptan-2-yl]ethan-1-one |
| | | HPLC (Acidic): |
| | | Rt = 0.887 min ((M+H)+ 496.2) |
| Example 31 | 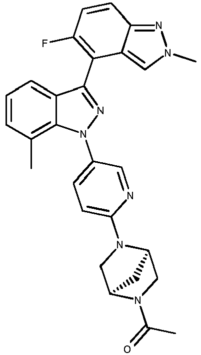 From Intermediate 44 | 1-[(1R,4R)-5-(5-{5"-fluoro-2",7-dimethyl-1H,2"H-[3,4"-biindazol]-1-yl}pyridin-2-yl)-2,5-diazabicyclo[2.2.1]heptan-2-yl]ethan-1-one |
| | | HPLC (Acidic): |
| | | Rt = 0.890 min ((M+H)+ 496.2) |
| Example 32 | 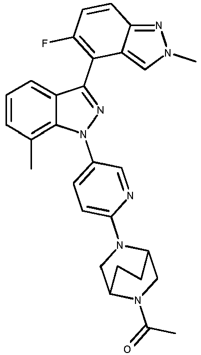 From Intermediate 11 | 1-[5-(5-{5"-fluoro-2",7-dimethyl-1H,2"H-[3,4"-biindazol]-1-yl}pyridin-2-yl)-2,5-diazabicyclo[2.2.2]octan-2-yl]ethan-1-one |
| | | HPLC (Acidic): |
| | | Rt = 0.926 min ((M+H)+ 510.2) |
| Example 33 | 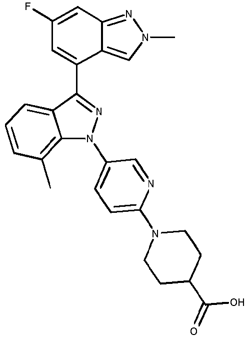 From Intermediate 48 | 1-(5-{6"-fluoro-2",7-dimethyl-1H,2"H-[3,4"-biindazol]-1-yl}pyridin-2-yl)piperidine-4-carboxylic acid |
| | | HPLC (Acidic): |
| | | Rt = 0.961 min ((M+H)+ 485.2) |
| Example 34 | 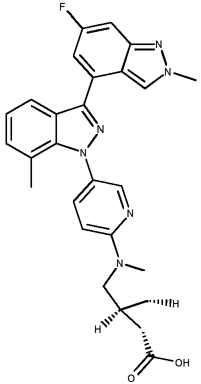 From Intermediate 4 | (1R,5S,6R)-3-(5-{6"-fluoro-2",7-dimethyl-1H,2"H-[3,4"-biindazol]-1-yl}pyridin-2-yl)-3-azabicyclo[3.1.0]hexane-6-carboxylic acid |
| | | HPLC (Acidic): |
| | | Rt = 0.926 min ((M+H)+ 483.2) |
| Example 35 | 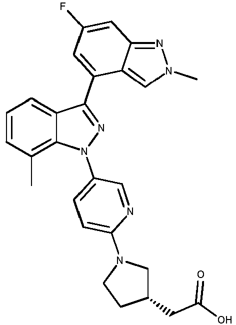 From Intermediate 48 | 2-[(3S)-1-(5-{6"-fluoro-2",7-dimethyl-1H,2"H-[3,4"-biindazol]-1-yl}pyridin-2-yl)pyrrolidin-3-yl]acetic acid |
| | | HPLC (Acidic): |
| | | Rt = 0.880 min ((M+H)+ 485.2) |
| Example 36 | 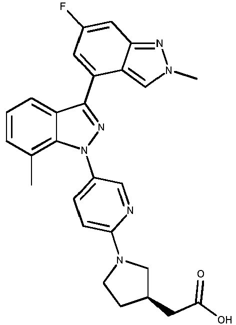 From Intermediate 48 | 2-[(3R)-1-(5-{6"-fluoro-2",7-dimethyl-1H,2"H-[3,4"-biindazol]-1-yl}pyridin-2-yl)pyrrolidin-3-yl]acetic acid |
| | | HPLC (Acidic): |
| | | Rt = 0.880 min ((M+H)+ 485.2) |
| Example 37 | 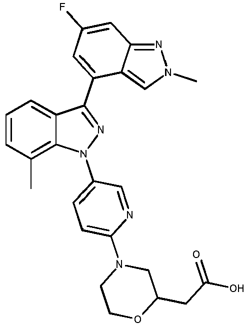 | |
| Example 37-1 | R-Isomer: | 2-[(2R)-4-(5-{6"-fluoro-2",7-dimethyl-1H,2"H-[3,4"-biindazol]-1-yl}pyridin-2-yl)morpholin-2-yl]acetic acid |
| | From Intermediate 48 | |
| | | HPLC (Acidic): |
| | | Rt = 1.032 min ((M+H)+ 501.2) |
| Example 37-2 | S-Isomer: | 2-[(2S)-4-(5-{6"-fluoro-2",7-dimethyl-1H,2"H-[3,4"-biindazol]-1-yl}pyridin-2-yl)morpholin-2-yl]acetic acid |
| | From Intermediate 48 | |
| | | HPLC (Acidic): |
| | | Rt = 1.031 min ((M+H)+ 501.2) |
| Example 38 | 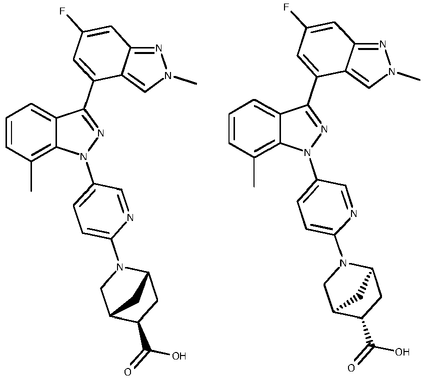 | (1S,4R,5R)-2-(5-{6"-fluoro-2",7-dimethyl-1H,2"H-[3,4"-biindazol]-1-yl}pyridin-2-yl)-2-azabicyclo[2.2.1]heptane-5-carboxylic acid |
| | | (1R,4S,5S)-2-(5-{6"-fluoro-2",7-dimethyl-1H,2"H-[3,4"-biindazol]-1-yl}pyridin-2-yl)-2-azabicyclo[2.2.1]heptane-5-carboxylic acid |
| Example 38-1 | Enantiomer 1: Suzuki reaction of Intermediate 31 with Intermediate 46 followed by a hydrolysis step | HPLC (Acidic): |
| | | Rt = 0.886 min ((M+H)+ 497.0) |
| Example 38-2 | Enantiomer 2: Suzuki reaction of Intermediate 31 with Intermediate 47 followed by a hydrolysis step | HPLC (Acidic): |
| | | Rt = 0.886 min ((M+H)+ 497.0) |
| Example 39 | 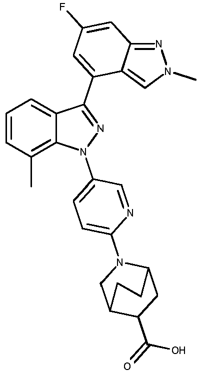 | 2-(5-{6"-fluoro-2",7-dimethyl-1H,2"H-[3,4"-biindazol]-1-yl}pyridin-2-yl)-2-azabicyclo[2.2.2]octane-5-carboxylic acid |
| | | HPLC (Acidic): |
| | | Rt = 0.911 min ((M+H)+ 511.0) |
| Example 40 | 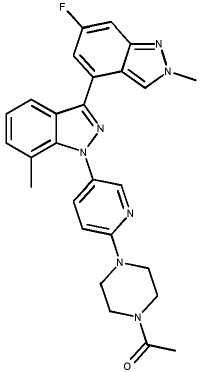 From Intermediate 4 | 1-[4-(5-{6"-fluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 1.012 min ((M+H)+ 484.2) |
| Example 41 | 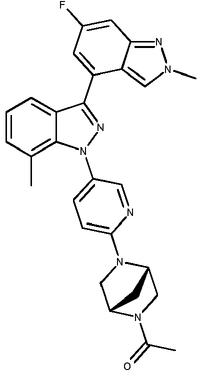 From Intermediate 48 | 1-[(1S,4S)-5-(5-{6"-fluoro-2",7-dimethyl-1H,2"H-[3,4"-biindazol]-1-yl}pyridin-2-yl)-2,5-diazabicyclo[2.2.1]heptan-2-yl]ethan-1-one |
| | | HPLC (Acidic): |
| | | Rt = 0.914 min ((M+H)+ 496.2) |
| Example 42 | 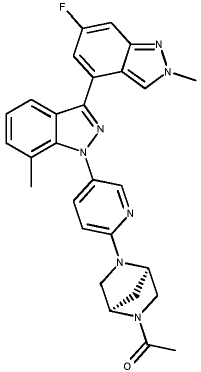 From Intermediate 48 | 1-[(1R,4R)-5-(5-{6"-fluoro-2",7-dimethyl-1H,2"H-[3,4"-biindazol]-1-yl}pyridin-2-yl)-2,5-diazabicyclo[2.2.1]heptan-2-yl]ethan-1-one |
| | | HPLC (Acidic): |
| | | Rt = 0.917 min ((M+H)+ 496.2) |
| Example 43 | 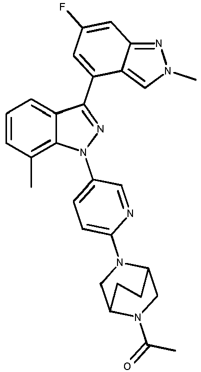 From Intermediate 4 and acetylation step | 1-[5-(5-{6"-fluoro-2",7-dimethyl-1H,2"H-[3,4"-biindazol]-1-yl}pyridin-2-yl)-2,5-diazabicyclo[2.2.2]octan-2-yl]ethan-1-one |
| | | HPLC (Acidic): |
| | | Rt = 0.959 min ((M+H)+ 510.2) |
| Example 44 | 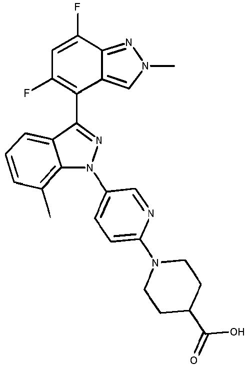 From Intermediate 13 | 1-(5-{5",7"-difluoro-2",7-dimethyl-1H,2"H-[3,4"-biindazol]-1-yl}pyridin-2-yl)piperidine-4-carboxylic acid |
| | | HPLC (Acidic): |
| | | Rt = 0.962 min ((M+H)+ 503.2) |
| Example 45 | 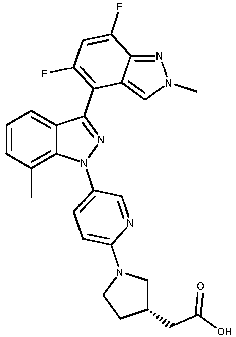 From Intermediate 13 | 2-[(3S)-1-(5-{5",7"-difluoro-2",7-dimethyl-1H,2"H-[3,4"-biindazol]-1-yl}pyridin-2-yl)pyrrolidin-3-yl]acetic acid |
| | | HPLC (Acidic): |
| | | Rt = 0.883 min ((M+H)+ 503.2 |
| Example 46 | 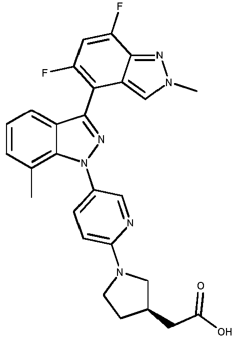 From Intermediate 13 | 2-[(3R)-1-(5-{5",7"-difluoro-2",7-dimethyl-1H,2"H-[3,4"-biindazol]-1-yl}pyridin-2-yl)pyrrolidin-3-yl]acetic acid |
| | | HPLC (Acidic): |
| | | Rt = 0.883 min ((M+H)+ 503.2 |
| Example 47 | 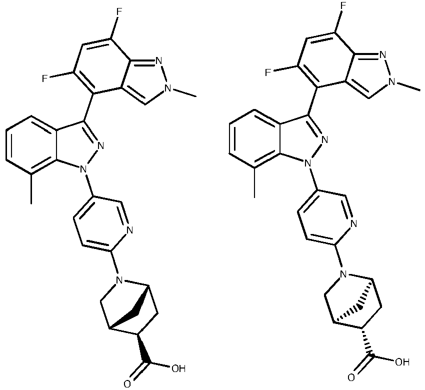 | (1S,4R,5R)-2-(5-{5",7"-difluoro-2",7-dimethyl-1H,2"H-[3,4"-biindazol]-1-yl}pyridin-2-yl)-2-azabicyclo[2.2.1]heptane-5-carboxylic acid |
| | | (1R,4S,5S)-2-(5-{5",7"-difluoro-2",7-dimethyl-1H,2"H-[3,4"-biindazol]-1-yl}pyridin-2-yl)-2-azabicyclo[2.2.1]heptane-5-carboxylic acid |
| Example 47-1 | Enantiomer 1: Method described for Example 2 with Intermediate 13 and Intermediate 53 | HPLC (Acidic): |
| | | Rt = 0.904 min ((M+H)+ 515.2) |
| Example 47-2 | Enantiomer 2: Method described for Example 2 with Intermediate 13 and Intermediate 54 | HPLC (Acidic): |
| | | Rt = 0.904 min ((M+H)+ 515.2) |
| Example 48 | 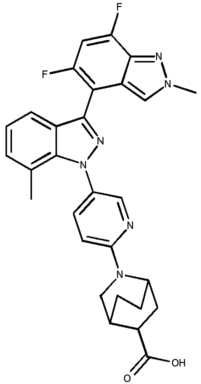 | (1R,4S,5R)-2-(5-{5",7"-difluoro-2",7-dimethyl-1H,2"H-[3,4"-biindazol]-1-yl}pyridin-2-yl)-2-azabicyclo[2.2.2]octane-5-carboxylic acid |
| | | (1S,4R,5S)-2-(5-{5",7"-difluoro-2",7-dimethyl-1H,2"H-[3,4"-biindazol]-1-yl}pyridin-2-yl)-2-azabicyclo[2.2.2]octane-5-carboxylic acid |
| | | (1R,4S,5S)-2-(5-{5",7"-difluoro-2",7-dimethyl-1H,2"H-[3,4"-biindazol]-1-yl}pyridin-2-yl)-2-azabicyclo[2.2.2]octane-5-carboxylic acid |
| | | (1S,4R,5R)-2-(5-{5",7"-difluoro-2",7-dimethyl-1H,2"H-[3,4"-biindazol]-1-yl}pyridin-2-yl)-2-azabicyclo[2.2.2]octane-5-carboxylic acid |
| Example 48-1 | Enantiomer 1: Suzuki reaction of Intermediate 35 with Intermediate 60 followed by a hydrolysis step | HPLC (Acidic): |
| | | Rt = 0.917 min ((M+H)+ 529.2) |
| Example 48-2 | Enantiomer 2: Suzuki reaction of Intermediate 36 with Intermediate 60 followed by a hydrolysis step | HPLC (Acidic): |
| | | Rt = 0.916 min ((M+H)+ 529.2) |
| Example 48-3 | Enantiomer 3: Suzuki reaction of Intermediate 37 with Intermediate 60 followed by a hydrolysis step | HPLC (Acidic): |
| | | Rt = 0.920 min ((M+H)+ 529.2) |
| Example 48-4 | Enantiomer 4: Suzuki reaction of Intermediate 38 with Intermediate 60 followed by a hydrolysis step | HPLC (Acidic): |
| | | Rt = 0.920 min ((M+H)+ 529.2) |
| Example 49 | 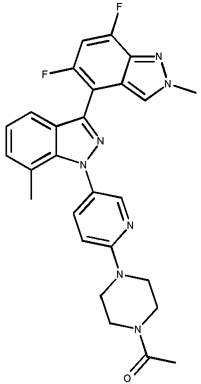 From Intermediate 13 | 1-[4-(5-{5",7"-difluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 1.007 min ((M+H)+ 502.2) |
| Example 50 | 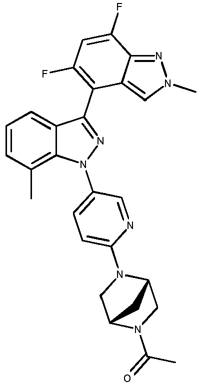 Acylation of Intermediate 62 | 1-[(1S,4S)-5-(5-{5",7"-difluoro-2",7-dimethyl-1H,2"H-[3,4"-biindazol]-1-yl}pyridin-2-yl)-2,5-diazabicyclo[2.2.1]heptan-2-yl]ethan-1-one |
| | | HPLC (Acidic): |
| | | Rt = 0.919 min ((M+H)+ 514.2) |
| Example 51 | 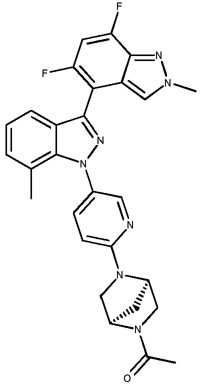 Acylation of Intermediate 64 | 1-[(1R,4R)-5-(5-{5",7"-difluoro-2",7-dimethyl-1H,2"H-[3,4"-biindazol]-1-yl}pyridin-2-yl)-2,5-diazabicyclo[2.2.1]heptan-2-yl]ethan-1-one |
| | | HPLC (Acidic): |
| | | Rt = 0.913 min ((M+H)+ 514.2) |
| Example 52 | 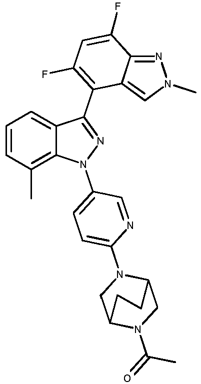 Acylation of Intermediate 65 | 1-[5-(5-{5",7"-difluoro-2",7-dimethyl-1H,2"H-[3,4"-biindazol]-1-yl}pyridin-2-yl)-2,5-diazabicyclo[2.2.2]octan-2-yl]ethan-1-one |
| | | HPLC (Acidic): |
| | | Rt = 0.959 min ((M+H)+ 528.2) |
| Example 53 | 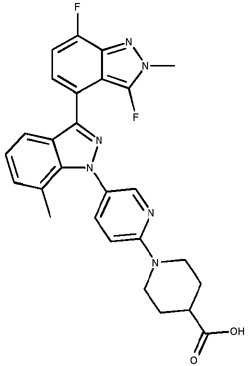 From Intermediate 66 | 1-(5-{3",7"-difluoro-2",7-dimethyl-1H,2"H-[3,4"-biindazol]-1-yl}pyridin-2-yl)piperidine-4-carboxylic acid |
| | | ¹H NMR (400 MHz, DMSO-d₆) δ ppm 1.61 (m, 2 H), 1.93 (br dd, *J*=13.24, 3.23 Hz, 2 H), 2.20 (s, 3 H), 2.58 (m, 1 H), 3.10 (m, 2 H), 4.06 (d, *J*=1.77 Hz, 3 H), 4.29 (m, 2 H), 7.04 (d, *J*=9.13 Hz, 1 H), 7.22 (m, 3 H), 7.43 (dd, *J*=7.67, 4.12 Hz, 1 H), 7.79 (dd, *J*=9.06, 2.72 Hz, 1 H), 7.83 (d, *J*=7.98 Hz, 1 H), 8.33 (d, *J*=2.66 Hz, 1 H) |
| Example 54 | 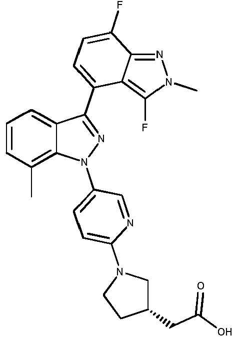 From Intermediate 66 | 2-[(3S)-1-(5-{3",7"-difluoro-2",7-dimethyl-1H,2"H-[3,4"-biindazol]-1-yl}pyridin-2-yl)pyrrolidin-3-yl]acetic acid |
| | | HPLC (Acidic): |
| | | Rt = 0.863 min ((M+H)+ 503.2) |
| Example 55 | 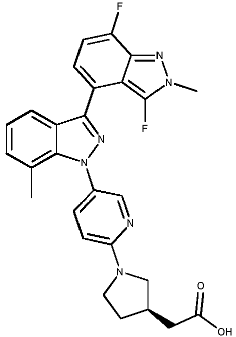 From Intermediate 66 | 2-[(3R)-1-(5-{3",7"-difluoro-2",7-dimethyl-1H,2"H-[3,4"-biindazol]-1-yl}pyridin-2-yl)pyrrolidin-3-yl]acetic acid |
| | | HPLC (Acidic): |
| | | Rt = 0.864 min ((M+H)+ 503.2) |
| Example 56 | 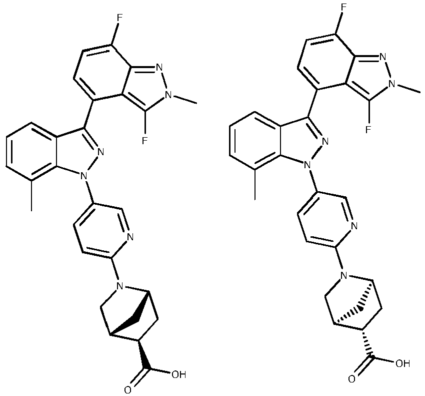 | (1S,4R,5R)-2-(5-{3",7"-difluoro-2",7-dimethyl-1H,2"H-[3,4"-biindazol]-1-yl}pyridin-2-yl)-2-azabicyclo[2.2.1]heptane-5-carboxylic acid |
| | | (1R,45,55)-2-(5-{3",7"-difluoro-2",7-dimethyl-1H,2"H-[3,4"-biindazol]-1-yl}pyridin-2-yl)-2-azabicyclo[2.2.1]heptane-5-carboxylic acid |
| Example 56-1 | Enantiomer 1: Suzuki reaction of Intermediate 27 with Intermediate 46 followed by a hydrolysis step | HPLC (Acidic): |
| | | Rt = 0.874 min ((M+H)+ 515.0) |
| Example 56-2 | Enantiomer 2: Suzuki reaction of Intermediate 27 with Intermediate 47 followed by a hydrolysis step | HPLC (Acidic): |
| | | Rt = 0.875 min ((M+H)+ 515.0) |
| Example 57 | 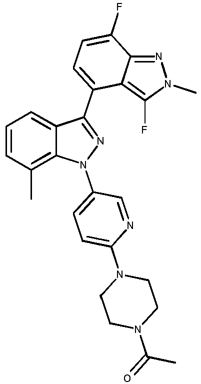 From Intermediate 66 | 1-[4-(5-{3",7"-difluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 0.988 min ((M+H)+ 502.2) |
| Example 58 | 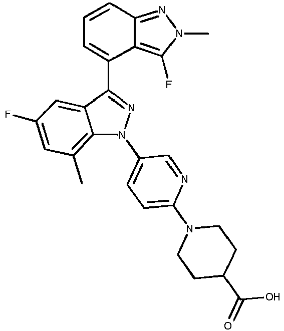 From Intermediate 68 | 1-(5-{3",5-difluoro-2",7-dimethyl-1H,2"H-[3,4"-biindazol]-1-yl}pyridin-2-yl)piperidine-4-carboxylic acid |
| | | HPLC (Acidic): |
| | | Rt = 0.950 min ((M+H)+ 503.2) |
| Example 59 | 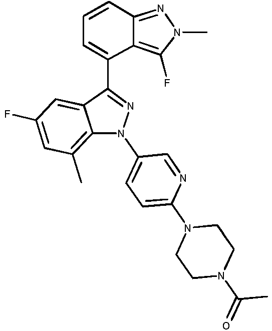 From Intermediate 68 | 1-[4-(5-{3",5-difluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 0.992 min ((M+H)+ 502.0) |
| Example 60 | 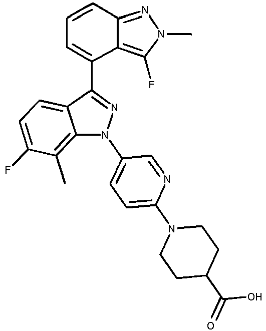 From Intermediate 70 | 1-(5-{3",6-difluoro-2",7-dimethyl-1H,2"H-[3,4"-biindazol]-1-yl}pyridin-2-yl)piperidine-4-carboxylic acid |
| | | HPLC (Acidic): |
| | | Rt = 0.954 min ((M+H)+ 503.2) |
| Example 61 | 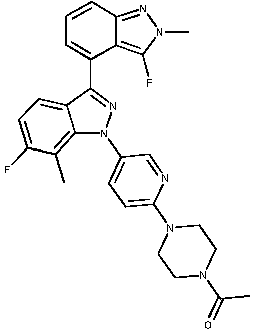 From Intermediate 70 | 1-[4-(5-{3",6-difluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 0.999 min ((M+H)+ 502.2) |
| Example 62 | 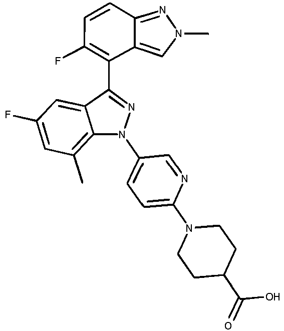 From Intermediate 71 | 1-(5-{5,5"-difluoro-2",7-dimethyl-1H,2"H-[3,4"-biindazol]-1-yl}pyridin-2-yl)piperidine-4-carboxylic acid |
| | | HPLC (Acidic): |
| | | Rt = 0.964 min ((M+H)+ 503.2) |
| Example 63 | 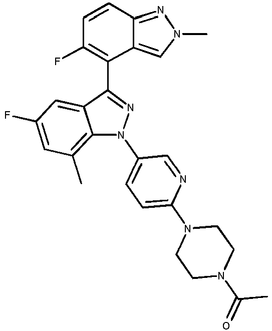 From Intermediate 71 | 1-[4-(5-{5,5"-difluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 1.013 min ((M+H)+ 502.2) |
| Example 64 | 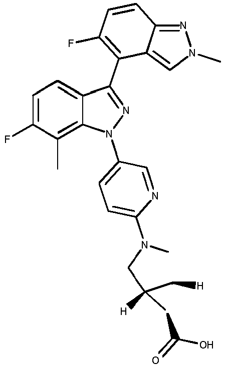 From Intermediate 72 | (1R,5S,6R)-3-(5-{5",6-difluoro-2",7-dimethyl-1H,2"H-[3,4"-biindazol]-1-yl}pyridin-2-yl)-3-azabicyclo[3.1.0]hexane-6-carboxylic acid |
| | | HPLC (Acidic): |
| | | Rt = 0.928 min ((M+H)+ 501.0) |
| Example 65 | 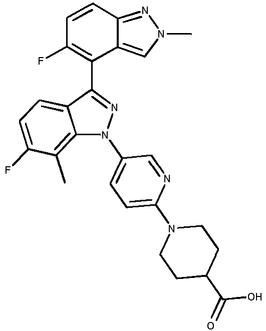 From Intermediate 72 | 1-(5-{5",6-difluoro-2",7-dimethyl-1H,2"H-[3,4"-biindazol]-1-yl}pyridin-2-yl)piperidine-4-carboxylic acid |
| | | HPLC (Acidic): |
| | | Rt = 0.967 min ((M+H)+ 503.2) |
| Example 66 | 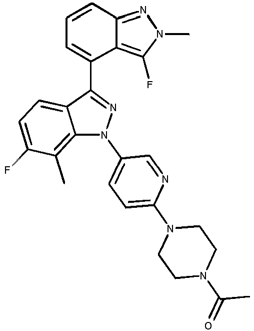 From Intermediate 72 | 1-[4-(5-{5",6-difluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 1.018 min ((M+H)+ 502.2) |
| Example 67 | 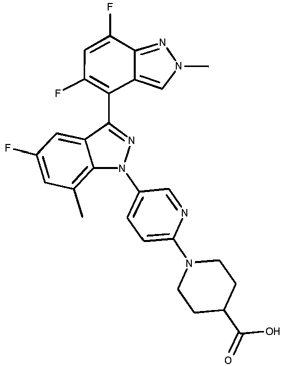 From Intermediate 74 | 1-(5-{5,5",7"-trifluoro-2",7-dimethyl-1H,2"H-[3,4"-biindazol]-1-yl}pyridin-2-yl)piperidine-4-carboxylic acid |
| | | HPLC (Acidic): |
| | | Rt = 1.011 min ((M+H)+ 521.2) |
| Example 68 | 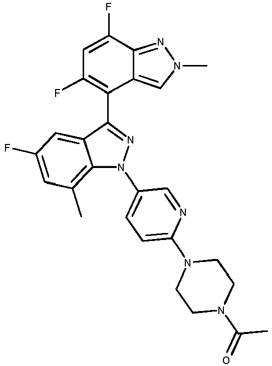 From Intermediate 74 | 1-[4-(5-{5,5",7"-trifluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 1.053 min ((M+H)+ 520.2) |
| Example 69 | 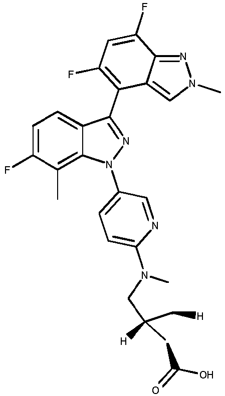 From Intermediate 76 | (1R,5S,6S)-3-(5-{5",6,7"-trifluoro-2",7-dimethyl-1H,2"H-[3,4"-biindazol]-1-yl}pyridin-2-yl)-3-azabicyclo[3.1.0]hexane-6-carboxylic acid |
| | | HPLC (Acidic): |
| | | Rt = 0.968 min ((M+H)+ 519.2) |
| Example 70 | 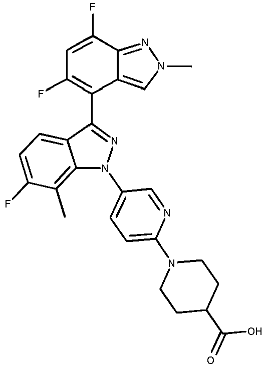 From Intermediate 76 | 1-(5-{5",6,7"-trifluoro-2",7-dimethyl-1H,2"H-[3,4"-biindazol]-1-yl}pyridin-2-yl)piperidine-4-carboxylic acid |
| | | HPLC (Acidic): |
| | | Rt = 1.018 min ((M+H)+ 521.2) |
| Example 71 | 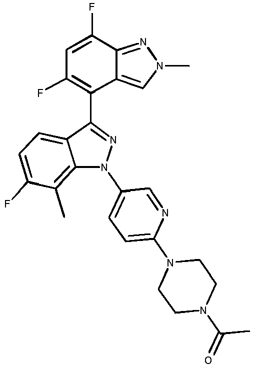 From Intermediate 76 | 1-[4-(5-{5",6,7"-trifluoro-2",7-dimethyl-1H,2"H-[3,4"-biindazol]-1-yl}pyridin-2-yl)piperazin-1-yl]ethan-1-one |
| | | HPLC (Acidic): |
| | | Rt = 1.060 min ((M+H)+ 520.2) |
| Example 72 | 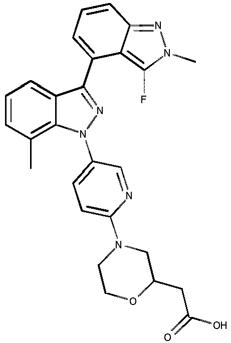 From Intermediate 6 | Chiral Preparative HPLC: |
| | | HPLC: Sepiatec PrepSFC50 |
| | | Column: Chiralpak^{®} IH_10 x 250 mm_5µm |
| | | Eluent: A: 75% scCO2; B: 25% MeOH+20mM NH₃ |
| | | Detection: UV: 220 nm |
| | | Column temp.: 40 °C |
| | | Flow: 15 ml/min |
| | | Gradient: isocratic |
| | | HPLC (Acidic): |
| | | Rt = 0.952 min ((M+H)+ 501.2) |
| Example 72-1 | Enantiomer 1 | 2-[(2R)-4-(5-{3"-fluoro-2",7-dimethyl-1H,2"H-[3,4"-biindazol]-1-yl}pyridin-2-yl)morpholin-2-yl]acetic acid |
| Example 72-2 | Enantiomer 2 | 2-[(2S)-4-(5-{3"-fluoro-2",7-dimethyl-1H,2"H-[3,4"-biindazol]-1-yl}pyridin-2-yl)morpholin-2-yl]acetic acid |
| Example 73 | 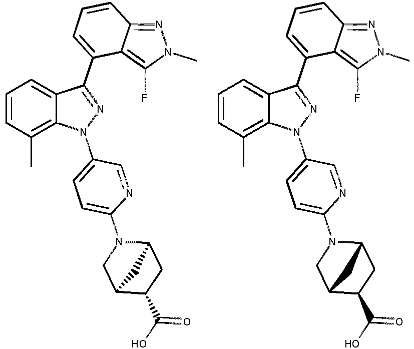 | (1R,4S,5S)-2-(5-{3"-fluoro-2",7-dimethyl-1H,2"H-[3,4"-biindazol]-1-yl}pyridin-2-yl)-2-azabicyclo[2.2.1]heptane-5-carboxylic acid |
| | | (1S,4R,5R)-2-(5-{3"-fluoro-2",7-dimethyl-1H,2"H-[3,4"-biindazol]-1-yl}pyridin-2-yl)-2-azabicyclo[2.2.1]heptane-5-carboxylic acid |
| Example 73-1 | From Intermediate 77 | HPLC (Acidic): |
| | | Rt = 0.862 min ((M+H)+ 497.2) |
| Example 73-2 | From Intermediate 78 | HPLC (Acidic): |
| | | Rt = 0.862 min ((M+H)+ 497.2) |
| Example 74 | 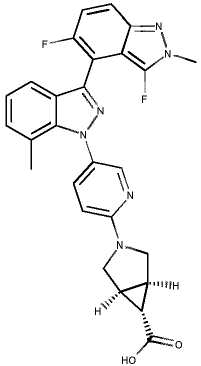 Using the method described for Example 2: Intermediate 4 and Intermediate 79 | (1R,5S,6R)-3-(5-{3",5"-difluoro-2",7-dimethyl-1H,2"H-[3,4"-biindazol]-1-yl}pyridin-2-yl)-3-azabicyclo[3.1.0]hexane-6-carboxylic acid |
| | | HPLC (Acidic): |
| | | Rt = 0.895 min ((M+H)+ 501.2) |
| Example 75 | 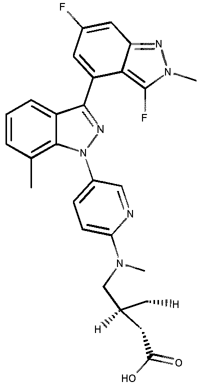 Using the method described for Example 2: Intermediate 4 and Intermediate 80 | (1R,5S,6R)-3-(5-{3",6"-difluoro-2",7-dimethyl-1H,2"H-[3,4"-biindazol]-1-yl}pyridin-2-yl)-3-azabicyclo[3.1.0]hexane-6-carboxylic acid |
| Example 76 | 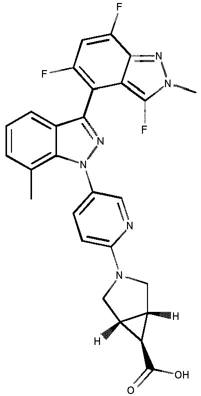 Using the method described for Example 10: Intermediate 15 with Intermediate 82 followed by a hydrolysis step | (1R,5S,6R)-3-(5-{3",5",7"-trifluoro-2",7-dimethyl-1H,2"H-[3,4"-biindazol]-1-yl}pyridin-2-yl)-3-azabicyclo[3.1.0]hexane-6-carboxylic acid |
| | | HPLC (Acidic): |
| | | Rt = 0.914 min ((M+H)+ 519.2) |
| Example 77 | 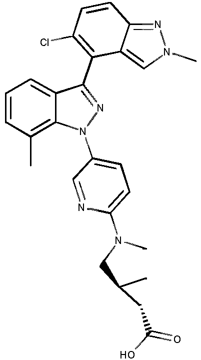 From Intermediate 84 | (1R,5S,6S)-3-(5-{5"-chloro-2",7-dimethyl-1H,2"H-[3,4"-biindazol]-1-yl}pyridin-2-yl)-3-azabicyclo[3.1.0]hexane-6-carboxylic acid |
| | | HPLC (Acidic): |
| | | Rt = 0.904 min ((M+H)+ 499.2) |
| Example 78 | 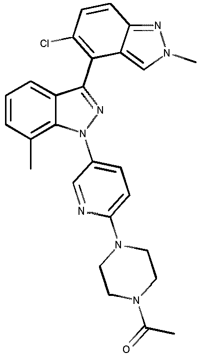 From Intermediate 84 | 1-[4-(5-{5"-chloro-2",7-dimethyl-1H,2"H-[3,4"-biindazol]-1-yl}pyridin-2-yl)piperazin-1-yl]ethan-1-one |
| | | HPLC (Acidic): |
| | | Rt = 0.981 min ((M+H)+ 500.2) |

### PHARMACOLOGICAL ACTIVITY

### BIOLOGICAL EXAMPLES

The compounds of the present disclosure were tested in two assays as described below. That is, the compounds were tested in a dog differential scanning fluorimetry assay, as well as a dog whole blood assay. Representative results from the compounds of the present invention are compiled in Tables 2 and 3 below. Further assays for testing the compounds are also described in the following.

### Dog Differential Scanning Fluorimetry (DSF) Assay

### Dog STING (sSTING) protein production and purification

The protein used for the biophysical experiments was a recombinant dog STING protein comprising its cytosolic cGAMP binding ectodomain. A codon optimized DNA sequence (for expression in Escherichia coli) encoding amino acid residues 149 to 375 of dog STING was synthesized by GeneArt (Regensburg, Germany) and inserted into a pET17b E. coli expression vector. The protein construct encodes an N-terminal 8x His-tag followed by tobacco etch virus protease (TEV) cleavage site and the above STING gene sequence. The resulting protein sequence for the used dog STING protein (SEQ ID No. 1) is listed below:

### His-TEV-dSTING

MHHHHHHHHENLYFQSGEKRNFNVAHGLAWSYFIGYLRLILPGLPARIQALHNNMLQGIGSHRLHIL
FPLDCGVPDDLSVVDPNIRFLYELPQQSANRAGIKRRVYTNSVYELLEKGQPAGICVLEYATPLQTLFA
MSQDGRAGFSREDRLEQAKLFCRTLEDILADAPELQNNCRLIVYQEPAEGSSFSLSQEILRHLRQEERE
VTMGSMDTSIVPTSSTLSQEPNLFISGLEQPLPLRTDIF

For expression of the above recombinant dog STING, the construct was transformed into E. coli BL21 DE3 strain and grown in shake flasks in LB-medium at 15 °C. Expression was induced by addition of isopropyl β-D-1-thiogalactopyranoside to a final concentration of 1mM and cultures shaken overnight. Cell pellets were centrifuged and stored at -70 °C until further use. Protein was purified by cell thawing in lysis buffer (20mM TRIS-HCl, pH 8, 500mM NaCl, 1mM DTT, 0,5 mg/ml lysozyme, Complete Protease Inhibitor (Roche) and DNase (Roche)), followed by metal affinity purification using Ni-NTA resins and elution buffer consisting of 20mM TRIS-HCl, pH 8, 500 mM NaCl, 1 mM DTT, 300 mM imidazole. Cleavage of the His-Tag using TEV-protease took place during dialysis in size exclusion buffer (20 mM TRIS-HCl, pH 8, 100 mM NaCl, 1 mM DTT) over night. To further purify the target protein a reverse Nickel affinity column was used and the flow through was applied to a size exclusion chromatography. The peak fraction was collected and concentrated to 5 mg/mL.

### Biophysical Assay - Determination of the increase of stability of dog STING protein against thermal denaturation, Differential Scanning Fluorimetry (DSF)

The binding affinity of the compounds of the invention was demonstrated using a thermal shift assay that measures the stability of a suitable protein material of dog STING against thermal denaturation in the presence of compounds. In this assay, the unfolding temperature of a protein is monitored in the presence of a fluorescent dye which exhibits affinity for the hydrophobic amino acids of the protein that are buried in its folded state and are gradually exposed during unfolding. Dye fluorescence is quenched in aqueous environment and increases upon association of the dye with the hydrophobic parts of the unfolding protein. A plot of the fluorescence intensity as a function of temperature typically displays a sigmoidal curve that is interpreted by a two-state model of protein unfolding (Differential Scanning Fluorimetry). The inflection point of the curve represents the "melting" temperature of the protein (Tm) which is calculated numerically using the Boltzmann equation.

The thermal stability of the dog STING protein was measured in assay buffer containing 20 mM Tris, 150 mM NaCl at pH7.5. The assay uses 384-Well qPCR Plates (Catalog #781358, BRAND), Microseal^{®}'B' Adhesive Seals for PCR Plates (Catalog# MSB-1001, BIO-RAD) and was run on a CFX384 Real-Time System (Bio-Rad). A DMSO stock solution of SYPRO orange (SIGMA S5692-500UL) was prepared. Compound stock solutions (10 mM in DMSO) were diluted 1:2 in DMSO to an intermediate compound concentration of 5 mM and then further diluted 1:40 in assay buffer resulting in a compound concentration of 125 µM and 2.5 % DMSO. Fluorescent dye stock solution (5000x SYPRO Orange) was then mixed with target protein and buffer to a concentration of 15 µM Protein and 25x SYPRO Orange. 2 µl of this protein-dye-mixture was added to 8 µl compound solution. Final volume was 10 µL. 3-6 well positions were used as negative control (protein with 2 % DMSO). The plates were prepared for duplicate measurement and centrifuged for 2 min at 1000 g. In the measurement, 160 cycles of 0.5 °C were used (temperature ramp 15 s/cycle, 15 °C to 95 °C).

Final Assay concentrations for compound characterization were 100 µM compound, 3 µM target protein, 5x SYPRO Orange, and 2% DMSO in 10 µl. All dispensing steps were performed using a HamiltonStar pipetting robot (Hamilton).

Dissociation curves were processed in Bio-Rad CFX Manager. Peak type was set to "negative". Compound codes for screen were assigned in the plate layout.

Two replicates of TM measurements were averaged, and the standard deviation was calculated. In cases of SD>1.5 °C the measurement was repeated.

The melting point (Tm) obtained for STING protein alone was subtracted from T obtained for protein incubated with ligand to generate ΔTm values.

### Dog Whole Blood Assay

For the detection of STING activation in physiological environment dog whole blood (dWB) was stimulated by the cyclic dinucleotide cGAMP or a test compound. Pathway activity was monitored by measuring the IFNb production.

Compounds were delivered as 10 mM DMSO solution, diluted and transferred by using an Echo acoustic dispenser to the 384well assay plate (Greiner # 781182), pre-filled with 10 µl 1x HBSS in each well (10x HBSS (+Ca/+Mg), #14065-049, Gibco). Typically, 8 concentrations were used with the highest concentration at 10 µM in the final assay volume followed by approximately 1:4 dilution steps. DMSO concentration was set to 0.1 % in the final assay volume. The 384-well assay plate contained 20 test compounds and DMSO in control and cGAMP standard wells. The dog whole blood was collected as Na-Citrate blood (e.g., 3.8 % in Monovettes from Sarstedt) and kept at 4 °C overnight until use in the assay. 80µl of the whole blood samples were transferred to each well of the 384-well assay plates filled with compound/1xHBSS. Blood plates were kept at room temperature for 60 minutes and continuous shaking with 450 rpm, covered with the lid, but not sealed. A 10x cGAMP assay solution was diluted from a 2 mM stock solution in 1xHBSS immediately before use at room temperature. 10 µl of the 10x cGAMP/HBSS were added to the high control wells, whereas HBSS only was added to all compound and low control wells. After covering assay plates with the lid, blood plates were kept for a 4h incubation at 37 °C in the incubator, without shaking. For the detection of IFNb in dog plasma the ELISA Kit for Canine Interferon Beta (Biotrend # SEA222Ca) was used. Whole blood assay plates were centrifuged at 1000 g for 10 minutes at 8 °C. 40 µl of supernatant was transferred with a 96 well pipetting robotics from the 384 well whole blood plate to the corresponding 96 well ELISA plate, pre-filled with 60µl assay diluent in each well. Plates were sealed with microplate seals and kept at 4 °C overnight again. ELISA plates were brought to room temperature, followed by a 1h incubation at 37 °C in the incubator. Detection Reagent A working solution was prepared by diluting Detection Reagent A 1:100 in Assay Reagent A. Liquid was then removed from the 96 well ELISA plate to add 100 µL Detection Reagent A working solution to each well. ELISA plates were covered with the plate sealer and incubated for 1 hour at 37 °C in the incubator. The 1x Wash buffer was prepared by diluting the 30x Wash Buffer Concentrate in H2O. Detection Reagent B working solution was prepared by diluting Detection Reagent B 1:100 in Assay Reagent B. The ELISA plates were washed three times with 350 µl wash buffer and afterwards inverted and blotted against absorbent paper to remove any liquid. 100 µl of Detection Reagent B working solution was added to each well of the ELISA plates, which are then covered with the plate sealer and incubated for 30 minutes at 37 °C in the incubator. After incubation the ELISA assay plates were washed five times with 350 µl wash buffer and were again inverted and blotted against absorbent paper to remove any remaining liquid. 90 µL of TMB Substrate was added to each well of the ELISA plates, which were then covered with the plate sealer and incubated for 15 minutes at 37 °C in the incubator. The reaction was stopped by adding 50 µL stop solution and absorbance was measured at 450 nm immediately.

### Data evaluation and calculation:

For data evaluation and calculation, % control calculation of each well was based on the mean of high (cGAMP stimulated control) and mean of low (unstimulated control) controls by using the following standard 4 parameter logistic regression formula: $\left[y=\left(a-d\right)/\left(1+{\left(x/c\right)}^{∧}b\right)+d\right]$
a = low value,
d = high value,
x = conc M,
c = EC50 M,
b = slope.

### Dog Liver Microsome (dLM) Assay

The metabolic degradation of the test compound is assayed at 37 °C with pooled liver microsomes from dogs (Beagle). The final incubation volume of 100 µl per time point contains TRIS buffer pH 7.6 at RT (0.1 M), magnesium chloride (5 mM), microsomal protein (1 mg/ml) and the test compound at a final concentration of 1 µM. Following a short preincubation period at 37 °C, the reactions were initiated by addition of beta-nicotinamide adenine dinucleotide phosphate, reduced form (NADPH, 1 mM) and terminated by transferring an aliquot into solvent after different time points. Additionally, the NADPH-independent degradation was monitored in incubations without NADPH, terminated at the last time point. The [%] remaining test compound after NADPH independent incubation is reflected by the parameter c(control) (metabolic stability). The quenched incubations are pelleted by centrifugation (10000 g, 5 min).

An aliquot of the supernatant is assayed by LC-MS/MS for the amount of parent compound. The half-life (t1/2 INVITRO) is determined by the slope of the semilogarithmic plot of the concentration-time profile. The intrinsic clearance (CL_INTRINSIC) is calculated by considering the amount of protein in the incubation: CL_INTRINSIC [µl/min/mg protein] = (Ln 2 / (half-life [min] * protein content [mg/ml])) * 1000. For better across species comparison the predicted clearance is expressed as percent of the liver blood flow [% QH] in the individual species. In general, high stability (corresponding to low % QH) of the compounds across species is desired.

### Mouse Liver Microsome (mLM) Assay

The metabolic degradation of the test compound is assayed at 37 °C with pooled liver microsomes from (male/female) mice (CD1). The final incubation volume of 100 µl per time point contains TRIS buffer pH 7.6 at RT (0.1 M), magnesium chloride (5 mM), microsomal protein (0.5 mg/ml) and the test compound at a final concentration of 1 µM. Following a short preincubation period at 37 °C, the reactions were initiated by addition of beta-nicotinamide adenine dinucleotide phosphate, reduced form (NADPH, 1 mM) and terminated by transferring an aliquot into solvent after different time points. Additionally, the NADPH-independent degradation was monitored in incubations without NADPH, terminated at the last time point. The [%] remaining test compound after NADPH independent incubation is reflected by the parameter c(control) (metabolic stability). The quenched incubations are pelleted by centrifugation (10000 g, 5 min).

An aliquot of the supernatant is assayed by LC-MS/MS for the amount of parent compound. The half-life (t1/2 INVITRO) is determined by the slope of the semilogarithmic plot of the concentration-time profile. The intrinsic clearance (CL_INTRINSIC) is calculated by considering the amount of protein in the incubation: CL_INTRINSIC [µl/min/mg protein] = (Ln 2 / (half-life [min] * protein content [mg/ml])) * 1000. For better across species comparison the predicted clearance is expressed as percent of the liver blood flow [% QH] in the individual species. In general, high stability (corresponding to low % QH) of the compounds across species is desired.

### Dog Hepatocyte (dHep) Assay

The metabolic degradation of the test compound is assayed in a suspension of dog hepatocyte cells.

Incubation: Cryopreserved dog hepatocyte cells are incubated in an appropriate buffer system (KHB buffer or similar buffer or standard cell culture medium) containing 50 % species serum. Following an acclimation period (15-30 min) in an incubator (37 °C, 5-10 % CO2, 85 - 95 % humidity) the test compound is added to the hepatocyte suspension (pH 7.4, typical cell density of about 1 million cells/mL; final concentration of test compound is 1 µM, final DMSO concentration <0.05 % v/v). The cells are incubated for up to 6 hours and samples are taken at 6 different time points. Samples are then quenched with acetonitrile and pelleted by centrifugation. The remaining amount of parent compound in the supernatants is then analysed by HPLC-MS/MS.

Calculation: The elimination rate constant (ke) is calculated using the slope of the linear regression from natural log [substrate % remaining or substrate concentration] vs. time [h]. $ke = \left(-1\right) * Slope$ ke = elimination rate constant [1/h]

Half-life is calculated from the elimination rate constant $t \frac{1}{2} = ln \left(2\right) / ke$ $t \frac{1}{2} = half - life \left[h\right]$

Calculation of intrinsic in_vitro hepatic clearance: $CL_int_in vitro = ke * 1000 / \left(CD*60\right)$
CL_int _in vitro = intrinsic hepatic clearance, in vitro [µl/min/Mio cells]
CD = Cell density [Mio cells/mL]
Note: the above equation is only true when [Substrate] << Km.

The calculated in *vitro* hepatic intrinsic clearance can be scaled up to the intrinsic *in vivo* hepatic Clearance and used to predict hepatic *in vivo* blood clearance (CL_ws) by the use of a liver model (well stirred model). $CL_int_invivo = \left(CL_int_in vitro*H*L\right) / 1000$
CL_int_invivo = intrinsic hepatic clearance, *in vivo* [mL/min/kg]
H = hepatocellularity [Mio cells/g liver]
L = liver factor [g/kg body weight] $CL_ws = CL_int_invivo * Q / \left(CL_int_invivo+Q\right)$
CL = Estimate of hepatic clearance, in vivo [mL/min/kg]
Q = hepatic blood flow [mL/min/kg]
ws= well stirred $QH % = CL_ws * 100 / Q$
QH% = Clearance expressed as a percent of hepatic blood flow
CONC: cell concentration at incubation time (10^6/ml)
T_LAST: terminal time point used (h)

### Mouse Hepatocyte (mHep) Assay

The metabolic degradation of the test compound is assayed in a suspension of mouse hepatocyte cells.

Incubation: Cryopreserved mouse hepatocyte cells are incubated in an appropriate buffer system (KHB buffer or similar buffer or standard cell culture medium) containing 50 % species serum. Following an acclimation period (15-30 min) in an incubator (37 °C, 5-10% CO2, 85 - 95 % humidity) the test compound is added to the hepatocyte suspension (pH 7.4, typical cell density of about 1 million cells/mL; final concentration of test compound is 1 µM, final DMSO concentration <0.05 % v/v). The cells are incubated for up to 6 hours and samples are taken at 6 different time points. Samples are then quenched with acetonitrile and pelleted by centrifugation. The remaining amount of parent compound in the supernatants is then analysed by HPLC-MS/MS. Calculation: The elimination rate constant (ke) is calculated using the slope of the linear regression from natural log [substrate % remaining or substrate concentration] vs. time [h]. $ke = \left(-1\right) * Slope$ ke = elimination rate constant [1/h].

Half-life is calculated from the elimination rate constant $t \frac{1}{2} = ln \left(2\right) / ke$ $t \frac{1}{2} = half - life \left[h\right]$

Calculation of intrinsic _in vitro hepatic clearance: $CL_int_in vitro = ke * 1000 / \left(CD*60\right)$
CL_int _in vitro = intrinsic hepatic clearance, in *vitro* [µl/min/Mio cells]
CD = Cell density [Mio cells/mL]
Note: the above equation is only true when [Substrate] << Km.

The calculated in *vitro* hepatic intrinsic clearance can be scaled up to the intrinsic *in vivo* hepatic Clearance and used to predict hepatic *in vivo* blood clearance (CL_ws) by the use of a liver model (well stirred model). $CL_int_invivo = \left(CL_int_in vitro*H*L\right) / 1000$
CL_int_invivo = intrinsic hepatic clearance, *in vivo* [mL/min/kg]
H = hepatocellularity [Mio cells/g liver]
L = liver factor [g/kg body weight] $CL_ws = CL_int_invivo * Q / \left(CL_int_invivo+Q\right)$
CL = Estimate of hepatic clearance, *in vivo* [mL/min/kg]
Q = hepatic blood flow [mL/min/kg]
ws = well stirred $QH % = CL_ws * 100 / Q$
QH% = Clearance expressed as a percent of hepatic blood flow
CONC: cell concentration at incubation time (10^6/ml)
T_LAST: terminal time point used (h)
Hepatocellularity, mouse: 120x10e6 cells / g liver
Liver factor, mouse: 55 g / kg bodyweight
Blood flow, mouse: 90 ml/(min x kg).

### Permeability Assay (MDCK-PGP)

The assay provides information on the potential of a compound to pass the blood brain barrier. Permeability measurements across polarized, confluent MDCK-MDR1 cell monolayers grown on permeable filter supports are used as the in *vitro* absorption model.

Apparent permeability coefficients (PE) of the compounds across the MDCK-MDR1 cell monolayers are measured (pH 7.4, 37 °C) in apical-to-basal (AB) and basal-to-apical (BA) transport direction. AB permeability (PEAB) represents drug absorption from the blood into the brain and BA permeability (PEBA) drug efflux from the brain back into the blood via both passive permeability as well as active transport mechanisms mediated by efflux and uptake transporters that are expressed on the MDCK-MDR1 cells, predominantly by the overexpressed human MDR1 P-gp. The compounds are assigned to permeability/absorption classes by comparison of the AB permeabilities with the AB permeabilities of reference compounds with known in *vitro* permeability and oral absorption in the human. Identical or similar permeabilities in both transport directions indicate passive permeation, vectorial permeability points to additional active transport mechanisms. Higher PEBA than PEAB indicates the involvement of active efflux mediated by MDR1 P-gp. Active transport is concentration-dependently saturable.

MDCK-MDR1 cells (1-2 x 10^5 cells/1 cm^2 area) are seeded on filter inserts (Costar transwell polycarbonate or PET filters, 0.4 µm pore size) and cultured (DMEM) for 7 days. Subsequently, the MDR1 expression is boosted by culturing the cells with 5 mM sodium butyrate in full medium for 2 days. Compounds are dissolved in appropriate solvent (like DMSO, 1 -20 mM stock solutions). Stock solutions are diluted with HTP-4 buffer (128.13 mM NaCl, 5.36 mM KCl, 1 mM MgSO4, 1.8 mM CaCl2, 4.17 mM NaHCO3, 1.19 mM Na2HPO4 x 7H2O, 0.41 mM NaH2PO4xH2O, 15 mM HEPES, 20 mM glucose, 0.25 % BSA, pH 7.4) to prepare the transport solutions (0.1 - 300 µM compound, final DMSO <= 0.5 %). The transport solution (TL) is applied to the apical or basolateral donor side for measuring A-B or B-A permeability (3 filter replicates), respectively. The receiver side contains the same buffer as the donor side. Samples are collected at the start and end of experiment from the donor and at various time intervals for up to 2 hours also from the receiver side for concentration measurement by HPLC-MS/MS or scintillation counting. Sampled receiver volumes are replaced with fresh receiver solution.

### Results

**Table 2: Interaction with dog STING as determined by DSF (dDSF)**

| **Example No.** | **Delta TM (°C)** |
|---|---|
| Example 1 | 35.0 |
| Example 2 | 34.5 |
| Example 3 | 32.2 |
| Example 4 | 31.0 |
| Example 5 | 36.5 |
| Example 6 | 35.5 |
| Example 7 | 35.5 |
| Example 8 | 32.5 |
| Example 9 | 28.5 |
| Example 10 | 33.1 |
| Example 11 | 34.8 |
| Example 12 | 21.7 |
| Example 13 | 33.3 |
| Example 14 | 38.0 |
| Example 15 | 36.8 |
| Example 16 | 34.0 |
| Example 17 | 35.8 |
| Example 18 | 28.8 |
| Example 19 | 34.3 |
| Example 20-1 | 20.9 |
| Example 20-2 | 22.6 |
| Example 20-3 | 35.9 |
| Example 20-4 | 36.9 |
| Example 21 | 24.9 |
| Example 22 | 30.4 |
| Example 23 | 29.6 |
| Example 24 | 35.5 |
| Example 25 | 37.0 |
| Example 26-1 | 31.0 |
| Example 26-2 | 32.0 |
| Example 27-1 | 36.9 |
| Example 27-2 | 35.6 |
| Example 28-1 | 26.6 |
| Example 28-2 | 25.9 |
| Example 28-3 | 37.1 |
| Example 28-4 | 37.9 |
| Example 29 | 32.0 |
| Example 30 | 30.8 |
| Example 31 | 30.5 |
| Example 32 | 31.0 |
| Example 33 | 22.5 |
| Example 34 | 24.8 |
| Example 35 | 22.5 |
| Example 36 | 24.0 |
| Example 37-1 | 16.0 |
| Example 37-2 | 18.0 |
| Example 38-1 | 24.8 |
| Example 38-2 | 23.0 |
| Example 39 | -0.3 |
| Example 40 | 17.8 |
| Example 41 | 16.0 |
| Example 42 | 15.8 |
| Example 43 | 16.0 |
| Example 44 | 34.5 |
| Example 45 | 33.0 |
| Example 46 | 35.3 |
| Example 47-1 | 34.0 |
| Example 47-2 | 35.5 |
| Example 48-1 | 23.9 |
| Example 48-2 | 24.6 |
| Example 48-3 | 35.4 |
| Example 48-4 | 36.1 |
| Example 49 | 30.5 |
| Example 50 | 25.0 |
| Example 51 | 29.0 |
| Example 52 | 25.8 |
| Example 53 | 33.0 |
| Example 54 | 30.0 |
| Example 55 | 34.5 |
| Example 56-1 | 33.0 |
| Example 56-2 | 33.0 |
| Example 57 | 27.5 |
| Example 58 | 33.0 |
| Example 59 | 22.0 |
| Example 60 | 34.5 |
| Example 61 | 30.8 |
| Example 62 | 38.0 |
| Example 63 | 27.4 |
| Example 64 | 36.5 |
| Example 65 | 36.5 |
| Example 66 | 25.8 |
| Example 67 | 37.0 |
| Example 68 | 28.5 |
| Example 69 | 35.0 |
| Example 70 | 34.5 |
| Example 71 | 31.3 |
| Example 72-1 | 30.0 |
| Example 72-3 | 28.5 |
| Example 73-1 | 34.5 |
| Example 73-2 | 33.3 |
| Example 74 | 36.4 |
| Example 75 | 21.8 |
| Example 76 | 33.0 |
| Example 77 | 27.4 |
| Example 78 | 18.4 |

**Table 3: Cytokine secretion in dog whole blood (dWB) culture system as determined by canine Interferon-beta (IFNb) ELISA**

| **Example No.** | **EC50 (nM)** |
|---|---|
| Example 1 | 180.4 |
| Example 2 | 235.8 |
| Example 3 | 730.2 |
| Example 4 | 757.1 |
| Example 5 | 282.1 |
| Example 6 | 243.1 |
| Example 7 | 249.7 |
| Example 8 | 168.5 |
| Example 9 | 192.1 |
| Example 10 | 349.8 |
| Example 11 | 458.2 |
| Example 12 | 608.5 |
| Example 13 | 173.7 |
| Example 14 | 391.3 |
| Example 15 | 1170.4 |
| Example 16 | 267.3 |
| Example 17 | 184.9 |
| Example 18 | 1065.2 |
| Example 20-3 | 195.4 |
| Example 20-4 | 206.9 |
| Example 21 | 1005.4 |
| Example 22 | 243.5 |
| Example 23 | 219.4 |
| Example 24 | 269.9 |
| Example 25 | 282.5 |
| Example 27-1 | 211.1 |
| Example 27-2 | 247.1 |
| Example 28-3 | 222.3 |
| Example 28-4 | 205.5 |
| Example 29 | 139.6 |
| Example 30 | 226.8 |
| Example 31 | 100.8 |
| Example 34 | 3283.7 |
| Example 44 | 387.8 |
| Example 45 | 600.7 |
| Example 46 | 261.4 |
| Example 47-1 | 628.5 |
| Example 47-2 | 199.6 |
| Example 48-3 | 183.8 |
| Example 48-4 | 169.5 |
| Example 49 | 415.7 |
| Example 51 | 670.2 |
| Example 53 | 654.1 |
| Example 54 | 261.2 |
| Example 55 | 197.3 |
| Example 56-1 | 275.3 |
| Example 56-1 | 91.0 |
| Example 58 | 207.2 |
| Example 59 | 207.2 |
| Example 60 | 195.4 |
| Example 61 | 244.7 |
| Example 62 | 294.8 |
| Example 63 | 96.8 |
| Example 64 | 665.8 |
| Example 65 | 330.0 |
| Example 66 | 179.0 |
| Example 67 | 102.0 |
| Example 68 | 106.2 |
| Example 70 | 296.5 |
| Example 72-1 | 809.7 |
| Example 72-3 | 5232.3 |
| Example 73-1 | 87.5 |
| Example 73-2 | 232.2 |
| Example 74 | 160.4 |
| Example 76 | 694.7 |
| Example 77 | 1085.9 |
| Example 78 | 817.0 |

As demonstrated by the present examples, when measured with a binding assay, the compounds in accordance with the invention show an interaction with dog STING (dSTING) reflected by a shift in Tₘ of > 15 °C, more preferably > 20 °C, and even more preferably > 25 °C, as determined by DSF. Furthermore, the compounds in accordance with the present invention induce cytokine secretion in dog whole blood (dWB). In accordance with the invention, a combination of a high dDSF and low dWB is especially favorable.

## Claims

1. A compound of formula (I) 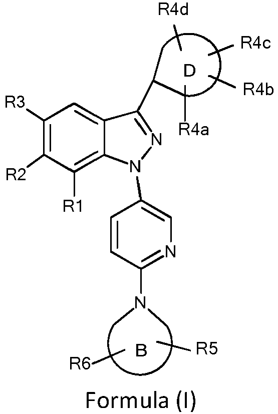 wherein
**B** is a group selected from among the group consisting of
a 5-7-membered monocyclic heterocyclyl containing 1 or 2 N-atoms,
a 6-membered bicyclic heterocyclyl containing 1 N-atom,
a 7-11 membered bicyclic heterocyclyl containing 1 or 2 N-atoms,
a 7-membered bicyclic heterocyclyl containing 1 N-atom and 1 O-atom,
a 6-membered monocyclic heterocyclyl containing 1 N-atom and 1 heteroatom selected from among the group consisting of O and S,
a 9-membered bicyclic heterocyclyl containing 3 heteroatoms, 2 of which are N and the other is O,
a 9-membered bicyclic heterocyclyl containing 1 N-atom and 1 S-atom,
a 10-membered bicyclic heterocyclyl containing 3 N-atoms, 2 of which are substituted with C₁₋₆-alkyl,
phenyl,
a 9-membered bicyclic heteroaryl containing 3 N-atoms,
-C₁₋₄-alkylene-pyrimidine, and
-C₁₋₄-alkylene-O-C₁₋₃-alkyl;
D is a group selected from among the group consisting of
a 9-membered bicyclic heteroaryl containing 2 N-atoms,
a 10-membered bicyclic heteroaryl containing 1 N-atom, and benzodioxole;
**R¹** is selected from among the group consisting of -H or -C₁₋₆-alkyl;
**R²** is selected from among the group consisting of -H, halogen, preferably fluorine or chlorine, more preferably fluorine, **and** -C₁₋₆-alkyl;
**R³** is selected from among the group consisting of -H, halogen, preferably fluorine or chlorine, more preferably fluorine, **and** -C₁₋₆-alkyl;
**R^{4a}, R^{4b}** and **R^{4c}** are each independently selected from -H, halogen, preferably fluorine or chlorine, more preferably fluorine, and C₁₋₆-alkyl, with the proviso that at least one of **R^{4a}, R^{4b}** and **R^{4c}** is halogen;
**R^{4d}** is selected from -C₁₋₆-alkyl and C₃₋₆ cycloalkyl;
**R⁵** is absent **or** is selected from among the group consisting of -H, -C₁₋₆-alkyl, -S(O₂)-C₁-₆-alkyl, -NH-S(O₂)-C₁-₆-alkyl, =O, -C(O)-C₁-₆-alkyl, -C(O)H, -C(O)OH, -C(O)NH₂, -C(O)O-C₁-₆-alkyl, -NR^{5.1}R^{5.2}, -C₁-₆-alkylene-C(O)OH, -S(O₂)-NH₂, -pyrolidin-2-one-1-yl, -tetrazolyl, **and** a 5-membered heteroaryl with 1 or 2 heteroatoms selected from among the group consisting of N and O which is substituted with R^{5.3};
**R^{5.1}** is selected from among the group consisting of -H, -C₁-₆-alkyl, -C(O)-C₁-₆-alkyl **and** -C₁-₆-alkylene-O-C₁-₆-alkyl;
**R^{5.2}** is selected from among the group consisting of -H, -C₁-₆-alkyl, -C(O)-C₁-₆-alkyl **and** -C₁-₆-alkylene-O-C₁-₆-alkyl;
**R^{5.3}** is selected from among the group consisting of -H, -C₁-₆-alkyl **and** a 6-membered heteroaryl with 1 or 2 heteroatoms selected from a group consisting of N and O;
**R⁶** is absent **or** is selected from among the group consisting of -H, -C₁₋₆-alkyl, =O **and** -C(O)OH;
or a pharmaceutically acceptable salt thereof.

2. The compound according to claim 1, wherein **R¹** is -C₁₋₆-alkyl; and
at least one of **R^{4a}, R^{4b}** and **R^{4c}** is fluorine or chlorine, and the other ones are -H or -C₁-₃-alkyl; and **R^{4d}** is -C₁₋₆-alkyl;
or a pharmaceutically acceptable salt thereof.

3. A compound according to any one of claims 1-2, wherein
**D** is selected from among the group consisting of 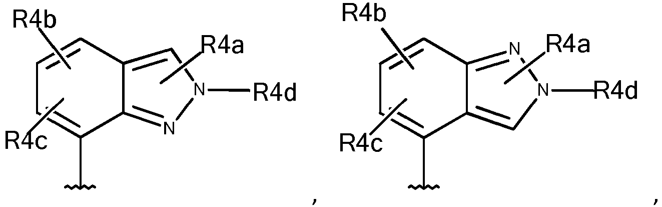 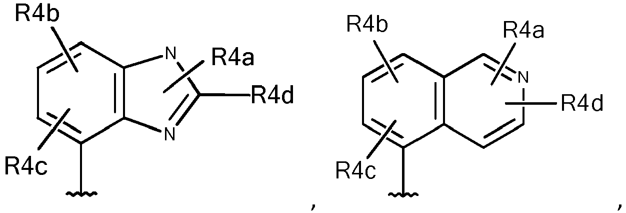 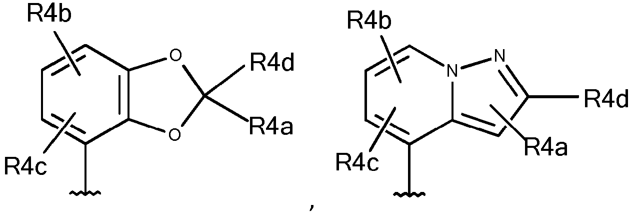 and 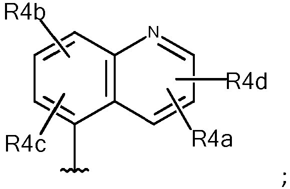 wherein
**R^{4d}** is C₁₋₆-alkyl;
or a pharmaceutically acceptable salt thereof.

4. A compound according to any one of claims 1-3, wherein **D** is 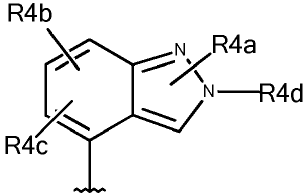 or a pharmaceutically acceptable salt thereof.

5. A compound according to any one of claims 1-4, wherein **D** is selected from among the group consisting of the following structures: 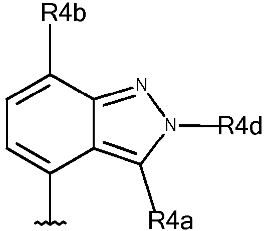 wherein
R^{4a} is -H;
R^{4b} is halogen, preferably chlorine or fluorine, more preferably fluorine; and
R^{4d} is methyl;
or
R^{4a} is halogen, preferably chlorine or fluorine, more preferably fluorine;
R^{4b} is -H; and
R^{4d} is methyl;
or
R^{4a} is halogen, preferably chlorine or fluorine, more preferably fluorine;
R^{4b} is halogen, preferably chlorine or fluorine, more preferably fluorine; and
R^{4d} is methyl;
or 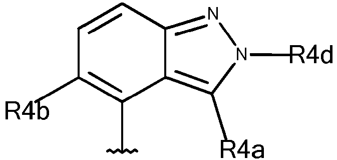
wherein
R^{4a} is -H;
R^{4b} is halogen, preferably chlorine or fluorine, more preferably fluorine; and
R^{4d} is methyl
or
R^{4a} is halogen, preferably chlorine or fluorine, more preferably fluorine;
R^{4b} is -H; and
R^{4d} is methyl;
or
R^{4a} is halogen, preferably chlorine or fluorine, more preferably fluorine;
R^{4b} is halogen, preferably chlorine or fluorine, more preferably fluorine; and
R^{4d} is methyl;
or 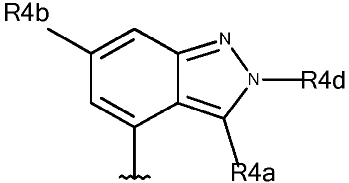 wherein
R^{4a} is -H;
R^{4b} is halogen, preferably chlorine or fluorine, more preferably fluorine; and
R^{4d} is methyl;
or
R^{4a} is halogen, preferably chlorine or fluorine, more preferably fluorine;
R^{4b} is -H; and
R^{4d} is methyl;
or
R^{4a} is halogen, preferably chlorine or fluorine, more preferably fluorine;
R^{4b} is halogen, preferably chlorine or fluorine, more preferably fluorine; and
R^{4d} is -methyl;
or 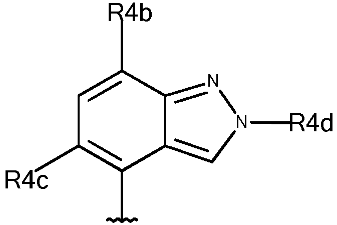 wherein
R^{4b} is -H or methyl;
R^{4c} is halogen, preferably chlorine or fluorine, more preferably fluorine; and
R^{4d} is methyl;
or
R^{4b} is halogen, preferably chlorine or fluorine, more preferably fluorine;
R^{4c} is -H or methyl; and
R^{4d} is methyl;
or
R^{4b} is halogen, preferably chlorine or fluorine, more preferably fluorine;
R^{4c} is halogen, preferably chlorine or fluorine, more preferably fluorine; and
R^{4d} is -methyl;
or a pharmaceutically acceptable salt thereof.

6. A compound according to any one of claims 1-5, wherein
R¹ is methyl; R² is -H or halogen; and R³-H or halogen;
or a pharmaceutically acceptable salt thereof.

7. A compound according to any one of claims 1-6, wherein
R¹ is methyl, R² is -H; and R³ is -H;
or
R¹ is methyl, R² is -H; and R³ is fluorine;
or
R¹ is methyl, R² is fluorine; and R³ is -H;
or a pharmaceutically acceptable salt thereof.

8. A compound according to any one of claims 1-7, wherein **B** is selected from among the group consisting of 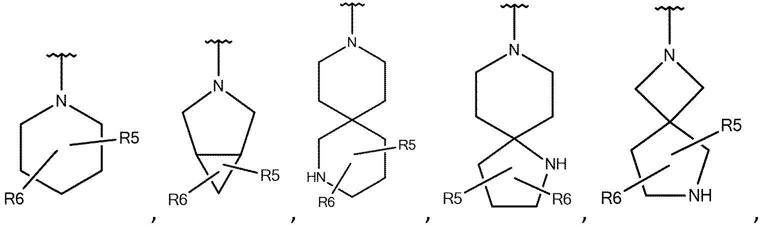 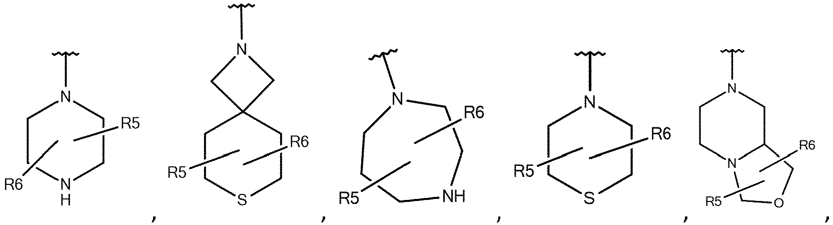 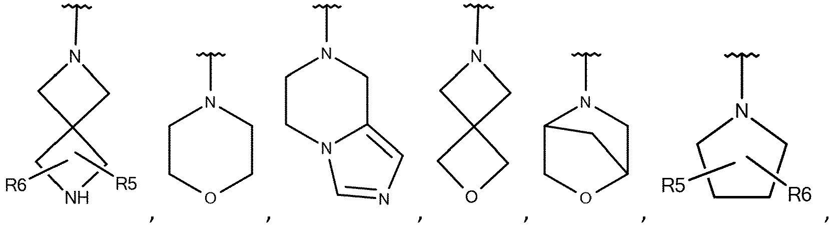 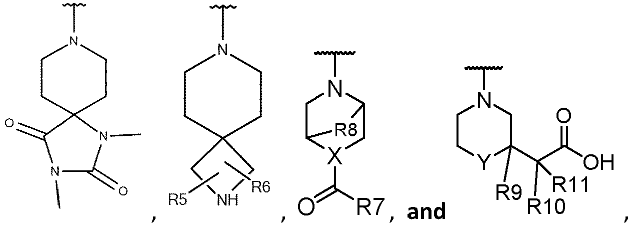
wherein **R⁷** is selected from H, -C₁₋₆-alkyl, -C₃-₆-cycloalkyl and -OH;
**R⁸** is (CH₂)ₙ, wherein n is an integer of 1-3, preferably 1 or 2;
**R⁹** is selected from among the group consisting of H, -C₁₋₆-alkyl, and -C₃-₆-cycloalkyl;
**R¹⁰** is selected from among the group consisting of H, -C₁₋₆-alkyl, and -C₃-₆-cycloalkyl;
**R¹¹** is selected from among the group consisting of H, -C₁₋₆-alkyl, and -C₃-₆-cycloalkyl;
**X** is CH or N; and
**Y** is -O-, -S-, -S(O)-, -S(O)₂-;
or a pharmaceutically acceptable salt thereof.

9. A compound according to any one of claims 1-8, wherein B is selected from among the group consisting of 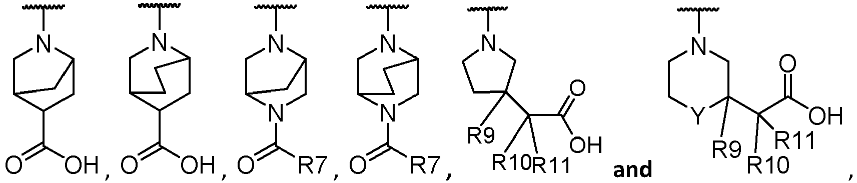
wherein **R⁷** is selected from among the group consisting of -H and -C₁₋₆-alkyl;
**R⁹** is selected from among the group consisting of -H and methyl, and is preferably -H;
**R¹⁰** is selected from among the group consisting of -H and methyl, and is preferably -H;
**R¹¹** is selected from among the group constating of -H and methyl, and is preferably -H; and
**Y** is O;
or a pharmaceutically acceptable salt thereof.

10. A compound according to claim 1 which is selected from among the group consisting of the following structures: 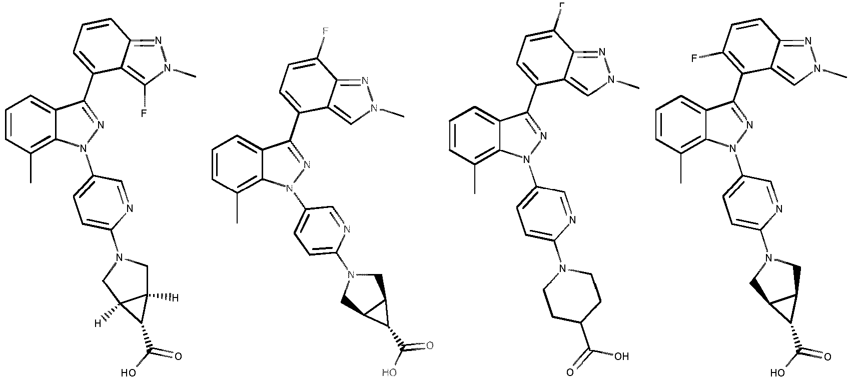 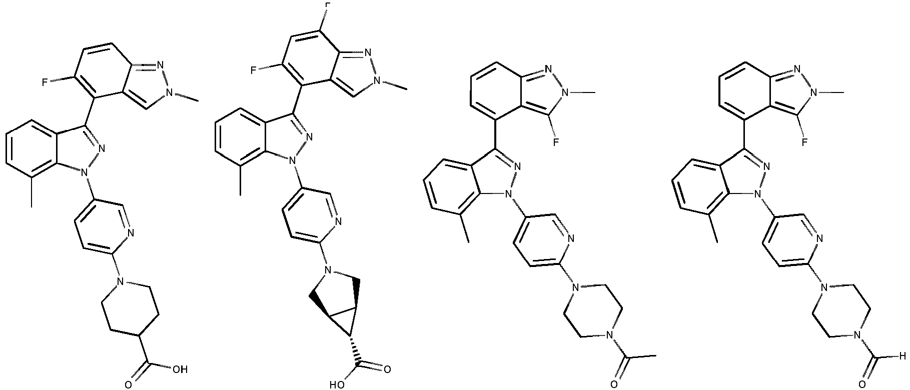 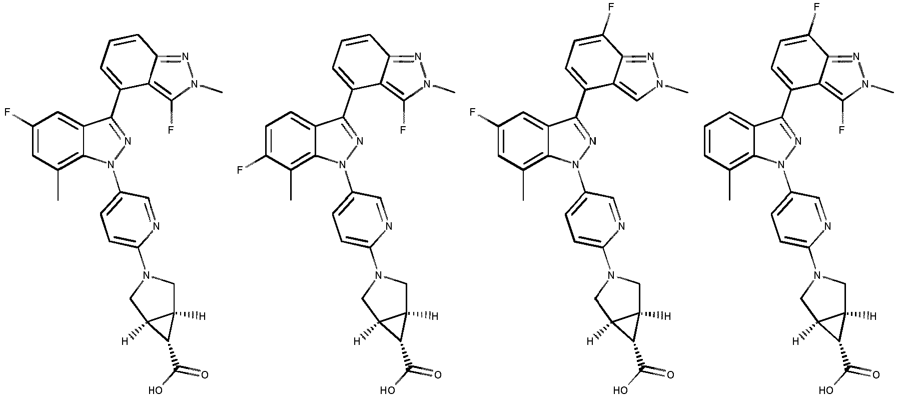 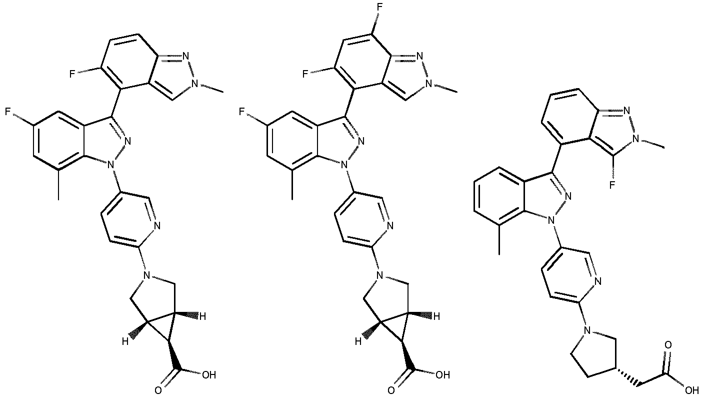 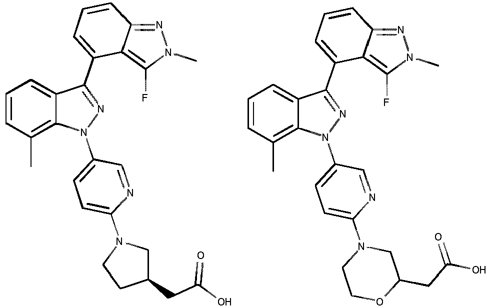 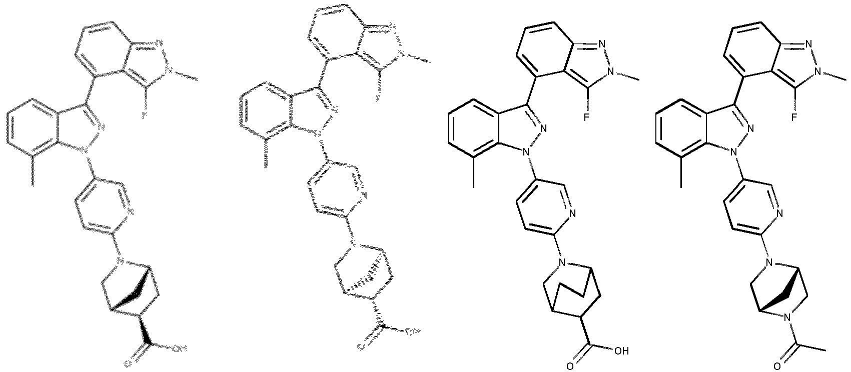 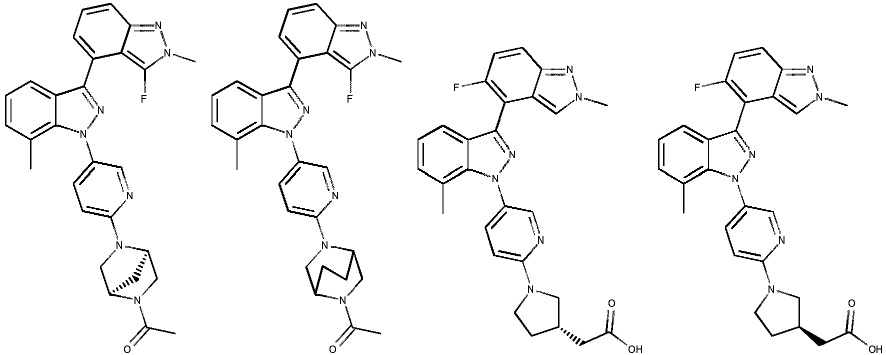 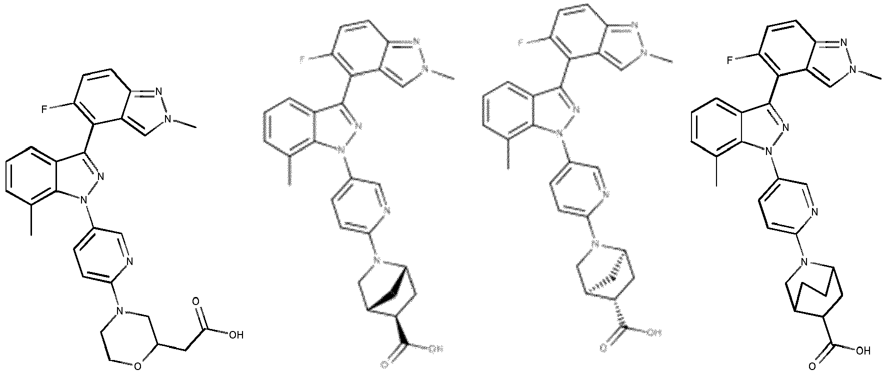 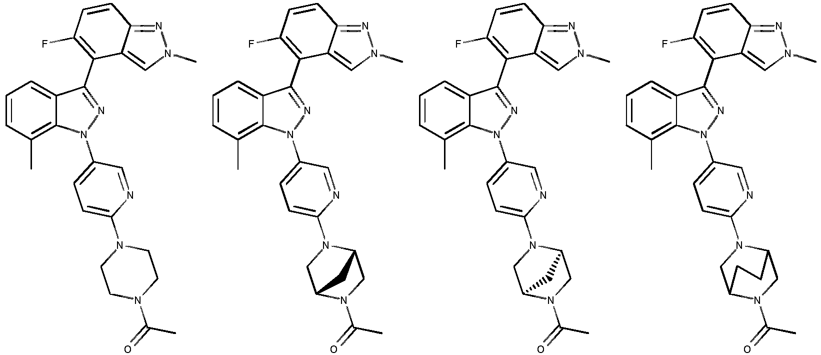 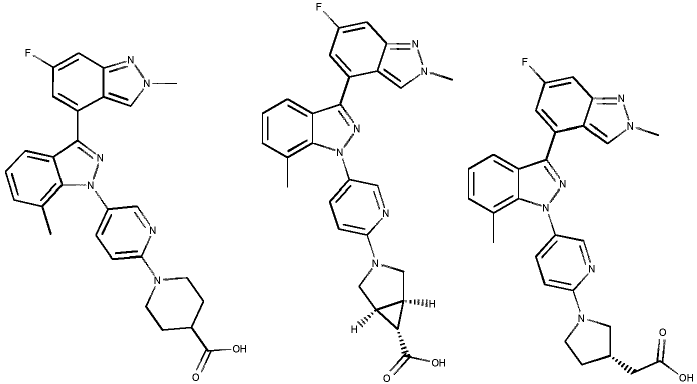 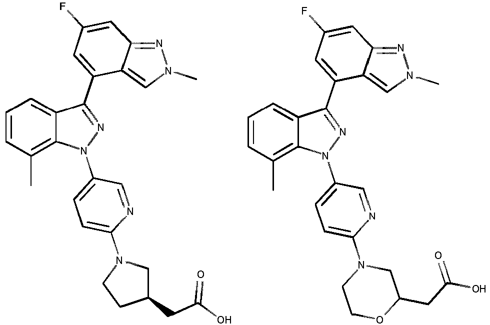 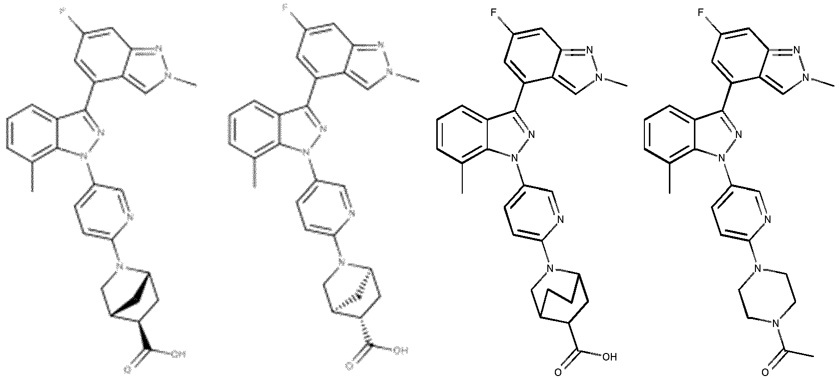 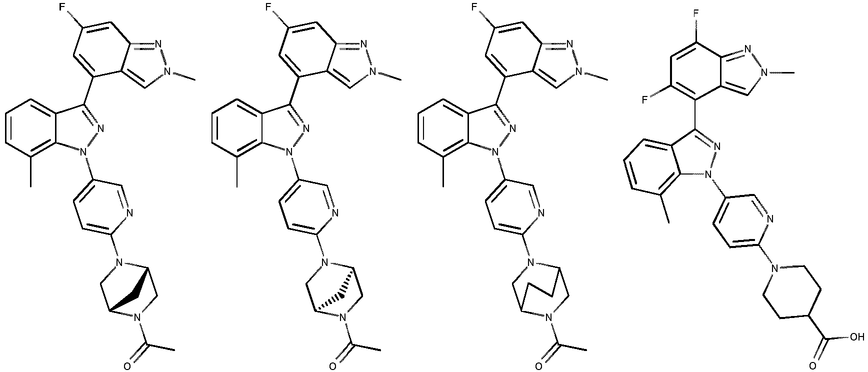 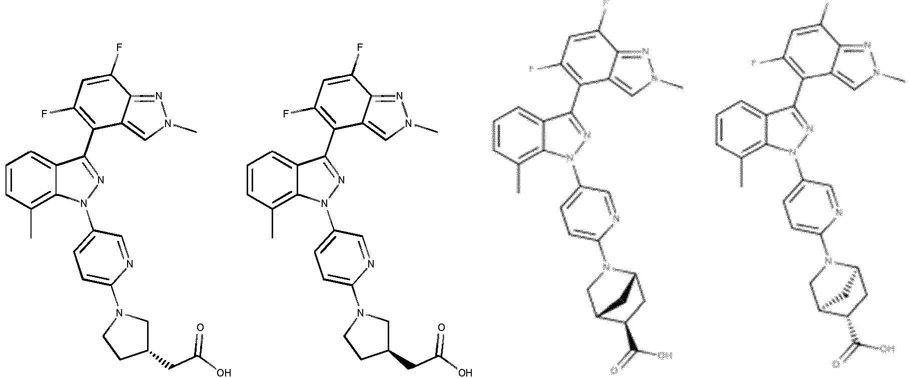 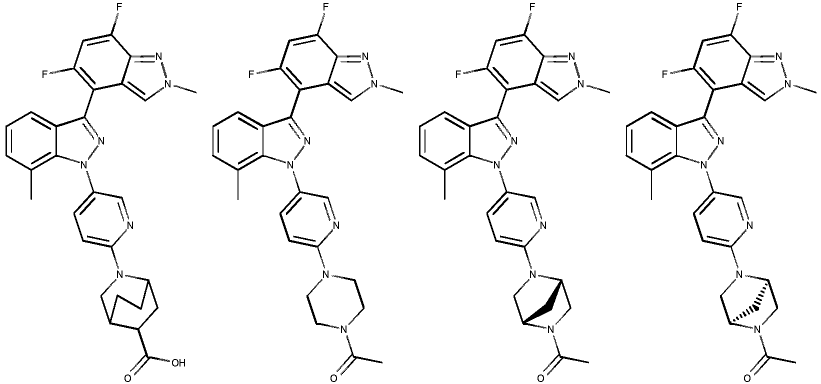 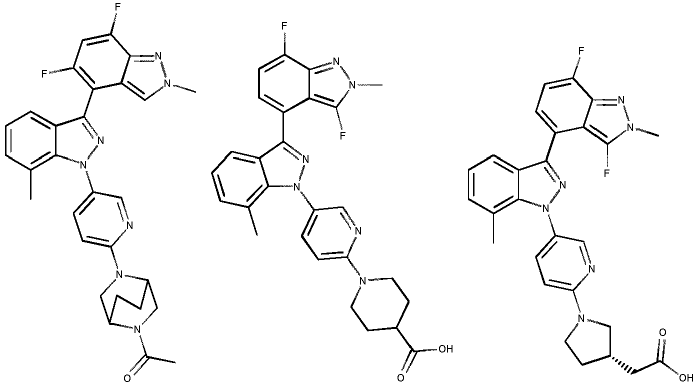 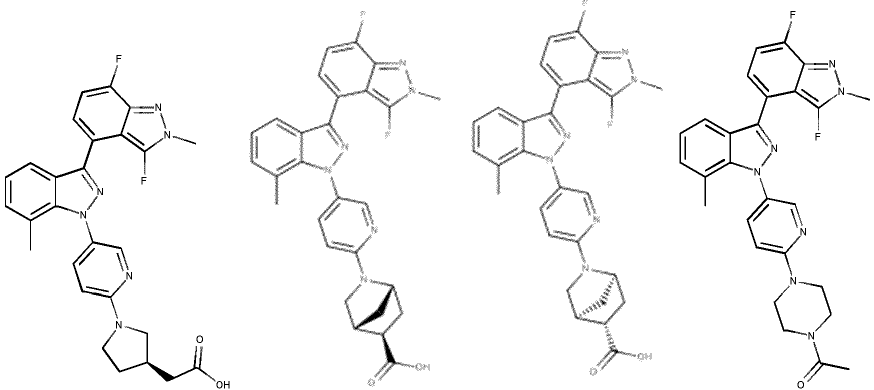 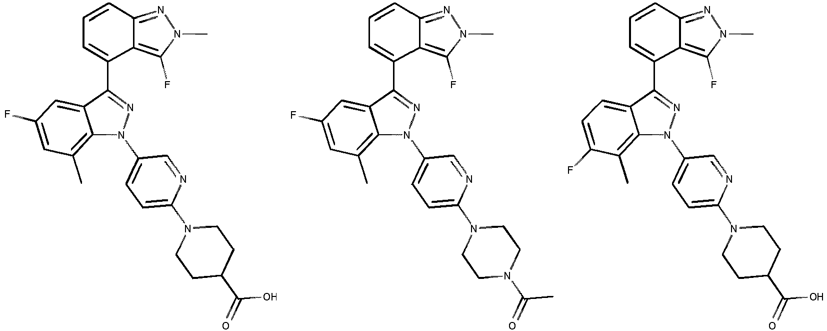 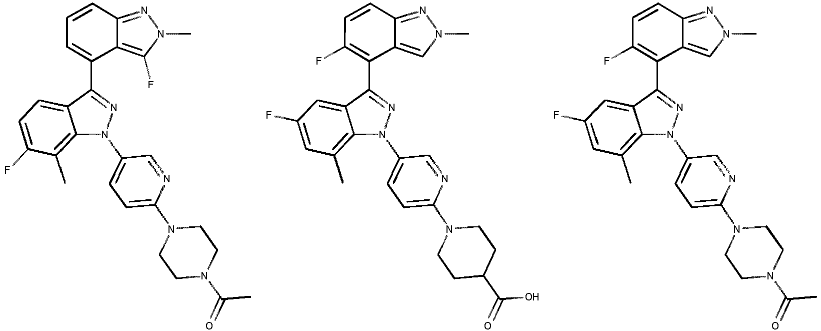 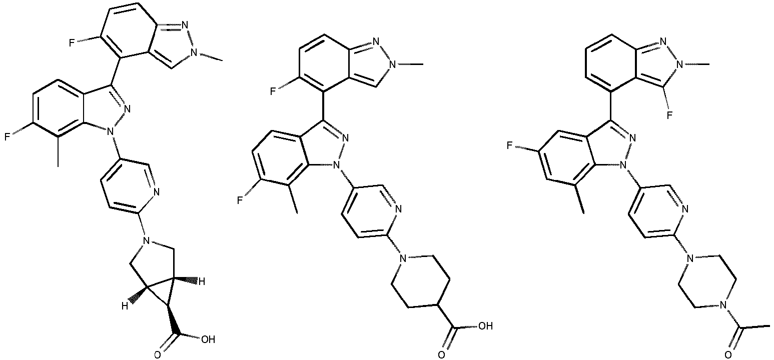 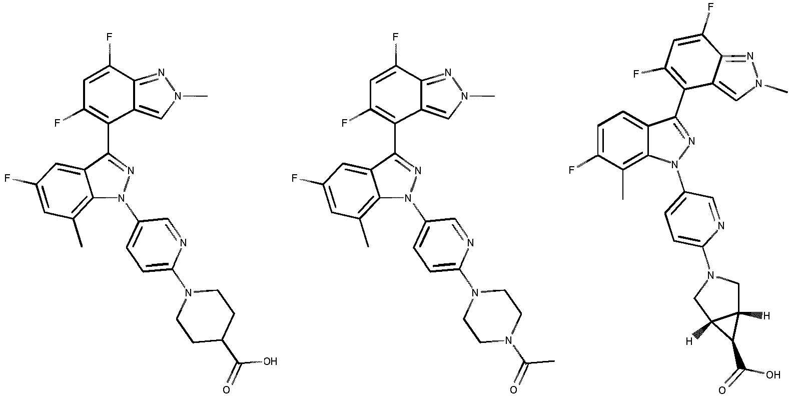 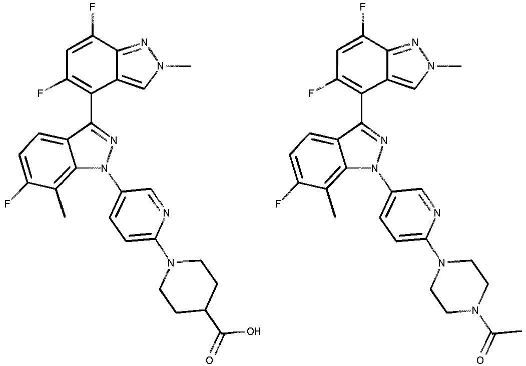 or a pharmaceutically acceptable salt thereof.

11. A pharmaceutical composition comprising at least one compound according to any one of claims 1-10 or a pharmaceutically acceptable salt thereof and a pharmaceutically acceptable carrier.

12. A compound according to any one of claims 1-10 or a pharmaceutically acceptable salt thereof or a pharmaceutical composition according to claim 11 for use as a medicament.

13. A compound according to any one of claims 1-10 or a pharmaceutically acceptable salt thereof or a pharmaceutical composition according to claim 11 for use in the treatment of feline or canine cancer.

14. The compound or a pharmaceutically acceptable salt thereof or the pharmaceutical composition for use according to claim 13, wherein the canine cancer is selected from osteosarcoma (OSA), oral melanoma, B-cell lymphoma, urothelial carcinoma (UC), hemangiosarcoma, mast cell tumor, soft tissue sarcoma, squamous cell carcinoma, T-cell lymphoma, mammary gland adenocarcinoma and anal sac carcinoma or wherein the feline cancer is selected from B-cell and/or T-cell lymphoma, squamous cell carcinoma, mammary gland adenocarcinoma, mast cell tumors and injection site sarcoma.

15. The compound or a pharmaceutically acceptable salt thereof or the pharmaceutical composition for use according to claim 13 or 14, wherein the compound, the pharmaceutically acceptable salt thereof or the pharmaceutical composition is for use in combination with radiotherapy.

## Patentansprüche

1. Verbindung der Formel (I), 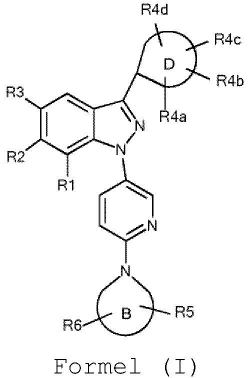 wobei
B für eine Gruppe steht, die ausgewählt ist aus der Gruppe bestehend aus
einem 5- bis 7-gliedrigen monocyclischen Heterocyclyl mit 1 oder 2 N-Atomen,
einem 6-gliedrigen bicyclischen Heterocyclyl mit 1 N-Atom,
einem 7- bis 11-gliedrigen bicyclischen Heterocyclyl mit 1 oder 2 N-Atomen,
einem 7-gliedrigen bicyclischen Heterocyclyl mit 1 N-Atom und 1 O-Atom,
einem 6-gliedrigen monocyclischen Heterocyclyl mit 1 N-Atom und 1 Heteroatom, das aus der Gruppe bestehend aus O und S ausgewählt ist,
einem 9-gliedrigen bicyclischen Heterocyclyl mit 3 Heteroatomen, von denen es sich bei 2 um N und bei dem anderen um O handelt,
einem 9-gliedrigen bicyclischen Heterocyclyl mit 1 N-Atom und 1 S-Atom,
einem 10-gliedrigen bicyclischen Heterocyclyl mit 3 N-Atomen, von denen 2 mit C₁-₆-Alkyl substituiert sind, Phenyl,
einem 9-gliedrigen bicyclischen Heteroaryl mit 3 N-Atomen,
-C₁-₄-Alkylenpyrimidin und
-C₁-₄-Alkylen-O-C₁-₃-alkyl;
D für eine Gruppe steht, die ausgewählt ist aus der Gruppe bestehend aus
einem 9-gliedrigen bicyclischen Heteroaryl mit 2 N-Atomen,
einem 10-gliedrigen bicyclischen Heteroaryl mit 1 N-Atom und
Benzodioxol;
R¹ aus der Gruppe bestehend aus -H oder -C₁-₆-Alkyl ausgewählt ist;
R² aus der Gruppe bestehend aus -H, Halogen, bevorzugt Fluor oder Chlor, besonders bevorzugt Fluor, und -C₁-₆-Alkyl ausgewählt ist;
R³ aus der Gruppe bestehend aus -H, Halogen, bevorzugt Fluor oder Chlor, besonders bevorzugt Fluor, und -C₁-₆-Alkyl ausgewählt ist;
R^{4a}, R^{4b} und R^{4c} jeweils unabhängig ausgewählt sind aus - H,-Halogen, bevorzugt Fluor oder Chlor, besonders bevorzugt Fluor, und C₁₋₆-Alkyl, mit der Maßgabe, dass es sich bei mindestens einem von R^{4a}, R^{4b} und R^{4c} um Halogen handelt;
R^{4d} aus -C₁-₆-Alkyl und C₃-₆-Cycloalkyl ausgewählt ist;
R⁵ fehlt oder ausgewählt ist aus der Gruppe bestehend aus -H, -C₁-₆-Alkyl, -S(O₂)-C₁-₆-Alkyl, -NH-S(O₂)-C₁-₆-Alkyl, =O, -C(O)-C₁-₆-Alkyl, -C(O)H, -C(O)OH, -C(O)NH₂, -C(O)O-C₁-₆-Alkyl, -NR^{5.1}R^{5.2}, -C₁-₆-Alkylen-C(O)OH, -S(O₂)-NH₂, - Pyrolidin-2-on-1-yl, -Tetrazolyl und einem 5-gliedrigen Heteroaryl mit 1 oder 2 Heteroatomen, die ausgewählt sind aus der Gruppe bestehend aus N und O, das mit R^{5.3} substituiert ist;
R^{5.1} ausgewählt ist aus der Gruppe bestehend aus -H, -C₁-₆-Alkyl, -C(O)-C₁-6-Alkyl und -C₁-₆-Alkylen-O-C₁-₆-alkyl; R^{5.2} ausgewählt ist aus der Gruppe bestehend aus -H, -C₁-₆-Alkyl, -C(O)-C₁-₆-Alkyl und -C₁-₆-Alkylen-O-C₁-₆-alkyl;
R^{5.3} ausgewählt ist aus der Gruppe bestehend aus -H, -C₁-₆-Alkyl und einem 6-gliedrigen Heteroaryl mit 1 oder 2 Heteroatomen, die aus einer Gruppe bestehend aus N und O ausgewählt sind;
R⁶ fehlt oder ausgewählt ist aus der Gruppe bestehend aus -H, -C₁-₆-Alkyl, =O und -C(O)OH;
oder ein pharmazeutisch annehmbares Salz davon.

2. Verbindung nach Anspruch 1, wobei R¹ für -C₁-₆-Alkyl steht; und
R^{4a} und/oder R^{4b} und/oder R^{4c} für Fluor oder Chlor steht und die jeweils anderen für -H oder -C₁-₃-Alkyl stehen;
und R^{4d} für -C₁-₆-Alkyl steht;
oder ein pharmazeutisch annehmbares Salz davon.

3. Verbindung nach einem der Ansprüche 1-2, wobei D ausgewählt ist aus der Gruppe bestehend aus 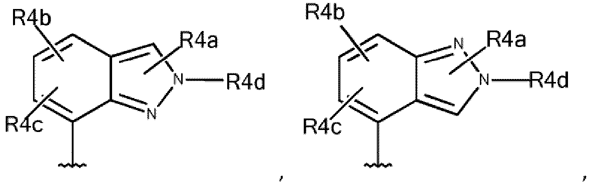 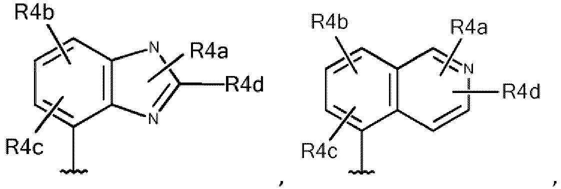 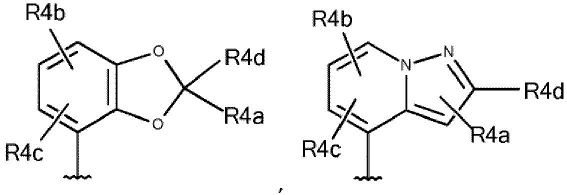 und 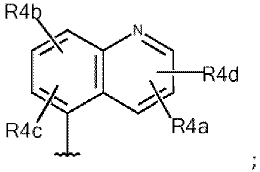 wobei
R^{4d} für -O-C₁-₆-Alkyl steht;
oder ein pharmazeutisch annehmbares Salz davon.

4. Verbindung nach einem der Ansprüche 1-3, wobei D für 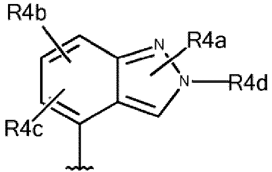 steht, oder ein pharmazeutisch annehmbares Salz davon.

5. Verbindung nach einem der Ansprüche 1-4, wobei D ausgewählt ist aus der Gruppe bestehend aus den folgenden Strukturen: 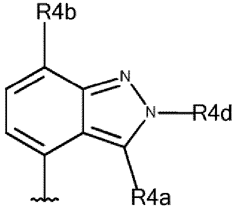 wobei
R^{4a} für -H steht;
R^{4b} für Halogen, bevorzugt Chlor oder Fluor, besonders bevorzugt Fluor, steht; und
R^{4d} für Methyl steht;
oder
R^{4a} für Halogen, bevorzugt Chlor oder Fluor, besonders bevorzugt Fluor, steht;
R^{4b} für -H steht; und
R^{4d} für Methyl steht;
oder
R^{4a} für Halogen, bevorzugt Chlor oder Fluor, besonders bevorzugt Fluor, steht;
R^{4b} für Halogen, bevorzugt Chlor oder Fluor, besonders bevorzugt Fluor, steht; und
R^{4d} für Methyl steht;
oder 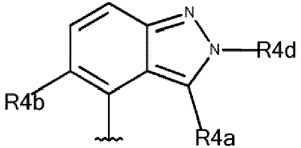 wobei
R^{4a} für -H steht;
R^{4b} für Halogen, bevorzugt Chlor oder Fluor, besonders bevorzugt Fluor, steht; und
R^{4d} für Methyl steht
oder
R^{4a} für Halogen, bevorzugt Chlor oder Fluor, besonders bevorzugt Fluor, steht;
R^{4b} für -H steht; und
R^{4d} für Methyl steht;
oder
R^{4a} für Halogen, bevorzugt Chlor oder Fluor, besonders bevorzugt Fluor, steht;
R^{4b} für Halogen, bevorzugt Chlor oder Fluor, besonders bevorzugt Fluor, steht; und
R^{4d} für Methyl steht;
oder 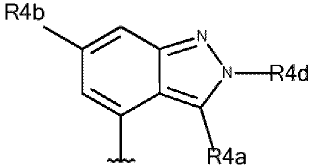 wobei
R^{4a} für -H steht;
R^{4b} für Halogen, bevorzugt Chlor oder Fluor, besonders bevorzugt Fluor, steht; und
R^{4d} für Methyl steht;
oder
R^{4a} für Halogen, bevorzugt Chlor oder Fluor, besonders bevorzugt Fluor, steht;
R^{4b} für -H steht; und
R^{4d} für Methyl steht;
oder
R^{4a} für Halogen, bevorzugt Chlor oder Fluor, besonders bevorzugt Fluor, steht;
R^{4b} für Halogen, bevorzugt Chlor oder Fluor, besonders bevorzugt Fluor, steht; und
R^{4d} für -Methyl steht;
oder 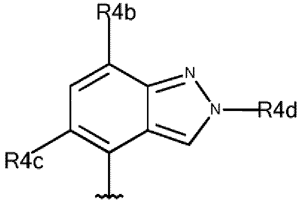 wobei
R^{4b} für -H oder Methyl steht;
R^{4c} für Halogen, bevorzugt Chlor oder Fluor, besonders bevorzugt Fluor, steht; und
R^{4d} für Methyl steht;
oder
R^{4b} für Halogen, bevorzugt Chlor oder Fluor, besonders bevorzugt Fluor, steht;
R^{4c} für -H oder Methyl steht; und
R^{4d} für Methyl steht;
oder
R^{4b} für Halogen, bevorzugt Chlor oder Fluor, besonders bevorzugt Fluor, steht;
R^{4c} für Halogen, bevorzugt Chlor oder Fluor, besonders bevorzugt Fluor, steht; und
R^{4d} für -Methyl steht;
oder ein pharmazeutisch annehmbares Salz davon.

6. Verbindung nach einem der Ansprüche 1-5, wobei
R¹ für Methyl, R² für -H oder Halogen und R³ für -H oder Halogen steht;
oder ein pharmazeutisch annehmbares Salz davon.

7. Verbindung nach einem der Ansprüche 1-6, wobei
R¹ für Methyl, R² für -H und R³ für -H steht;
oder
R¹ für Methyl, R² für -H und R³ für Fluor steht;
oder
R¹ für Methyl, R² für Fluor und R³ für -H steht;
oder ein pharmazeutisch annehmbares Salz davon.

8. Verbindung nach einem der Ansprüche 1-7, wobei B ausgewählt ist aus der Gruppe bestehend aus 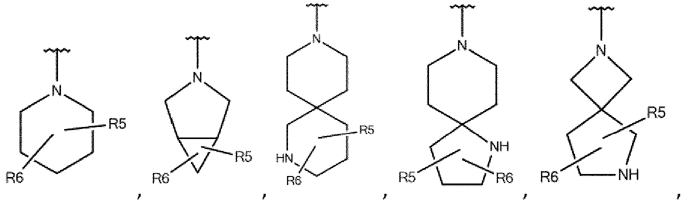 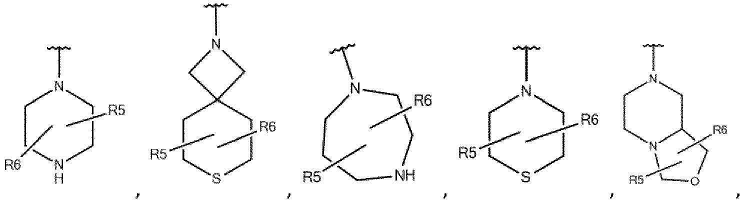 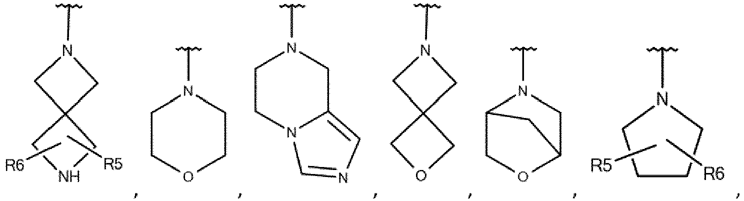 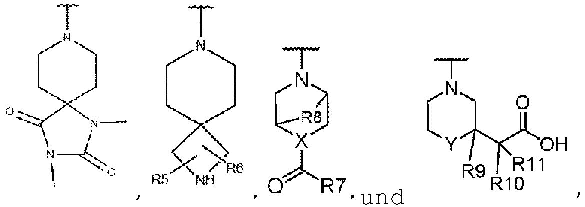
wobei R⁷ aus H, -C₁-₆-Alkyl, -C₃-₆-Cycloalkyl und -OH ausgewählt ist;
R⁸ für (CH₂)ₙ steht, wobei n für eine ganze Zahl von 1-3, bevorzugt 1 oder 2, steht;
R⁹ ausgewählt ist aus der Gruppe bestehend aus H, -C₁-₆-Alkyl und -C₃-₆-Cycloalkyl;
R¹⁰ ausgewählt ist aus der Gruppe bestehend aus H, -C₁-₆-Alkyl und -C₃-₆-Cycloalkyl;
R¹¹ ausgewählt ist aus der Gruppe bestehend aus H, -C₁-₆-Alkyl und -C₃-₆-Cycloalkyl;
X für CH oder N steht; und
Y für -O-, -S-, -S(O)-, -S(O)₂- steht;
oder ein pharmazeutisch annehmbares Salz davon.

9. Verbindung nach einem der Ansprüche 1-8, wobei B ausgewählt ist aus der Gruppe bestehend aus 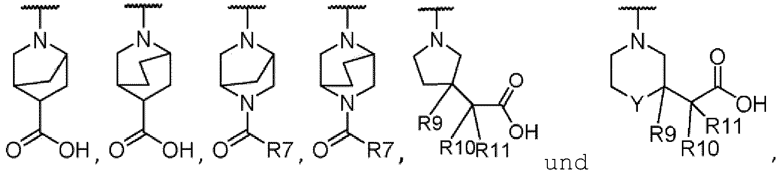
wobei R⁷ ausgewählt ist aus der Gruppe bestehend aus -H und -C₁-₆-Alkyl;
R⁹ ausgewählt ist aus der Gruppe bestehend aus -H und Methyl und bevorzugt für -H steht;
R¹⁰ ausgewählt ist aus der Gruppe bestehend aus -H und Methyl und bevorzugt für -H steht;
R¹¹ ausgewählt ist aus der Gruppe bestehend aus -H und Methyl und bevorzugt für -H steht;
und
Y für O steht;
oder ein pharmazeutisch annehmbares Salz davon.

10. Verbindung nach Anspruch 1, die aus der Gruppe ausgewählt ist, die aus den folgenden Strukturen besteht: 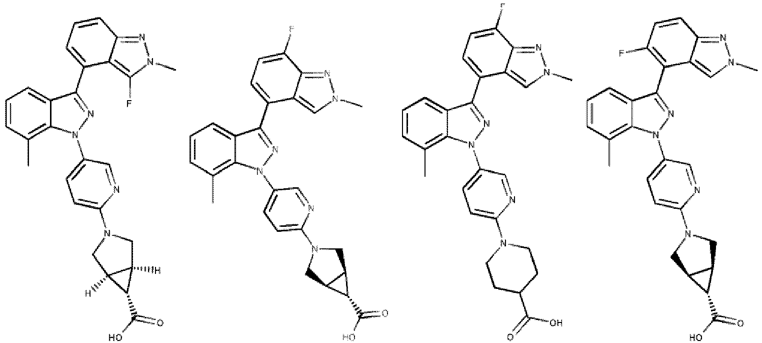 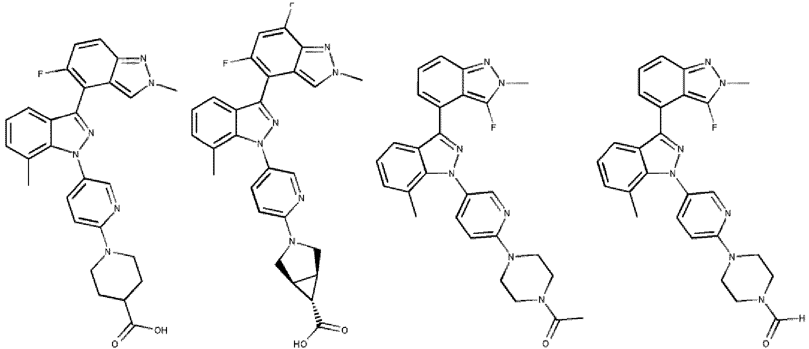 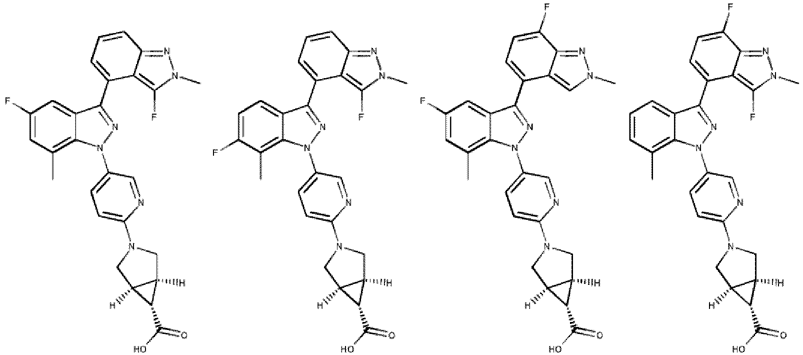 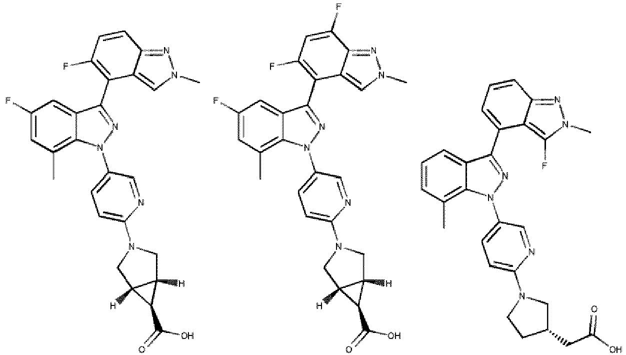 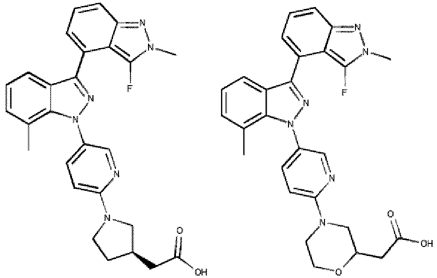 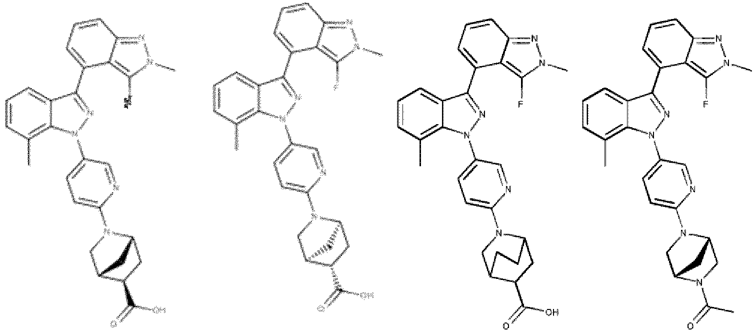 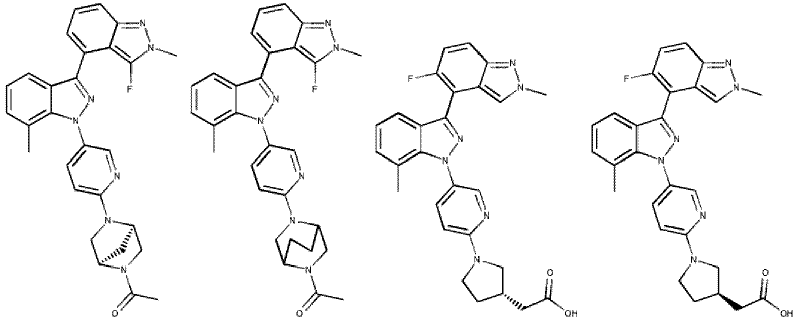 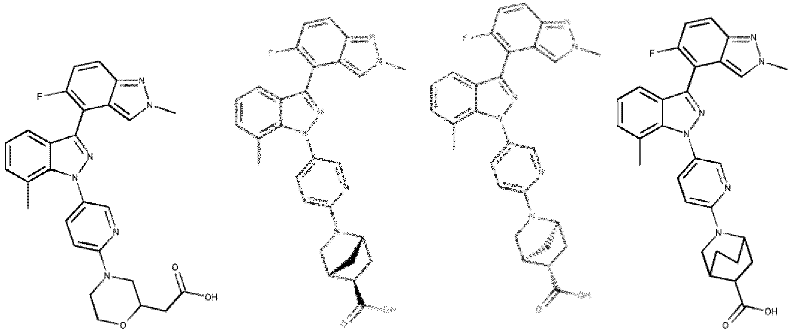 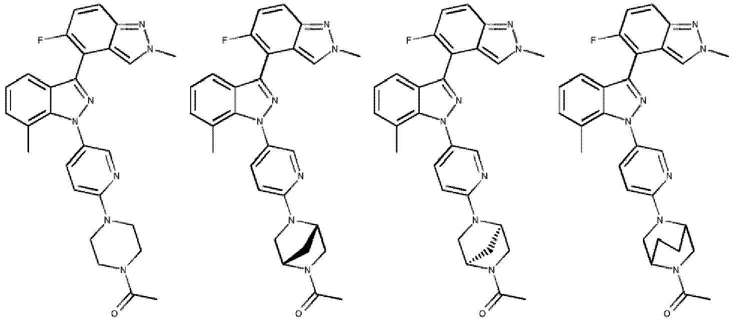 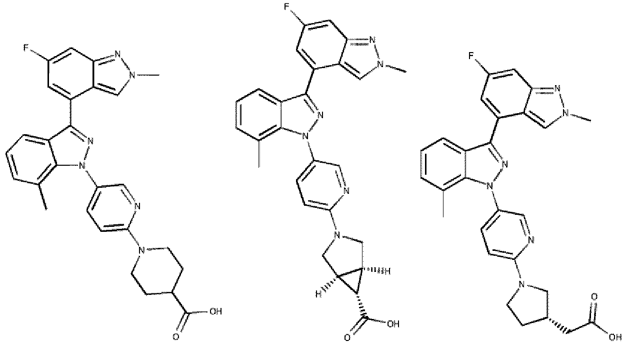 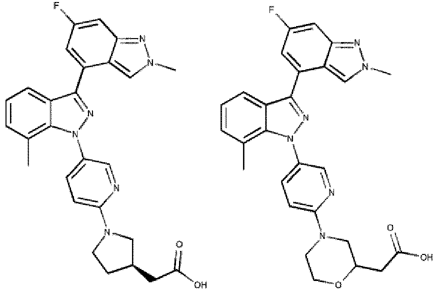 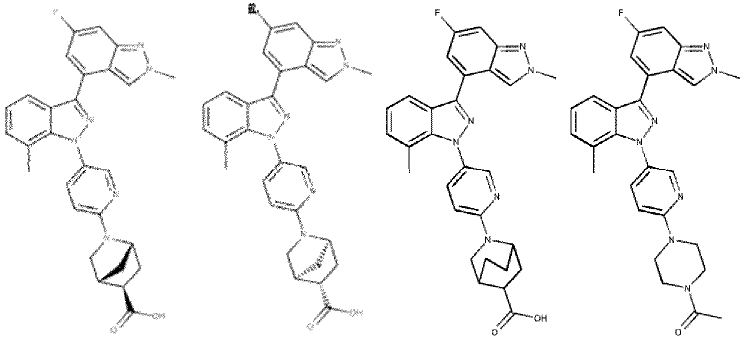 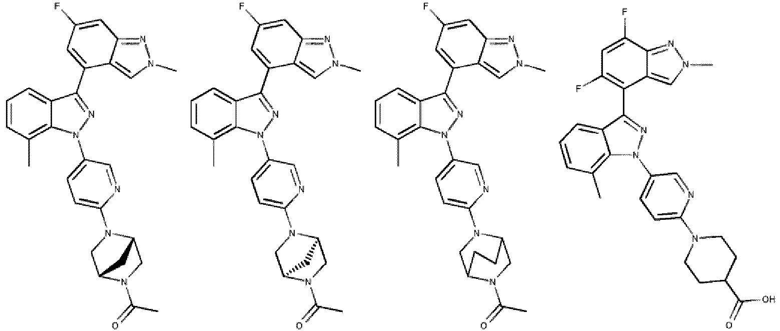 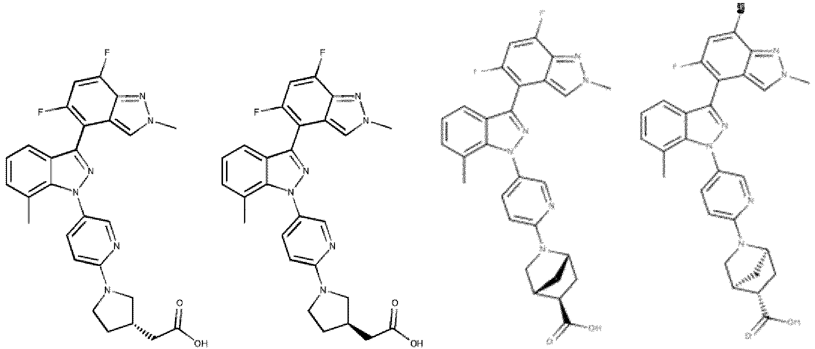 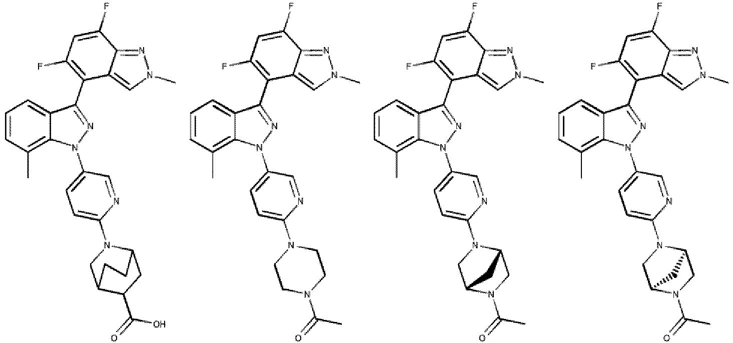 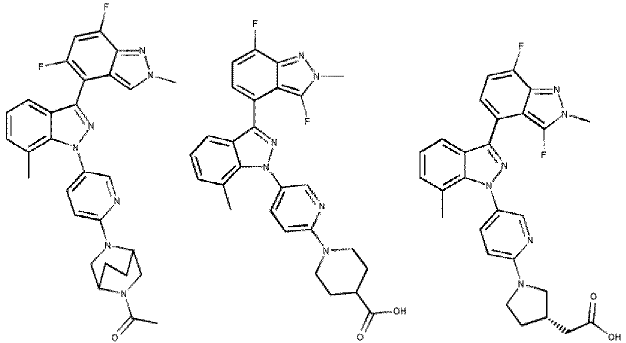 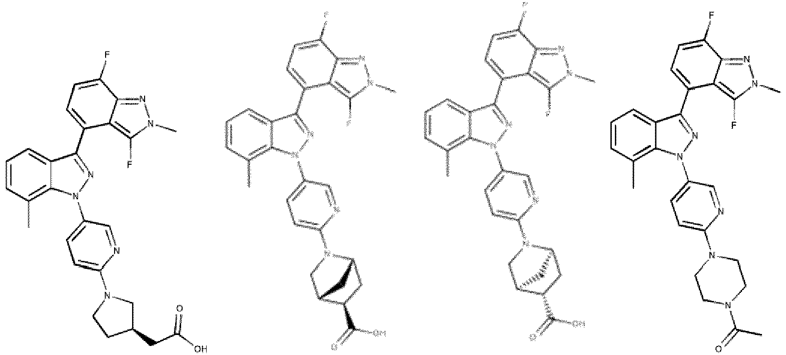 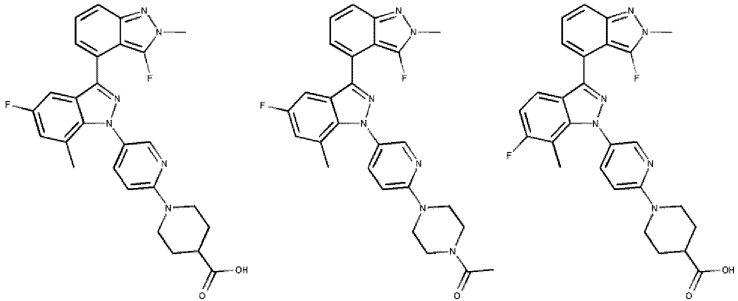 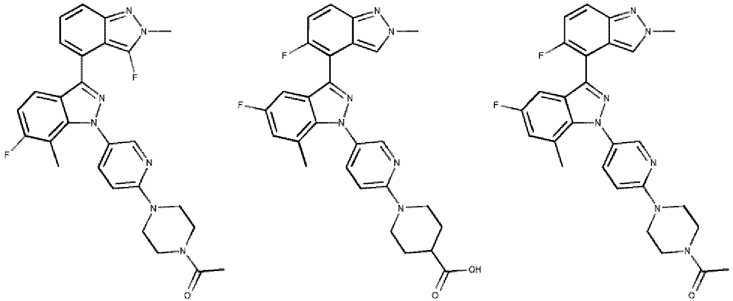 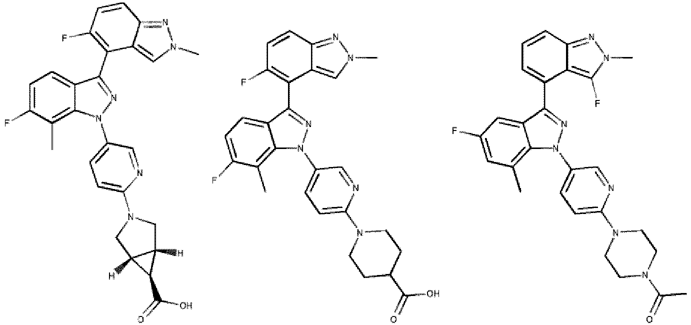 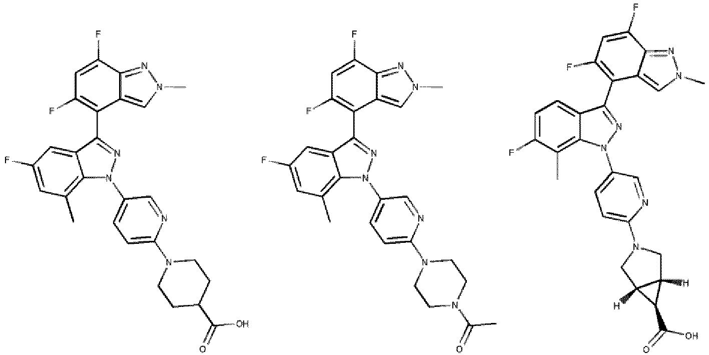 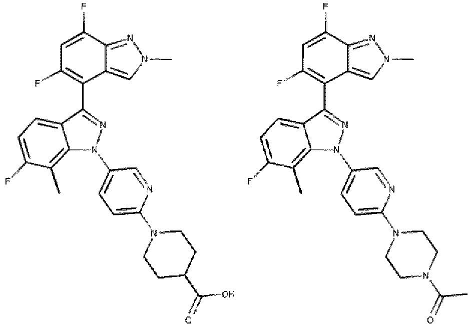 oder ein pharmazeutisch annehmbares Salz davon.

11. Pharmazeutische Zusammensetzung, umfassend mindestens eine Verbindung nach einem der Ansprüche 1-10 oder ein pharmazeutisch annehmbares Salz davon und einen pharmazeutisch annehmbaren Träger.

12. Verbindung nach einem der Ansprüche 1-10 oder ein pharmazeutisch annehmbares Salz davon oder pharmazeutische Zusammensetzung nach Anspruch 11 zur Verwendung als Arzneimittel.

13. Verbindung nach einem der Ansprüche 1-10 oder ein pharmazeutisch annehmbares Salz davon oder pharmazeutische Zusammensetzung nach Anspruch 11 zur Verwendung bei der Behandlung von Krebserkrankungen bei Katzen oder Hunden.

14. Verbindung oder ein pharmazeutisch annehmbares Salz davon oder pharmazeutische Zusammensetzung zur Verwendung nach Anspruch 13, wobei die Krebserkrankung bei dem Hund ausgewählt ist aus Osteosarkom (OSA), oralem Melanom, B-Zell-Lymphom, Urothelkarzinom (UC), Hämangiosarkom, Mastzelltumor, Weichteilsarkom, Plattenepithelkarzinom, T-Zell-Lymphom, Brustdrüsenadenokarzinom und Analbeutelkarzinom oder wobei die Krebserkrankung bei der Katze ausgewählt ist aus B-Zell- und/oder T-Zell-Lymphom, Plattenepithelkarzinom, Brustdrüsenadenokarzinom, Mastzelltumoren und Injektionsstellensarkom.

15. Verbindung oder ein pharmazeutisch annehmbares Salz davon oder pharmazeutische Zusammensetzung zur Verwendung nach Anspruch 13 oder 14, wobei die Verbindung, das pharmazeutisch annehmbare Salz davon oder die pharmazeutische Zusammensetzung zur Verwendung in Kombination mit Strahlentherapie bestimmt ist.

## Revendications

1. Composé de formule (I) 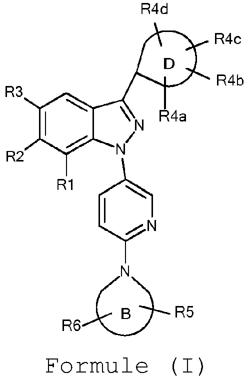
B étant un groupe choisi dans le groupe constitué par un hétérocyclyle monocyclique à 5 à 7 chaînons contenant 1 ou 2 atomes de N,
un hétérocyclyle bicyclique à 6 chaînons contenant 1 atome de N,
un hétérocyclyle bicyclique à 7 à 11 chaînons contenant 1 ou 2 atomes de N,
un hétérocyclyle bicyclique à 7 chaînons contenant 1 atome de N et 1 atome d'O,
un hétérocyclyle monocyclique à 6 chaînons contenant 1 atome de N et 1 hétéroatome choisi dans le groupe constitué par O et S,
un hétérocyclyle bicyclique à 9 chaînons contenant 3 hétéroatomes, 2 desquels étant N et l'autre étant O,
un hétérocyclyle bicyclique à 9 chaînons contenant 1 atome de N et 1 atome de S,
un hétérocyclyle bicyclique à 10 chaînons contenant 3 atomes de N, 2 desquels étant substitués par C₁-₆-alkyle, phényle,
un hétéroaryle bicyclique à 9 chaînons contenant 3 atomes de N,
-C₁-₄-alkylène-pyrimidine, et
-C₁-₄-alkylène-O-C₁-₃-alkyle ;
D étant un groupe choisi dans le groupe constitué par un hétéroaryle bicyclique à 9 chaînons contenant 2 atomes de N,
un hétéroaryle bicyclique à 10 chaînons contenant 1 atome de N, et
benzodioxole ;
R¹ étant choisi dans le groupe constitué par -H ou -C₁-₆-alkyle ;
R² étant choisi dans le groupe constitué par -H, halogène, préférablement fluor ou chlore, plus préférablement fluor, et -C₁-₆-alkyle ;
R³ étant choisi dans le groupe constitué par -H, halogène, préférablement fluor ou chlore, plus préférablement fluor, et -C₁-₆-alkyle ;
R^{4a}, R^{4b} et R^{4c} étant chacun indépendamment choisis parmi -H, halogène, préférablement fluor ou chlore, plus préférablement fluor, et C₁₋₆-alkyle, à la condition qu'au moins l'un parmi R^{4a}, R^{4b} et R^{4c} soit halogène ;
R^{4d} étant choisi parmi -C₁-₆-alkyle et C₃-₆ cycloalkyle ;
R⁵ étant absent ou étant choisi dans le groupe constitué par -H, -C₁-₆-alkyle, -S(O₂)-C₁-₆-alkyle, -NH-S(O₂)-C₁-₆-alkyle, =O, -C(O)-C₁-₆-alkyle, -C(O)H, -C(O)OH, -C(O)NH₂, -C(O)O-C₁-₆-alkyle, -NR^{5.1}R^{5.2}, -C₁-₆-alkylène-C(O)OH, - S(O₂)-NH₂, -pyrolidin-2-one-1-yle, -tétrazolyle, et un hétéroaryle à 5 chaînons comportant 1 ou 2 hétéroatomes choisis dans le groupe constitué par N et O qui est substitué par R^{5.3} ;
R^{5.1} étant choisi dans le groupe constitué par -H, -C₁-₆-alkyle, -C(O)-C₁-₆-alkyle et -C₁-₆-alkylène-O-C₁-₆-alkyle;
R^{5.2} étant choisi dans le groupe constitué par -H, -C₁-₆-alkyle, -C(O)-C₁-₆-alkyle et -C₁-₆-alkylène-O-C₁-₆-alkyle;
R^{5.3} étant choisi dans le groupe constitué par -H, -C₁-₆-alkyle et un hétéroaryle à 6 chaînons comportant 1 ou 2 hétéroatomes choisis dans le groupe constitué par N et O;
R⁶ étant absent ou étant choisi dans le groupe constitué par -H, -C₁-₆-alkyle, =O et -C(O)OH ;
ou sel pharmaceutiquement acceptable correspondant.

2. Composé selon la revendication 1, R¹ étant -C₁-₆-alkyle ; et
au moins l'un parmi R^{4a}, R^{4b} et R^{4c} étant fluor ou chlore, et les autres étant -H ou -C₁-₃-alkyle ; et R^{4d} étant -C₁-₆-alkyle ;
ou sel pharmaceutiquement acceptable correspondant.

3. Composé selon l'une quelconque des revendications 1-2,
D étant choisi dans le groupe constitué par 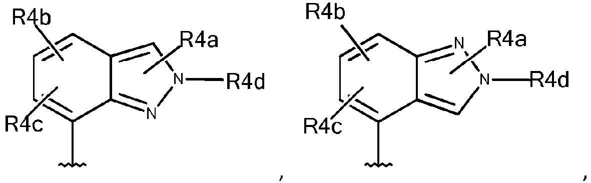 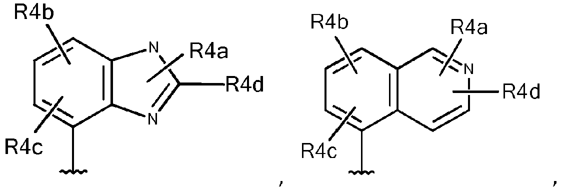 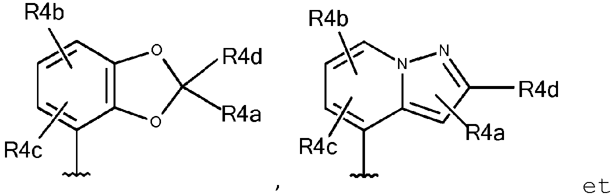 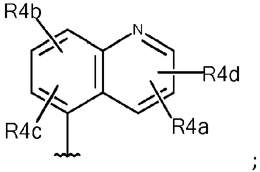
R^{4d} étant C₁₋₆-alkyle ;
ou sel pharmaceutiquement acceptable correspondant.

4. Composé selon l'une quelconque des revendications 1-3, D étant 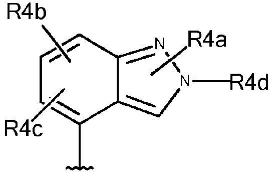 ou sel pharmaceutiquement acceptable correspondant.

5. Composé selon l'une quelconque des revendications 1-4, D étant choisi dans le groupe constitué par les structures suivantes : 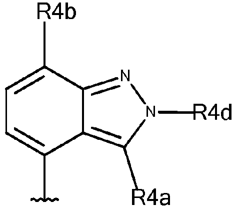
R^{4a} étant -H ;
R^{4b} étant halogène, préférablement chlore ou fluor, plus préférablement fluor ; et
R^{4d} étant méthyle ;
ou
R^{4a} étant halogène, préférablement chlore ou fluor, plus préférablement fluor ;
R^{4b} étant -H ; et
R^{4d} étant méthyle ;
ou
R^{4a} étant halogène, préférablement chlore ou fluor, plus préférablement fluor ;
R^{4b} étant halogène, préférablement chlore ou fluor, plus préférablement fluor ; et
R^{4d} étant méthyle ;
ou 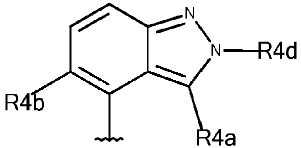
R^{4a} étant -H ;
R^{4b} étant halogène, préférablement chlore ou fluor, plus préférablement fluor ; et
R^{4d} étant méthyle
ou
R^{4a} étant halogène, préférablement chlore ou fluor, plus préférablement fluor ;
R^{4b} étant -H ; et
R^{4d} étant méthyle ;
ou
R^{4a} étant halogène, préférablement chlore ou fluor, plus préférablement fluor ;
R^{4b} étant halogène, préférablement chlore ou fluor, plus préférablement fluor ; et
R^{4d} étant méthyle ;
ou 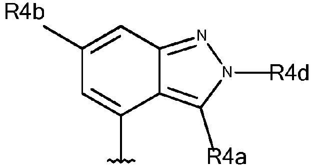
R^{4a} étant -H ;
R^{4b} étant halogène, préférablement chlore ou fluor, plus préférablement fluor ; et
R^{4d} étant méthyle ;
ou
R^{4a} étant halogène, préférablement chlore ou fluor, plus préférablement fluor ;
R^{4b} étant -H ; et
R^{4d} étant méthyle ;
ou
R^{4a} étant halogène, préférablement chlore ou fluor, plus préférablement fluor ;
R^{4b} étant halogène, préférablement chlore ou fluor, plus préférablement fluor ; et
R^{4d} étant -méthyle ;
ou 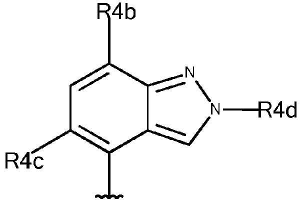
R^{4b} étant -H ou méthyle ;
R^{4c} étant halogène, préférablement chlore ou fluor, plus préférablement fluor ; et
R^{4d} étant méthyle ;
ou
R^{4b} étant halogène, préférablement chlore ou fluor, plus préférablement fluor ;
R^{4c} étant -H ou méthyle ; et
R^{4d} étant méthyle ;
ou
R^{4b} étant halogène, préférablement chlore ou fluor, plus préférablement fluor ;
R^{4c} étant halogène, préférablement chlore ou fluor, plus préférablement fluor ; et
R^{4d} étant -méthyle ;
ou sel pharmaceutiquement acceptable correspondant.

6. Composé selon l'une quelconque des revendications 1-5,
R¹ étant méthyle ; R² étant -H ou halogène ; et R³ -H ou halogène ;
ou sel pharmaceutiquement acceptable correspondant.

7. Composé selon l'une quelconque des revendications 1-6,
R¹ étant méthyle, R² étant -H ; et R³ étant -H ;
ou
R¹ étant méthyle, R² étant -H ; et R³ étant fluor ;
ou
R¹ étant méthyle, R² étant fluor ; et R³ étant -H ;
ou sel pharmaceutiquement acceptable correspondant.

8. Composé selon l'une quelconque des revendications 1-7, B étant choisi dans le groupe constitué par 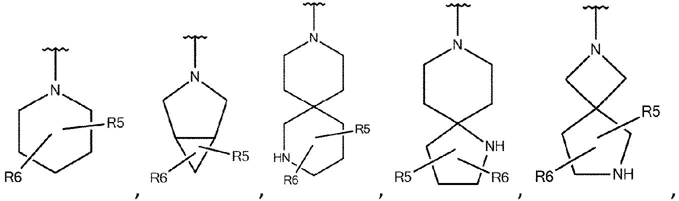 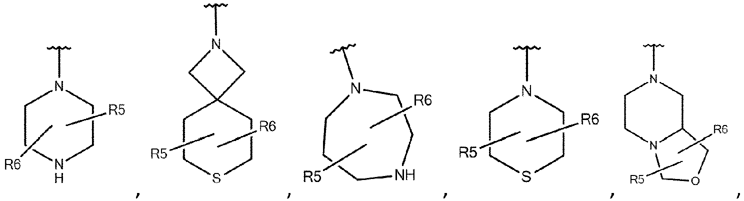 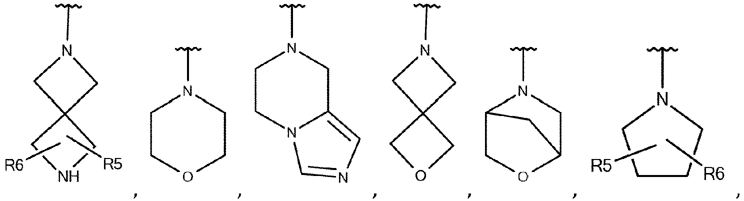 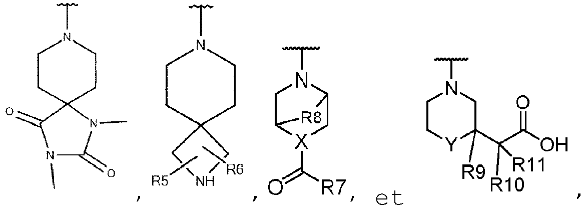
R⁷ étant choisi parmi H, -C₁-₆-alkyle, -C₃-₆-cycloalkyle et -OH ;
R⁸ étant (CH₂)ₙ, n étant un entier parmi 1-3, préférablement 1 ou 2 ;
R⁹ étant choisi dans le groupe constitué par H, -C₁-₆-alkyle, et -C₃-₆-cycloalkyle ;
R¹⁰ étant choisi dans le groupe constitué par H, -C₁-₆-alkyle, et -C₃-₆-cycloalkyle ;
R¹¹ étant choisi dans le groupe constitué par H, -C₁-₆-alkyle, et -C₃-₆-cycloalkyle ;
X étant CH ou N ; et
Y étant -O-, -S-, -S(O)-, -S(O)₂- ;
ou sel pharmaceutiquement acceptable correspondant.

9. Composé selon l'une quelconque des revendications 1-8, B étant choisi dans le groupe constitué par 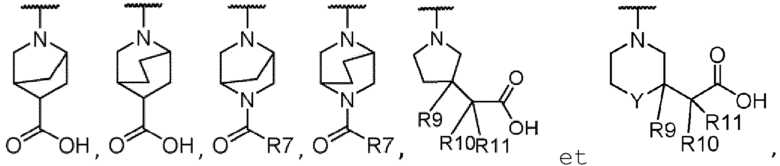
R⁷ étant choisi dans le groupe constitué par -H et -C₁-₆-alkyle ;
R⁹ étant choisi dans le groupe constitué par -H et méthyle, et étant préférablement -H ;
R¹⁰ étant choisi dans le groupe constitué par -H et méthyle, et étant préférablement -H ;
R¹¹ étant choisi dans le groupe constitué par -H et méthyle, et étant préférablement -H ;
et
Y étant O ;
ou sel pharmaceutiquement acceptable correspondant.

10. Composé selon la revendication 1, qui est choisi dans le groupe constitué par les structures suivantes : 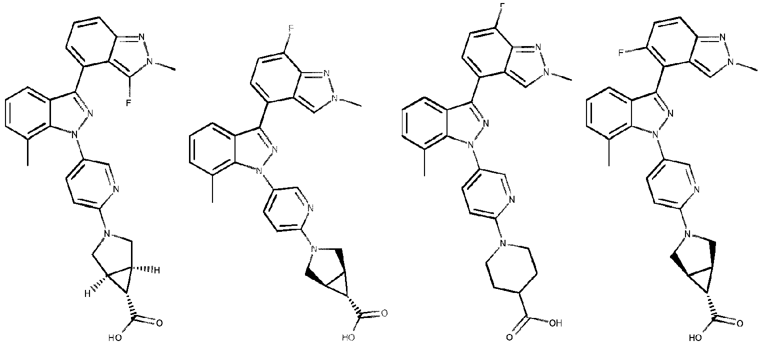 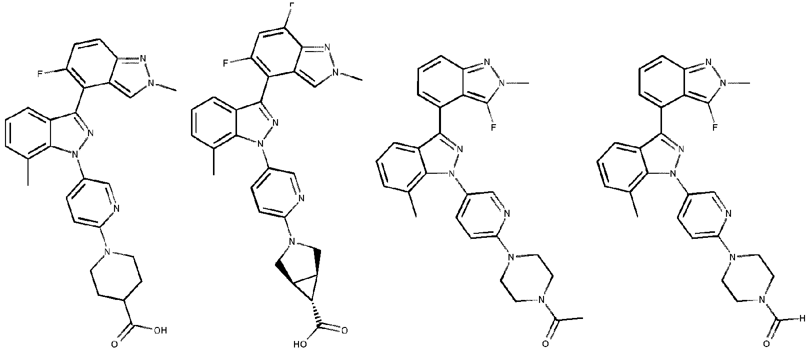 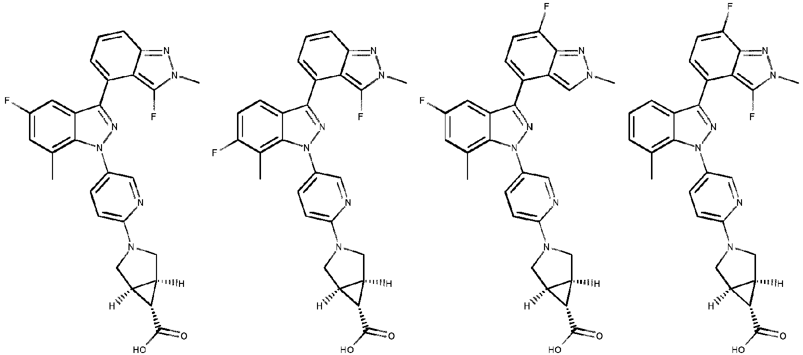 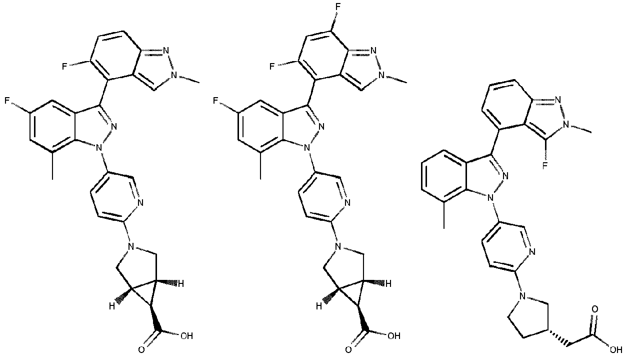 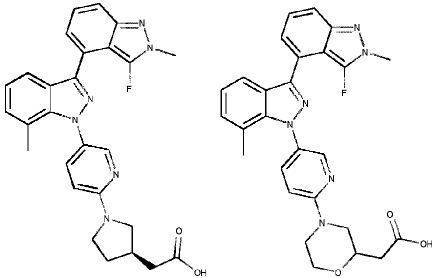 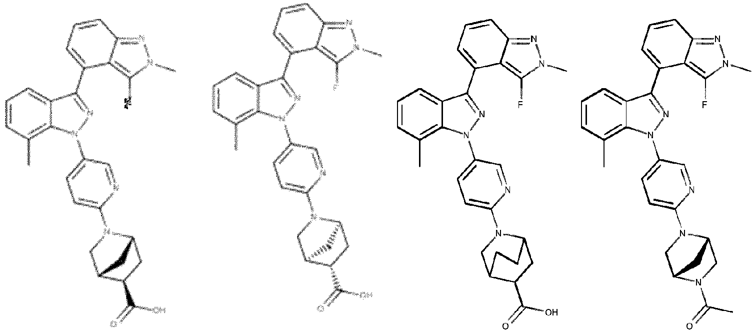 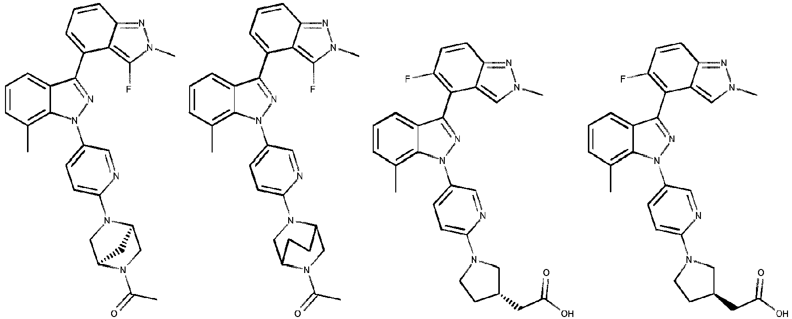 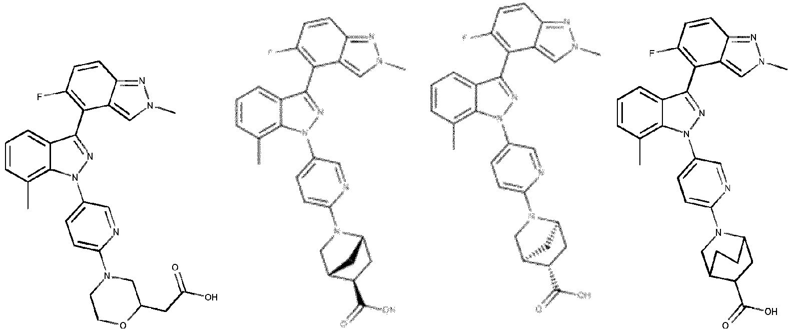 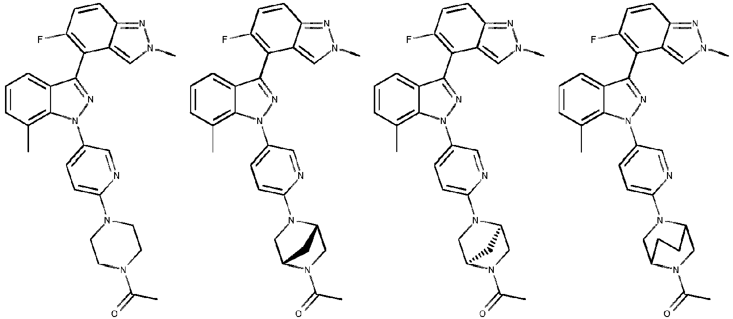 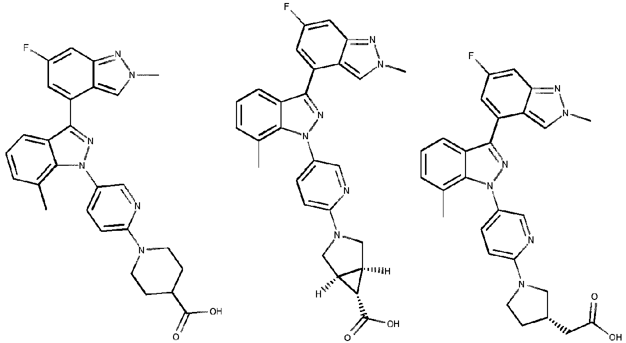 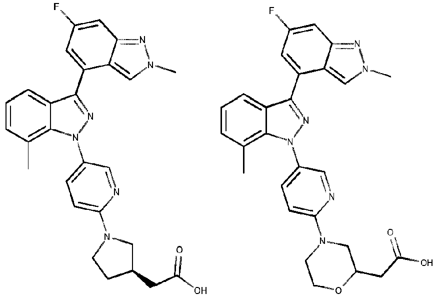 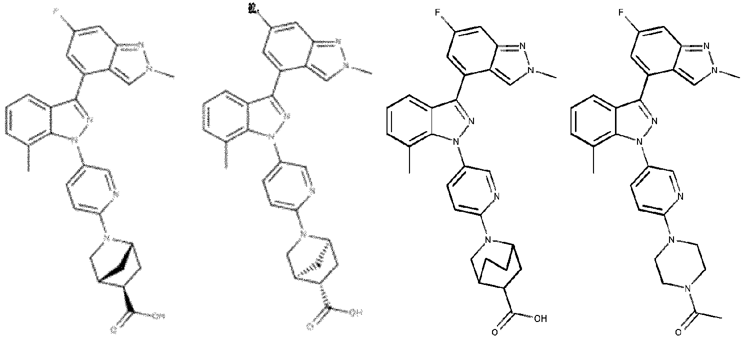 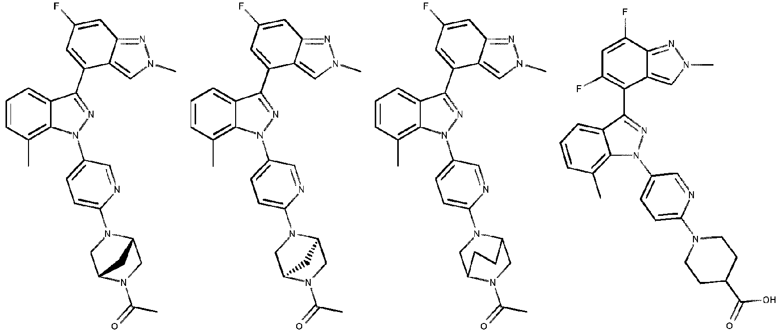 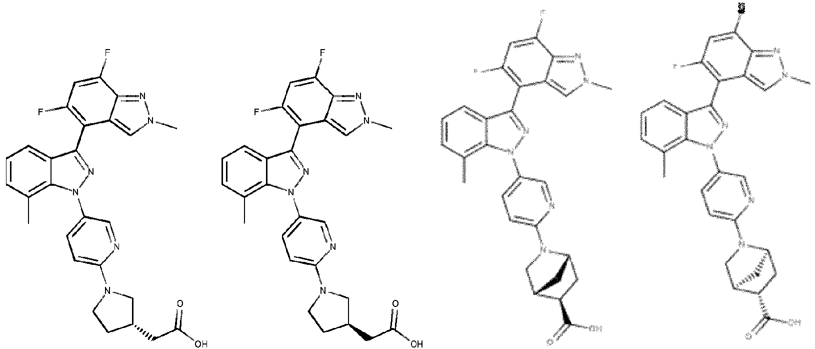 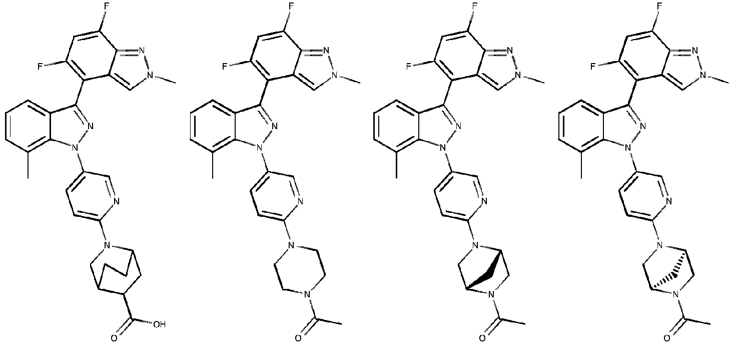 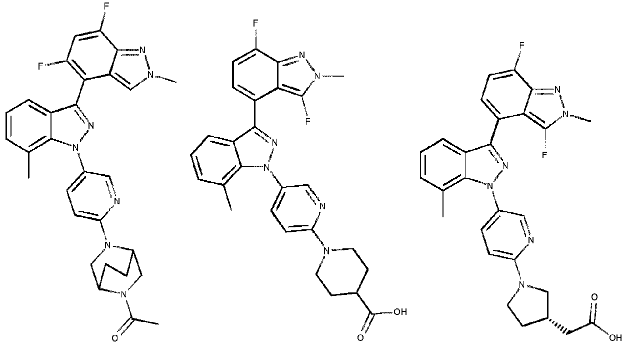 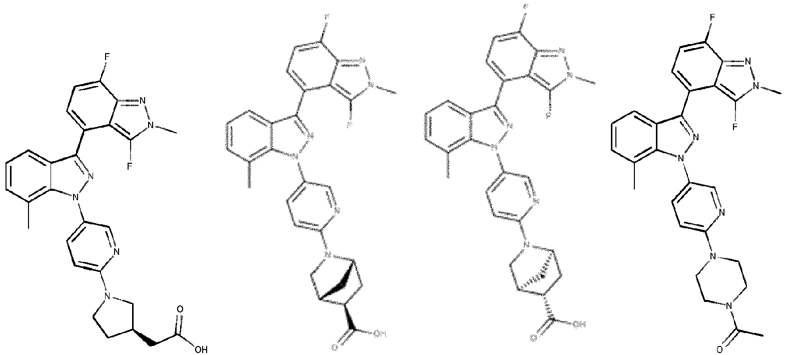 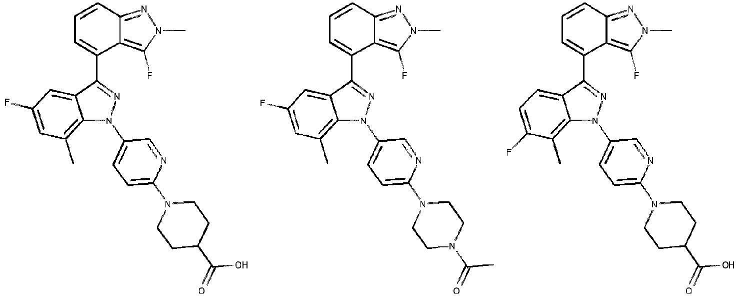 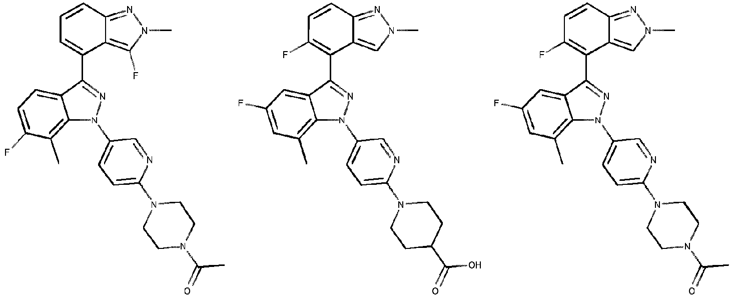 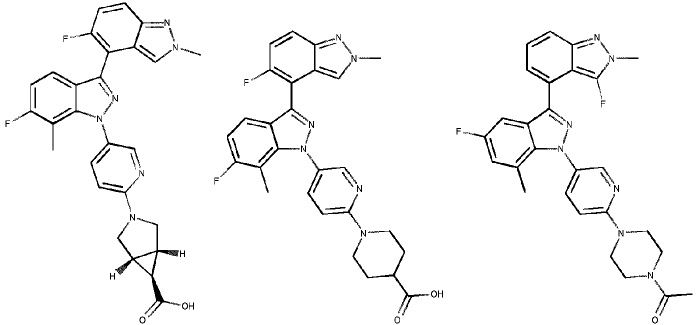 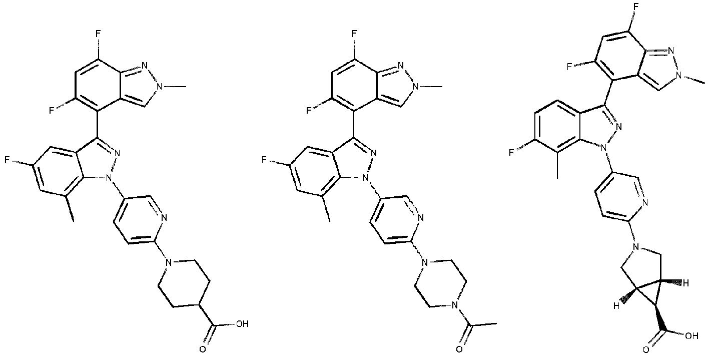 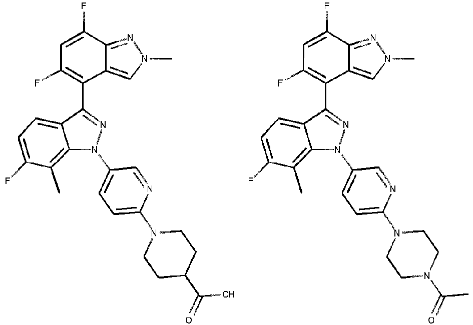 ou sel pharmaceutiquement acceptable correspondant.

11. Composition pharmaceutique comprenant au moins un composé selon l'une quelconque des revendications 1-10 ou un sel pharmaceutiquement acceptable de celui-ci et un support pharmaceutiquement acceptable.

12. Composé selon l'une quelconque des revendications 1-10 ou sel pharmaceutiquement acceptable de celui-ci ou composition pharmaceutique selon la revendication 11 pour utilisation en tant que médicament.

13. Composé selon l'une quelconque des revendications 1-10 ou sel pharmaceutiquement acceptable de celui-ci ou composition pharmaceutique selon la revendication 11 pour utilisation dans le traitement du cancer du félin ou du canin.

14. Composé ou sel pharmaceutiquement acceptable de celui-ci ou composition pharmaceutique pour utilisation selon la revendication 13, dans lequel le cancer canin est choisi parmi l'ostéosarcome (OSA), le mélanome buccal, le lymphome à cellules B, le carcinome urothélial (UC), l'hémangiosarcome, la tumeur à mastocytes, le sarcome des tissus mous, le carcinome épidermoïde, le lymphome à cellules T, l'adénocarcinome de la glande mammaire et le carcinome des glandes anales ou dans lequel le cancer du félin est choisi parmi le lymphome à cellules B et/ou à cellules T, le carcinome épidermoïde, l'adénocarcinome de la glande mammaire, les tumeurs à mastocytes et le sarcome au point d'injection.

15. Composé ou sel pharmaceutiquement acceptable de celui-ci ou composition pharmaceutique pour utilisation selon la revendication 13 ou 14, dans lequel le composé, le sel pharmaceutiquement acceptable de celui-ci ou la composition pharmaceutique est pour une utilisation en combinaison avec une radiothérapie.
